# EUROPEAN PATENT APPLICATION

(11) **EP 3 789 489 A1**
(43) Date of publication of application: **10.03.2021**
(21) Application number: 19777039.9
(22) Date of filing: 28.03.2019
(51) Int. Cl.: C12N 7/01, A61K 35/76, A61P 35/00, C07K 16/22, C12N 15/13

(54) **SWELLING INHIBITOR-TYPE ONCOLYTIC VIRUS**

(30) Priority: 30.03.2018 JP 2018068847
(71) Applicant: Todo, Tomoki, Koto-ku Tokyo 135-0034 (JP)
(72) Inventor: Todo, Tomoki, Koto-ku Tokyo 135-0034 (JP)
(74) Representative: Mewburn Ellis LLP
(86) International application number: PCT/JP2019/013767
(87) International publication number: WO 2019/189643

(57) **Abstract**

An object of the present invention is to provide a swelling-suppressive virus for treating a tumor. The present invention provides an oncolytic virus containing a gene encoding a vascular endothelial cell growth factor (VEGF) antagonist; and a pharmaceutical composition for treating a tumor, containing the oncolytic virus.

## Description

### Technical Field

The present invention relates to a swelling-suppressive oncolytic virus, in particular an oncolytic virus containing a gene encoding a VEGF antagonist. The present invention also relates to a pharmaceutical composition for treating a tumor and a method for treating a tumor, using the oncolytic virus.

### Background Art

As a new tumor treatment with a virus, ongoing researches relates to treatment of a tumor by infecting the tumor cells with a virus and destroying the tumor cells along with replication of the virus. Unlike existing tumor treatments, the new treatment elicits tumor-cell-specific anti-tumor immunity in the process of destroying tumor cells and exerts a therapeutic effect on the whole body via immunization, in addition to the direct cytopathic action associated with viral replication. In recent years, genetically modified oncolytic viruses that have enhanced tumor selectivity have been developed by introducing artificial genetic modifications into viral genomes such as HSV-1 and adenovirus (Patent Literatures 1 to 3, Non Patent Literatures 1 to 3).

### Citation List

### Patent Literature

Patent Literature 1: Japanese Patent No. 4212897
Patent Literature 2: Japanese Patent No. 4921669
Patent Literature 3: International publication No. WO 2011/101912

### Non Patent Literature

Non Patent Literature 1: Todo, T. et al. (2000). Molecular Therapy, 2, 588-595.
Non Patent Literature 2: Markert, J. M. et al. (2000). Gene Therapy, 7, 867-874.
Non Patent Literature 3: Martuza, R. L. et al. (2000). Journal of Clinical Investigation, 105, 841-846.

### Summary of Invention

### Technical Problem

During the clinical development of tumor therapy with an oncolytic virus, the present inventor has found that swelling occurs in and around the tumor shortly after administration of the virus. The occurrence of swelling, especially in viral therapy on brain tumors, can result in increased intracranial pressure and temporary worsening of neurological symptoms, which can be a factor interfering with the therapy. The occurrence of swelling in and around the tumor shortly after administration of the oncolytic virus is an event found in the course of clinical development by the present inventor.

Based on this background, an object of the present invention is to provide a swelling-suppressive oncolytic virus.

### Solution to Problem

Adrenocorticotropic hormone (steroid) is a pre-existing treatment for swelling in and around the tumor. However, adrenocorticotropic hormone has side effects that suppress immunity. Meanwhile, anti-tumor immunity elicitation is one of the key treatment mechanisms of viral therapy; thus, adrenocortical hormone is not suitable for combination use with viral therapy. In malignant gliomas (malignant brain tumors), bevacizumab (trade name: Avastin (registered trademark)), an anti-VEGF antibody drug, is sometimes administered for the purpose of reducing cerebral edema. However, bevacizumab has a side effect that prevents wound healing, thus it is problematic to use bevacizumab in combination with viral therapy for brain tumors in which virus is administered in surgery.

The present inventor has found, as a result of intensive studies, that the above problem can be solved by causing an oncolytic virus to express a VEGF antagonist that antagonizes VEGF, and have achieved the present invention. That is, the present invention relates to the following.
[1] An oncolytic virus comprising a gene encoding a vascular endothelial cell growth factor (VEGF) antagonist.
[2] The oncolytic virus according to [1], wherein the VEGF antagonist is an anti-VEGF antibody or a fragment thereof.
[3] The oncolytic virus according to [2], wherein the VEGF antagonist is an anti-VEGF antibody or a single-stranded anti-VEGF antibody comprising a V_{H} chain and a V_{L} chain.
[4] The oncolytic virus according to [2] or [3], wherein the oncolytic virus comprises:
   a gene encoding a polypeptide of any one of following (i) to (iii):
      (i) a polypeptide of SEQ ID NO: 2;
      (ii) a polypeptide that consists of an amino acid sequence in which one or several amino acids are deleted, substituted or added in the polypeptide of SEQ ID NO: 2, and has VEGF binding ability; and
      (iii) a polypeptide that has 75% or more, 80% or more, 85% or more, 90% or more, 95% or more, 98% or more, or 99% or more homology to the polypeptide of SEQ ID NO: 2, and has VEGF binding ability; and
   a gene encoding a polypeptide of any one of following (iv) to (vi):
      (iv) a polypeptide of SEQ ID NO: 4;
      (v) a polypeptide that consists of an amino acid sequence in which one or several amino acids are deleted, substituted or added in the polypeptide of SEQ ID NO: 4, and has VEGF binding ability; and
      (vi) a polypeptide that has 75% or more, 80% or more, 85% or more, 90% or more, 95% or more, 98% or more, or 99% or more homology to the polypeptide of SEQ ID NO: 4, and has VEGF binding ability.
[5] The oncolytic virus according to any of [2] to [4], wherein the anti-VEGF antibody is a human monoclonal antibody or a humanized monoclonal antibody.
[6] The oncolytic virus according to [1], wherein the VEGF antagonist is a soluble VEGF receptor.
[7] The oncolytic virus according to [6], wherein the oncolytic virus comprises a gene encoding a polypeptide of any one of following (xiii) to (xv):
   (xiii) a polypeptide encoded by a nucleotide sequence of SEQ ID NO: 26;
   (xiv) a polypeptide that consists of an amino acid sequence in which one or several amino acids are deleted, substituted or added in the polypeptide encoded by the nucleotide sequence of SEQ ID NO: 26, and has VEGF binding ability; and
   (xv) a polypeptide that has 75% or more, 80% or more, 85% or more, 90% or more, 95% or more, 98% or more, or 99% or more homology to the polypeptide encoded by the nucleotide sequence of SEQ ID NO: 26, and has VEGF binding ability.
[8] The oncolytic virus according to any of [1] to [7], wherein the oncolytic virus is a variant of a virus selected from the group consisting of herpes simplex virus type I and type II (HSV-1 and HSV-2), adenovirus, poliovirus, measles virus, reovirus, vaccinia virus, seneca virus, vesicular stomatitis virus (VSV), Newcastle disease virus and coxsackievirus.
[9] The oncolytic virus according to any of [1] to [7], wherein the oncolytic virus is a herpes simplex virus type I variant having one or more characteristics of (a) to (c):
   (a) ICP6 gene is deleted or inactivated, or expressed under control of a tumor-specific promoter or a tissue-specific promoter;
   (b) γ34.5 gene is deleted or inactivated; and
   (c) ICP47 gene is deleted or inactivated.
[10] A pharmaceutical composition for treating a tumor, comprising a therapeutically effective amount of the oncolytic virus according to any of [1] to [9].
[11] The pharmaceutical composition for treating a tumor according to [10], wherein the tumor is a human tumor selected from the group consisting of a nervous system tumor, a pituitary tumor, medulloblastoma, melanoma, a brain tumor, a prostate cancer, a head and neck cancer, an esophageal cancer, a kidney cancer, a renal cell carcinoma, a pancreatic cancer, a breast cancer, a lung cancer, a colorectal cancer, a colon cancer, a gastric cancer, a skin cancer, an ovarian cancer, a bladder cancer, sarcoma, a squamous cell cancer, neuroectodermal tumor, a thyroid tumor, lymphoma, a hepatocellular carcinoma, mesothelioma, an epidermoid cancer, and a benign tumor.
[12] The pharmaceutical composition for treating a tumor according to [10], wherein the tumor is a brain tumor and the pharmaceutical composition is administered topically and swelling-suppressive.
[13] The pharmaceutical composition for treating a tumor according to any of [10] to [12], wherein the pharmaceutical composition is used in combination with another tumor therapy selected from a chemotherapy and a radiation therapy.

The present invention also relates to the following methods and uses:
[14] A method for treating a tumor, comprising administering a therapeutically effective amount of the oncolytic virus according to any one of [1] to [9] to a subject in need of treatment of the tumor.
[15] The method according to [14], wherein the tumor is a human tumor selected from the group consisting of a nervous system tumor, a pituitary tumor, medulloblastoma, melanoma, a brain tumor, a prostate cancer, a head and neck cancer, an esophageal cancer, a kidney cancer, a renal cell carcinoma, a pancreatic cancer, a breast cancer, a lung cancer, a colorectal cancer, a colon cancer, a gastric cancer, a skin cancer, an ovarian cancer, a bladder cancer, sarcoma, a squamous cell cancer, neuroectodermal tumor, a thyroid tumor, lymphoma, a hepatocellular carcinoma, mesothelioma, an epidermoid cancer, and a benign tumor.
[16] The method according to [14], wherein the tumor is a brain tumor, the virus is administered topically, and the method suppresses a tumor swelling.
[17] The method according to any one of [14] to [16], wherein the method is used in combination with another tumor therapy selected from a chemotherapy and a radiation therapy.
[18] Use of the oncolytic virus according to any one of [1] to [9] in the manufacture of a medicament for treating a tumor.
[19] The use according to [18], wherein the tumor is a human tumor selected from the group consisting of a nervous system tumor, a pituitary tumor, medulloblastoma, melanoma, a brain tumor, a prostate cancer, a head and neck cancer, an esophageal cancer, a kidney cancer, a renal cell carcinoma, a pancreatic cancer, a breast cancer, a lung cancer, a colorectal cancer, a colon cancer, a gastric cancer, a skin cancer, an ovarian cancer, a bladder cancer, sarcoma, a squamous cell cancer, neuroectodermal tumor, a thyroid tumor, lymphoma, a hepatocellular carcinoma, mesothelioma, an epidermoid cancer, and a benign tumor.
[20] The use according to [18], wherein the tumor is a brain tumor, the medicament is administered topically, and the medicament is swelling-suppressive.
[21] The use according to any one of [18] to [20], wherein the medicament is used in combination with another tumor therapy selected from a chemotherapy and a radiation therapy.

### Advantageous Effects of Invention

According to the present invention, a swelling-suppressive oncolytic virus can be provided. This allows for safer and easier treatment of tumors.

### Brief Description of Drawings

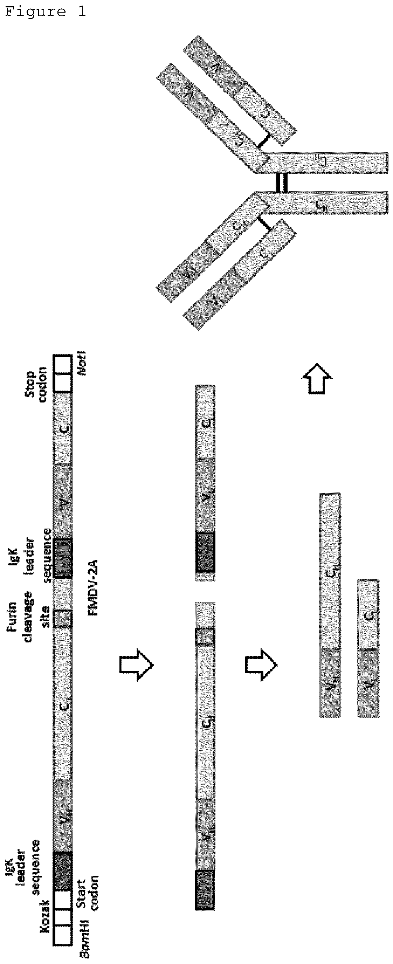

[Figure 1] Expression mechanism of an antibody from an antibody expression gene (modified from Zitvogel, L., et al. (1994) Human gene therapy 5, 1493-1506). When the foot-and-mouth-disease virus (FMDV) 2A sequence is used, gene expressions of upstream and downstream are roughly equivalent. Protein secretion is performed via signal recognition particles (SRPs). When the Ig kappa leader sequence of the H chain is translated in ribosomes, the SRP recognizes this and binds, then the bound body binds to the SRP receptor present on the endoplasmic reticulum, thereby the downstream polypeptide chain is transported into the rough endoplasmic reticulum via translocon on the membrane. At this time, the leader sequence at the N-terminal side is cleaved by a signal peptidase, and only the sequence at the C-terminal side is transported into the rough endoplasmic reticulum. The FMDV-2A sequence is cis-cleaved upon translation. In the same manner, the sequence of the L chain is also transported into the rough endoplasmic reticulum. The sequence is then sent to a Golgi body, and the basic amino acid target sequence Arg-X-(Lys/Arg)-Arg is specifically recognized and cleaved by furin. This isolates the residues of the self-cleaved 2A sequence, resulting in the formation of a double-stranded antibody and the extracellular secretion of the antibody.

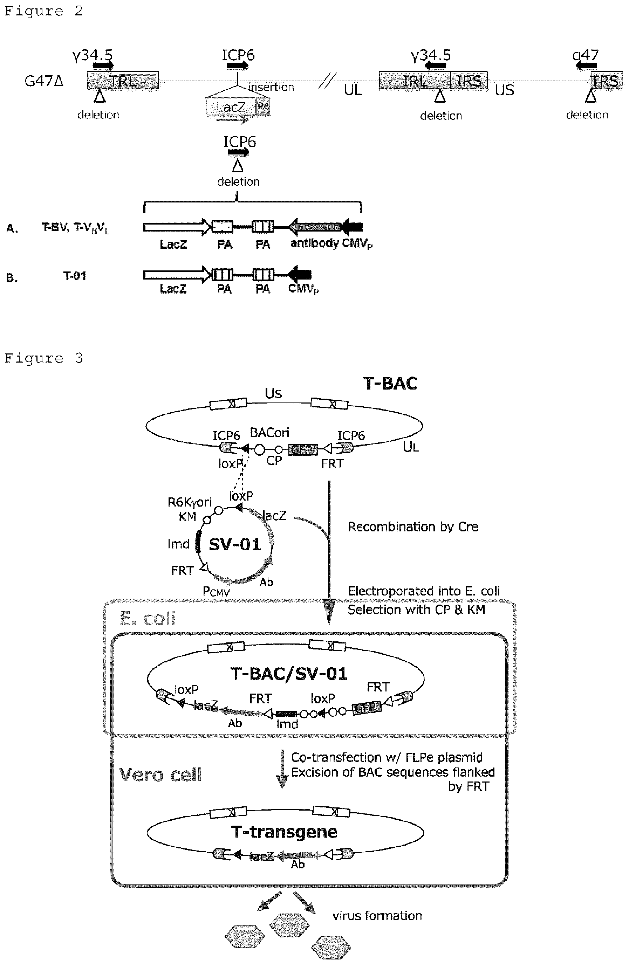

[Figure 2] The structure of the virus used in the present example. The structure has a basic backbone of G47Δ that has deletions in two γ34.5 present in TR_{L} and IR_{L}; inactivation by inserting the LacZ gene in ICP6 present in U_{L}; and a deletion in α47 present in U_{S} and the US11 promoter region overlapping with α47. T-BV and T-V_{H}V_{L} were generated by deleting ICP6, and inserting LacZ, polyA (PA), reverse directed cytomegalovirus promoter (CMVP), and each antibody expression gene at the downstream thereof (indicated as antibody in the Figure) into the deletion region (A). T-01 in which only LacZ, PA, and CMV_{P} were inserted was used as a control virus (B) .
[Figure 3] T-BAC system. In the T-BAC system, the maintenance and amplification of the G47Δ genome is facilitated by the insertion of BAC into the ICP6 deletion site of the G47Δ genome, and loxP and FRT sequences are included. By mixing the T-BAC with a shuttle vector plasmid SV-01 that similarly contains loxP and FRT sequences, and having a therapeutic gene inserted, and utilizing two recombinase systems, Cre/loxP and FLP/FRT, the insertion of a foreign gene and the excision of BAC occur, thereby G47Δ to which the foreign gene is inserted can be generated. KM: kanamycin resistance gene, CP: chloramphenicol resistance gene, lmd: λ staffer sequence, GFP: Green Fluorescent Protein.

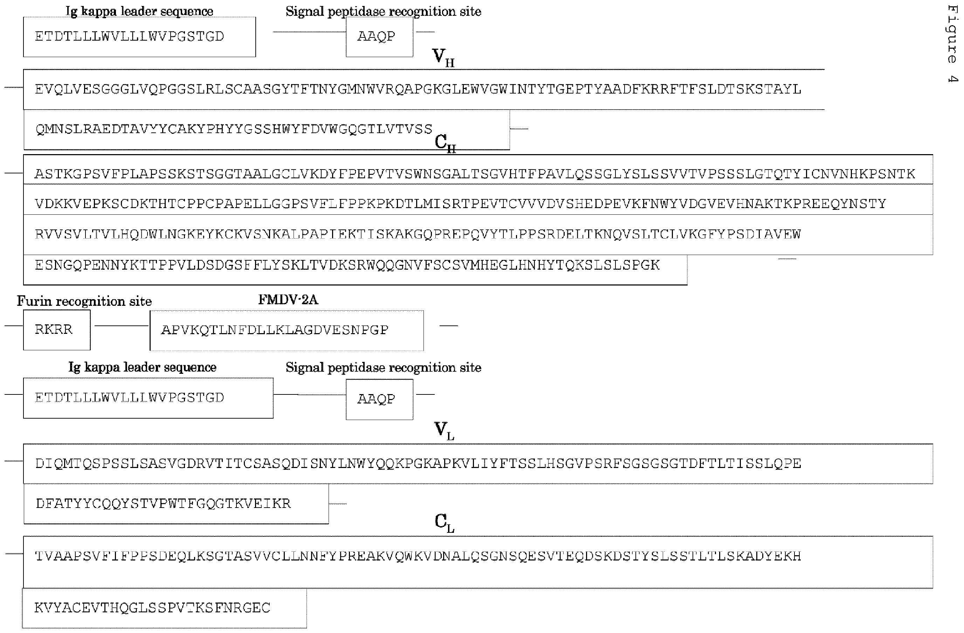

[Figure 4] Design of an amino acid sequence for anti-human VEGF antibody expression. The amino acid sequences of the Fab region of bevacizumab and the Fc region of human IgG1 were obtained from the databases of the International ImMunoGeneTics information system (IMGT) and National Center for Biotechnology Information (NCBI), respectively. The polypeptides corresponding to the heavy chain (H chain) and the light chain (L chain), which harbored an Ig kappa leader sequence and a signal peptidase recognition site as a secretory signal at their N-termini, were ligated with the foot-and-mouth-disease virus (FMDV)2A sequence and the furin recognition site, thereby an amino acid sequence for anti-human VEGF antibody expression was designed.

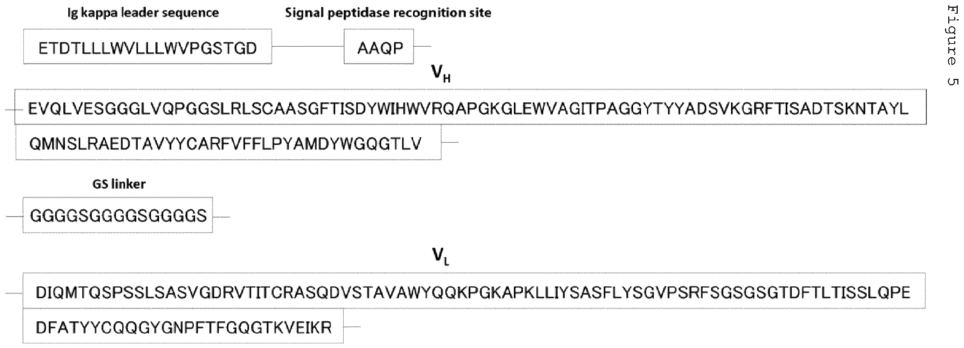

[Figure 5] Design of an amino acid sequence for anti-VEGF scFv expression. The amino acid sequences corresponding to the Fab of the H and L chains of the anti-VEGF antibody G6-31 described in Liang, W. C. et al. (2006). The Journal of Biological Chemistry, 281, 951-961 were ligated with a linker, and an Ig kappa leader sequence and a signal peptidase recognition sequence were ligated as a secretory signal at the N-terminal side, thereby an amino acid sequence for anti-VEGF scFv expression was designed. As the linker, the GS linker commonly used in scFv was used.

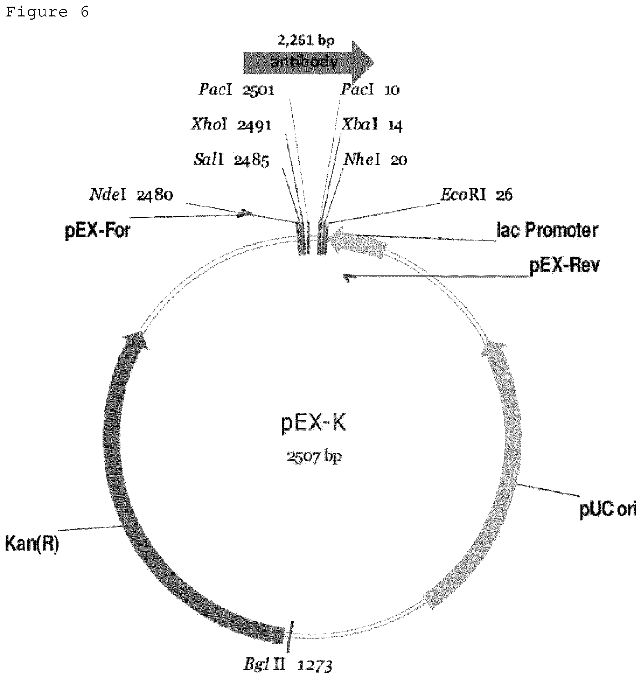

[Figure 6] A schematic diagram showing the structure of pEX-K-BV.

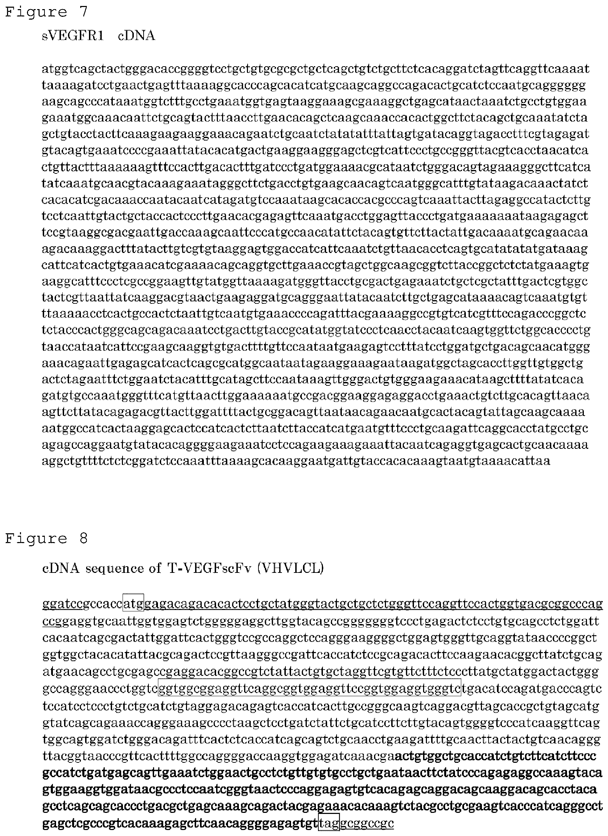

[Figure 7] A diagram showing the cDNA sequence of sVEGFR1.
[Figure 8] A diagram showing the cDNA sequence of T-VEGFscFv (V_{H}V_{L}C_{L}). The restriction enzyme (underlined), the Kozak sequence, the start codon (square frame), the Ig kappa leader sequence (underlined), the heavy chain VH, the linker (square frame), the light chain VL, the light chain CL (bold), the terminal codon (square frame), and the restriction enzyme (underlined) are described in this order.

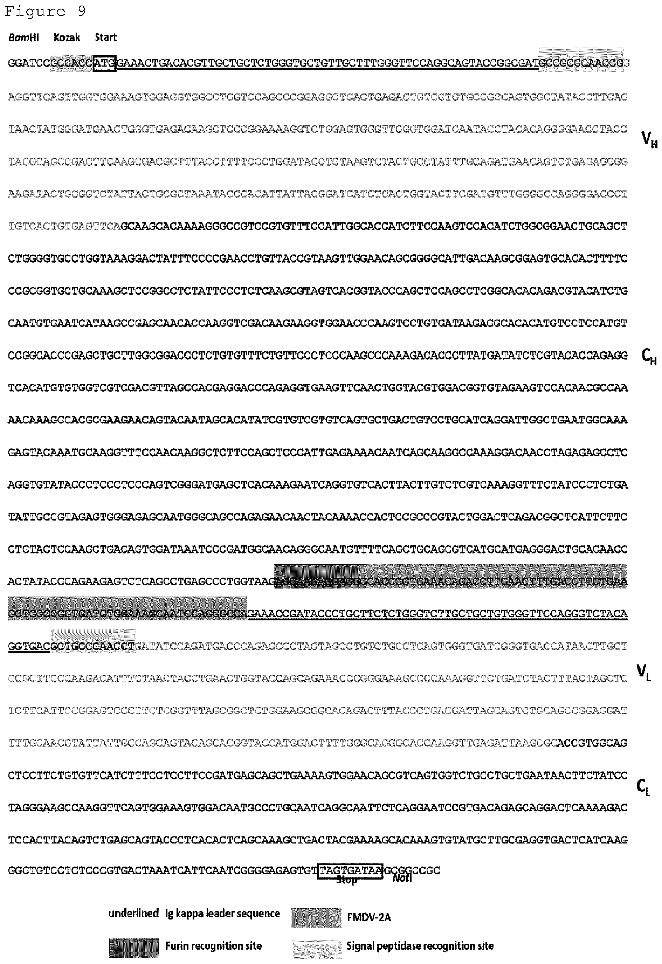

[Figure 9] cDNA of an anti-human VEGF antibody expression gene. The cDNA encoding an antibody for expression was designed based on the amino acid sequence of Figure 4 by converting the amino acid sequence to a nucleotide sequence optimized for codons in human, then ligating the Kozak sequence, the start codon, and the BamHI recognition sequence at the N-terminus, and the NotI recognition sequence at the C-terminus.

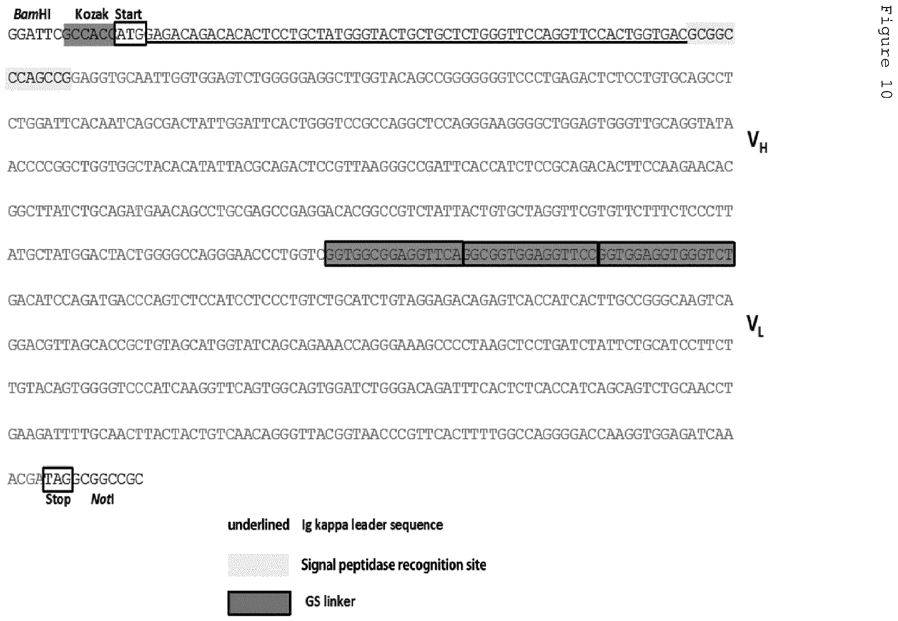

[Figure 10] cDNA of an anti-VEGF scFv expression gene. From the amino acid sequence G6-31 described in Liang, W. C. et al. (2006). The Journal of Biological Chemistry, 281, 951-961, cDNAs were designed using a genetic information processing software GENETYX. The cDNAs corresponding to each of the heavy and light chains were ligated with a linker to provide an anti-VEGF scFv expressing cDNA. As the linker, the GS linker commonly used in scFv was used. Similar to T-BV, an Ig kappa leader sequence and a signal peptidase recognition sequence were ligated as a secretory signal at the N-terminal side of the VH sequence.

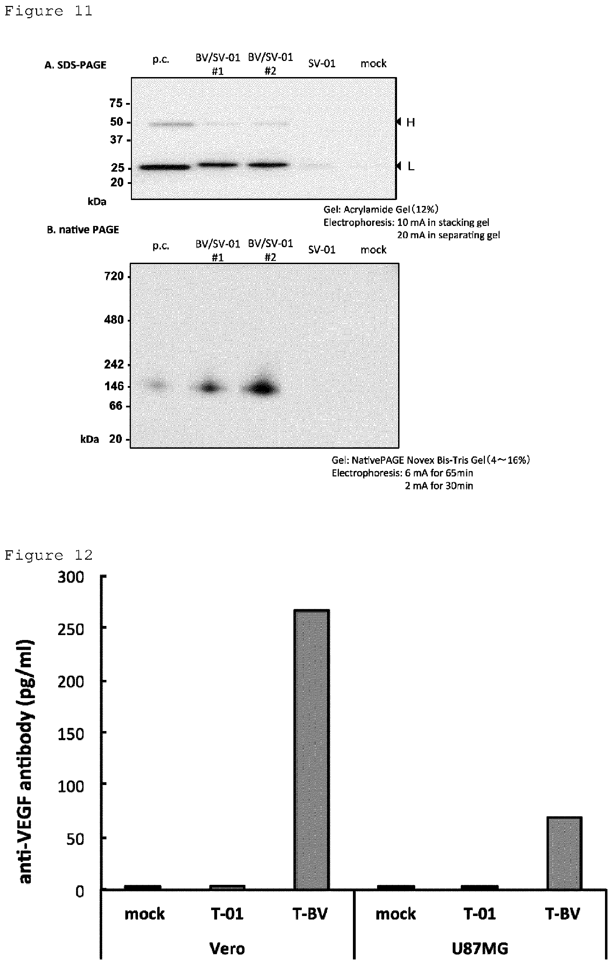

[Figure 11] Western blotting using a supernatant of HEK293T cells transfected with BV/SV-01. HEK293T was transfected with BV/SV-01, and the culture supernatant was used to perform SDS-PAGE and native PAGE. A supernatant of the cells transfected with SV-01 was used as a negative control, and Avastin(registered trademark) was used as a positive control. Anti-human IgG (H+L) antibodies were used to detect the proteins. The results were similar to those of Avastin(registered trademark), a positive control, in both SDS-PAGE (Figure 11A) and native PAGE (Figure 11B), and it was confirmed that the protein expressed by the anti-human VEGF antibody expression gene used to generate a novel virus in this study was a double-stranded antibody.
[Figure 12] Quantification of the expression amount of anti-human VEGF antibody by ELISA using a viral infected supernatant. Vero cells and U87MG cells dispersed on 6-well plates were infected with T-01, T-BV or mock at MOI of 0.2, cultured in a 2 mL of medium for 72 hours, and then the supernatant was collected and concentrated 2.7-fold by ultrafiltration to be used as specimens. As standards, eight serial dilutions of Avastin (registered trademark) were used. Human VEGF was immobilized in wells of a ELISA plate and detection was performed with anti-human IgG (Fc) antibodies. Absorbance of 450 nm was measured, and the anti-VEGF antibody concentration of the specimen was calculated. For Vero cells and U87MG cells, 256 pg/mL and 69 pg/mL anti-VEGF antibodies were respectively detected from the supernatant of the cells infected with T-BV, and antibodies were below the detection limit from the both culture supernatants of T-01 and mock.

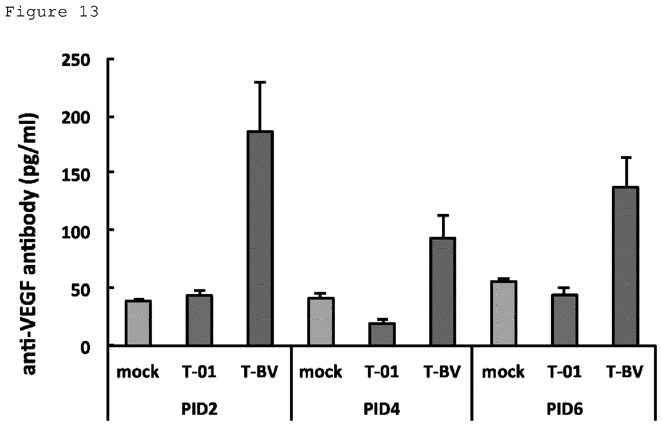

[Figure 13] Detection of anti-VEGF antibody from T-BV in U87MG subcutaneous tumors. Balb/c nu/nu female mice in which U87MG subcutaneous tumors were confirmed to be 5 mm were grouped into the T-01, T-BV and mock groups (9 mice each). 2 x 10⁶ pfu viruses were administered in a single dose into the tumor, mice were euthanized at PIDs 2, 4, and 6 by 3 mice each for each group, subcutaneous tumors were removed, and then anti-human VEGF antibodies contained therein were quantified by ELISA. In the T-BV group, the expression of anti-human VEGF antibody was observed at every time point. bar; SEM.

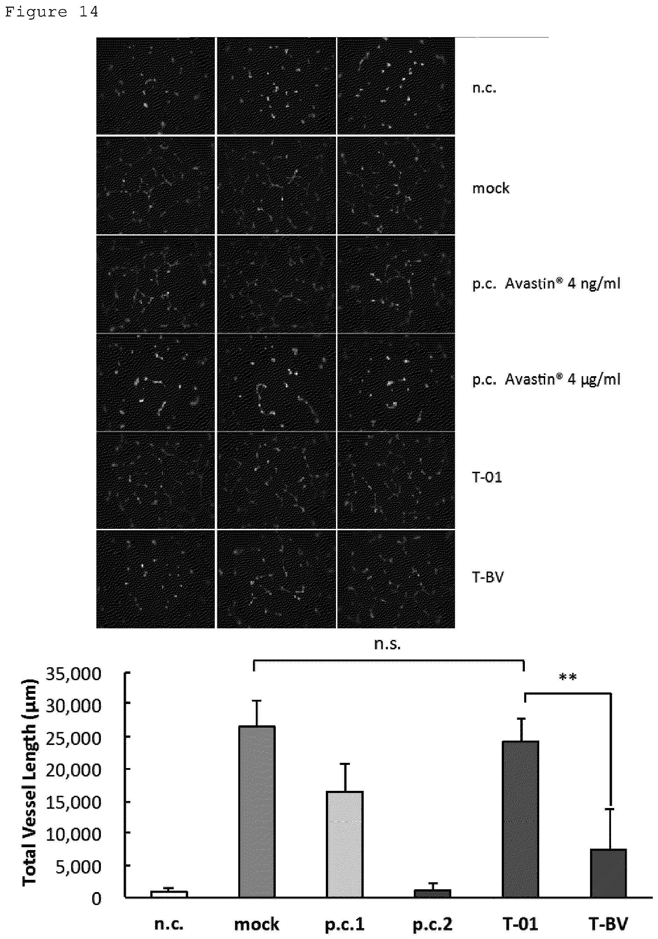

[Figure 14] A vascular endothelial cell tube formation test. Culture supernatants of Vero cells infected with T-01 or T-BV were used as specimens. As negative controls for VEGF effect, no supernatant (n.c.) and a supernatant of the virus-uninfected cells (mock) were used. As positive controls for anti-VEGF effect, two concentrations of bevacizumab (Avastin(registered trademark)), p.c.1 (4 ng/mL) and p.c.2 (4 µg/mL) in T-01 culture supernatant/medium were used. After HUVEC-2 was cultured for 22 hours in the medium to which each specimen was added, HUVEC-2 was stained with calcein. The tube formation images were taken by fluorescence microscopy, the length of the tube formed was measured, and the full length was calculated. The tube formation was significantly suppressed in the T-BV group relative to the T-01 group (p = 0.014). bar; SEM.

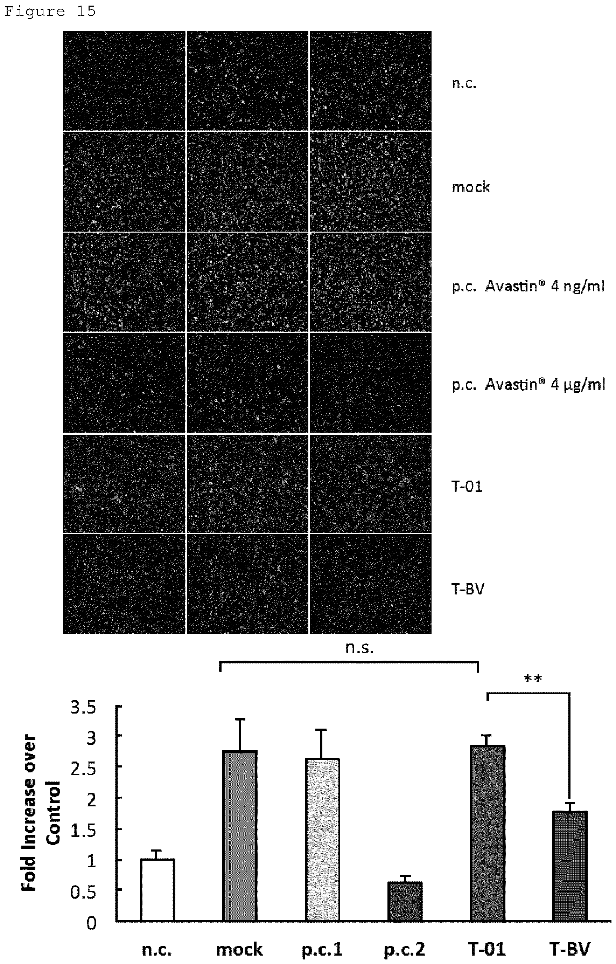

[Figure 15] A vascular endothelial cell migration test. Culture supernatants of Vero cells infected with T-01 or T-BV were used as specimens. As negative controls for VEGF effect, no supernatant (n.c.) and a supernatant of the virus-uninfected cells (mock) were used. As positive controls for anti-VEGF effect, two concentrations of bevacizumab (Avastin(registered trademark)), p.c.1 (4 ng/mL) and p.c.2 (4 µg/mL) in T-01 culture supernatant/medium were used. After HUVEC-2 was cultured for 16 hours in a medium to which each specimen was added, the migrated HUVEC-2 was stained with calcein, then imaged by fluorescence microscopy, and the fluorescence intensity was calculated to compare. The migration was significantly suppressed in the T-BV group relative to the T-01 group (p = 0.007). bar; SEM.

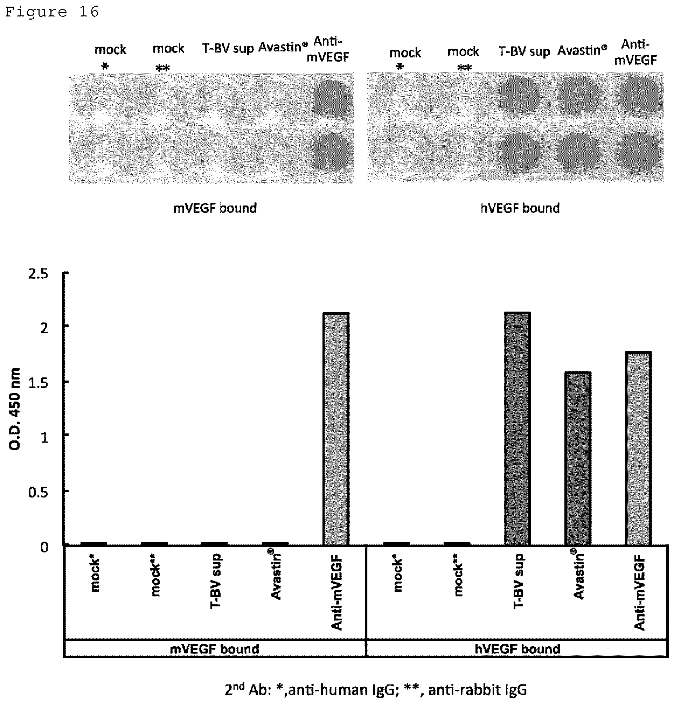

[Figure 16] A study of interspecies cross-reactivity of anti-VEGF antibodies. HEK293T cells was infected with T-BV at MOI of 3, cultured for 15 hours and the supernatant of the cells was used. As controls, Avastin(registered trademark), an anti-human VEGF antibody, and a rabbit anti-mouse VEGF antibody were used. Two types of detection antibodies were also reacted with mock to exclude false positives due to cross-reactions of the detection antibodies with immobilized VEGF. Absorbance at 450 nm was measured. The anti-mouse VEGF antibody bound both mouse VEGF (mVEGF) and human VEGF (hVEGF), while the T-BV-infected cell supernatant and Avastin(registered trademark) only bound hVEGF. From this result, it was considered that the expressed antibody of T-BV has specificity for hVEGF, similar to Avastin(registered trademark).

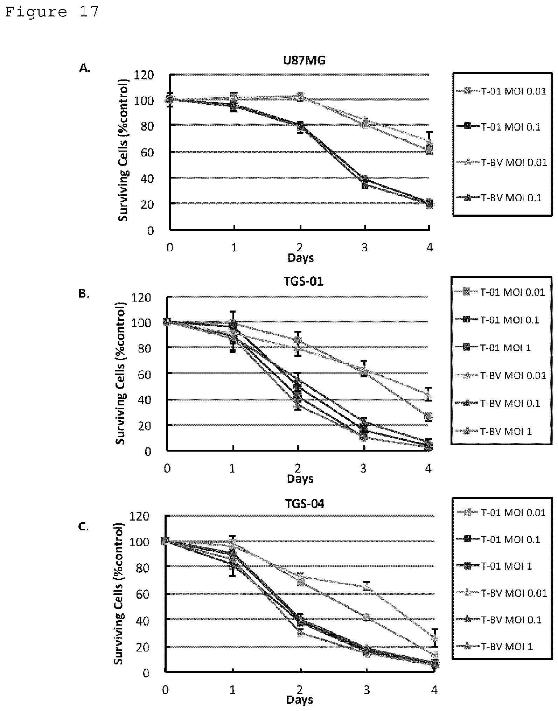

[Figure 17] In vitro cytotoxicity assay. In vitro cytotoxicity assays were performed in U87MG cells (Figure 17A), TGS-01 cells (Figure 17B), and TGS-04 cells (Figure 17C). Since TGS is a floating cell, the cytopathic effect was evaluated by MTS assay. The percent of the number of surviving cells in each group relative to the number of surviving cells in the control group was shown as a cytopathic effect. The cytopathic effects of T-01 and T-BV were considered to be approximately equivalent. bar; ± SD.

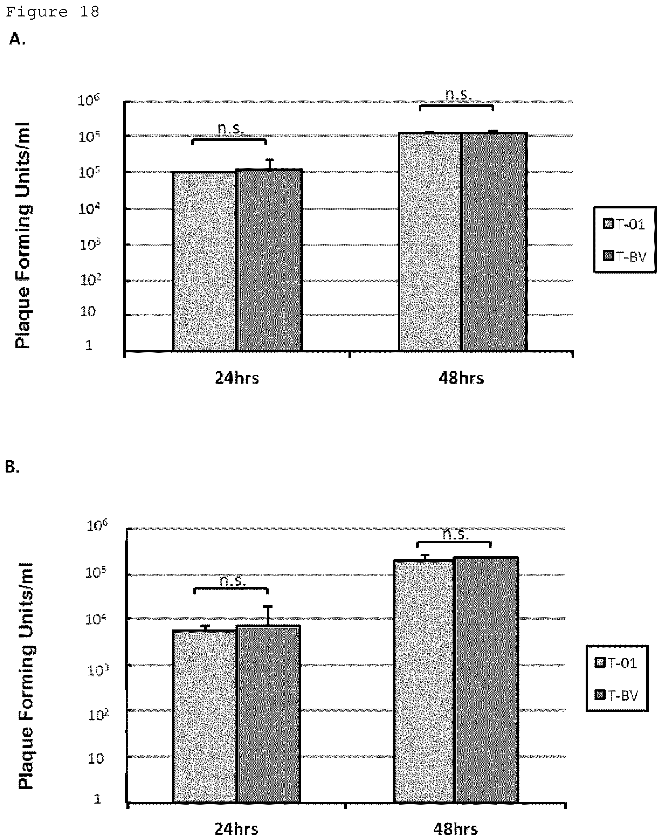

[Figure 18] In vitro replication assay. Vero cells (Figure 18A) and U87MG cells (Figure 18B) were infected with virus at MOI of 0.01. The cells were collected after 24 and 48 hours of culture together with the medium, and the titer of the virus contained therein was measured. Replication of the virus was observed between 24 and 48 hours in all cells and all viruses, and the replication abilities of T-01 and T-BV was considered to be approximately equivalent at any point. bar; SD.

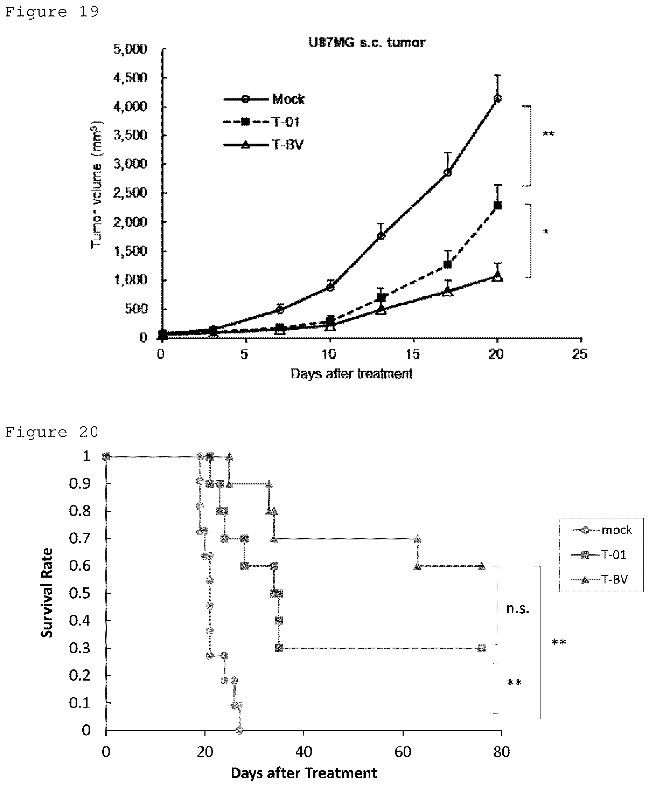

[Figure 19] A study of anti-tumor effects of T-BV in U87MG subcutaneous tumor models. 2 x 10⁶ cells of U87MG cells were implanted subcutaneously into the left abdomen of Balb/c nu/nu female mice. Upon the tumor diameter reached 6 mm, the mice were randomly divided into 3 groups of T-BV, T-01, and mock by 10 mice each. 2 x 10⁵ pfu viruses were administered twice with 3-days interval into the tumor. Tumor diameters were measured 2-3 times per week, and tumor volumes were calculated. On day 20 after virus administration, a significant tumor growth suppressive effect was observed in the T-01 group relative to the mock group (p = 0.00749, independent t-test). Furthermore, a significant tumor growth suppressive effect was observed in the T-BV group relative to the T-01 group (p = 0.0211, independent t-test). bar; SEM.
[Figure 20] A study of anti-tumor effects of T-BV in U87MG brain models. 2 x 10⁵ cells of U87MG cells were implanted into the right frontal lobe of Balb/c nu/nu female mice with a stereotaxic brain operation device. On day 10 after implantation, the mice were randomly divided into 3 groups of T-BV, T-01 and mock by 10 mice each. 1 x 10⁶ pfu viruses were administered in a single dose into the tumors, and the survival of each individual thereafter was recorded. On day 27 after virus administration, the mice in the mock group were all dead. In contrast, on day 76, the final follow-up time point, three survivors were observed in the T-01 group and six survivors were observed in the T-BV group. Both T-01 and T-BV showed significant prolonged survival effects relative to mock (p < 0.001 in both), and T-BV showed more prolonged survival tendency relative to T-01 (p = 0.163) .

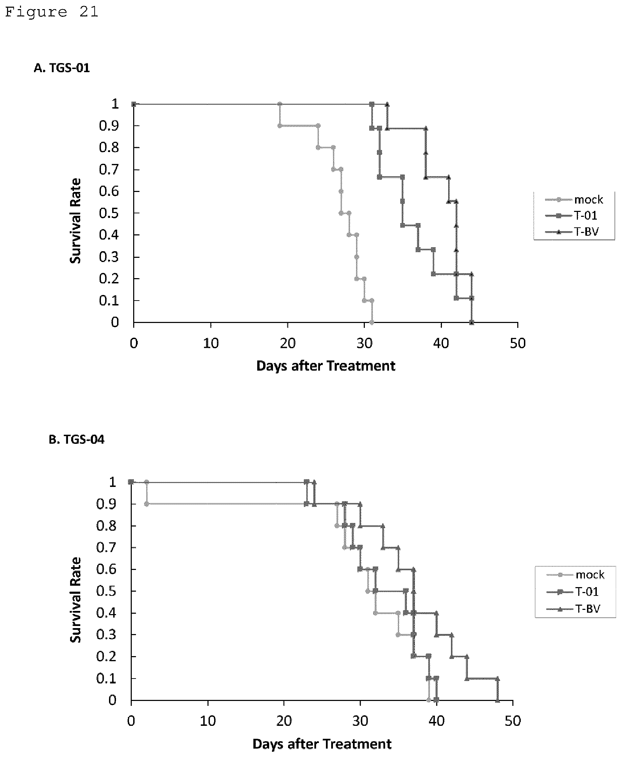

[Figure 21] A study of anti-tumor effects of T-BV in TGS-01 and TGS-04 brain tumor models. 1 x 10⁵ cells of TGS-01 cells (Figure 21A) or TGS-04 cells (Figure 21B) were implanted into the right frontal lobe of Balb/c nu/nu female mice with a stereotaxic brain operation device. On day 10 and day 20 after implantation, the mice were randomly divided into 3 groups of T-BV, T-01 and mock by 10 mice each. 2 x 10⁶ pfu viruses were administered in a single dose into the tumors, and the survival of each individual was recorded. In the TGS-01 brain tumor model, significant prolonged survival was observed in both the T-BV and T-01 groups relative to the mock group (p < 0.01 in both). Furthermore, T-BV showed significant prolonged survival even relative to T-01 (p = 0.0475). In the TGS-04 brain tumor model, no prolonged survival was observed in T-01 relative to mock, while significant prolonged survival was observed in T-BV (p = 0.0495).

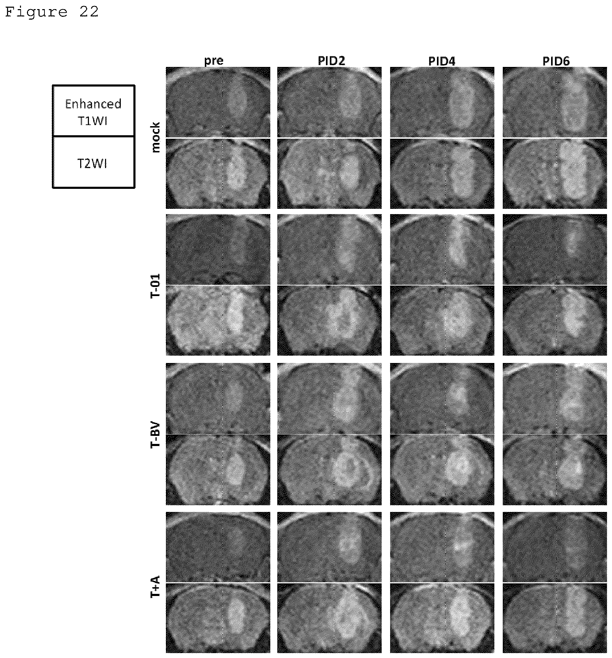

[Figure 22] MRI imaging of brain tumor models using brain tumor cell lines (U87MG cells). 2 x 10⁵ cells of U87MG cells were implanted into the right frontal lobe of Balb/c nu/nu female mice with a stereotaxic brain operation device. On day 11 after implantation, MRI of pre-virus administration (pre) was taken. On day 12, all 20 mice were randomly grouped into mock, T-01, T-BV, and T+A groups, then 2 x 10⁶ pfu viruses were administered in a single dose into the tumors. MRI (T2WI and T1WI (CE)) were taken at PIDs 2, 4, and 6. For the T+A group, 5 mg/kg Avastin(registered trademark) was administered intraperitoneally from date of virus administration to day 5 on a daily basis. The appearance of a high intensity area was observed around the tumor in T2WI after virus administration.

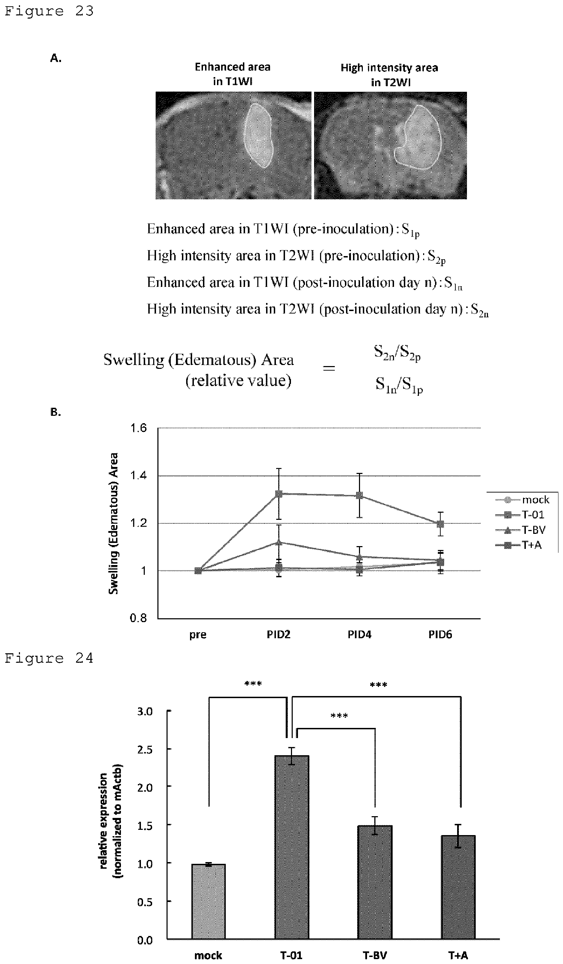

[Figure 23] A study (1) of swelling-suppressive effects of T-BV in U87MG brain tumor models (MRI). Quantification of swelling (edema) was performed based on MRI data of U87MG brain tumor models. Measurements of the high intensity areas in T2WI and T1WI (CE) were performed using a free software OsiriX. For each of T2WI and T1WI (CE), the ratio of the high intensity area for each imaging day to the high intensity area prior to virus administration was calculated, and the value in T2WI was divided by the value in T1WI (CE) to determine the relative value of the swelling area (Figure 23A). Little swelling was observed in the mock group, and the relative value was consistently remained at almost 1. In the T-01 group, elevation of relative values were observed at PIDs 2, 4, and 6. However, relative values were significantly low at PIDs 4, and 6 in the T-BV group and at PIDs 2, 4, and 6 in the T+A group (all p < 0.05), suggesting that the occurrence of swelling was suppressed (Figure 23B). bar; SEM.
[Figure 24] A study (2) of swelling-suppressive effects of T-BV in U87MG brain tumor models (RT-qPCR). U87MG brain tumor models of Balb/c nu/nu female mice were grouped into the mock, T-01, T-BV and T+A groups by 6 mice each, and 1 x 10⁶ pfu viruses were administered in a single dose into the tumors. For the T+A group, 5 mg/kg Avastin(registered trademark) was administered intraperitoneally from the date of virus administration to day 5 on a daily basis. The right frontal lobe was harvested at PID6, and cDNA was made using reverse transcriptase from extracted RNA to perform quantitative PCR against mouse AQP4. Mouse β-actin (mActb) was used as a house keeping gene. Expression of mouse AQP4 was significantly elevated in the T-01 group relative to the mock group (p < 0.01), and expression of AQP4 was significantly decreased in the T-BV and T+A groups relative to the T-01 group (p < 0.01 in both). bar; SEM.

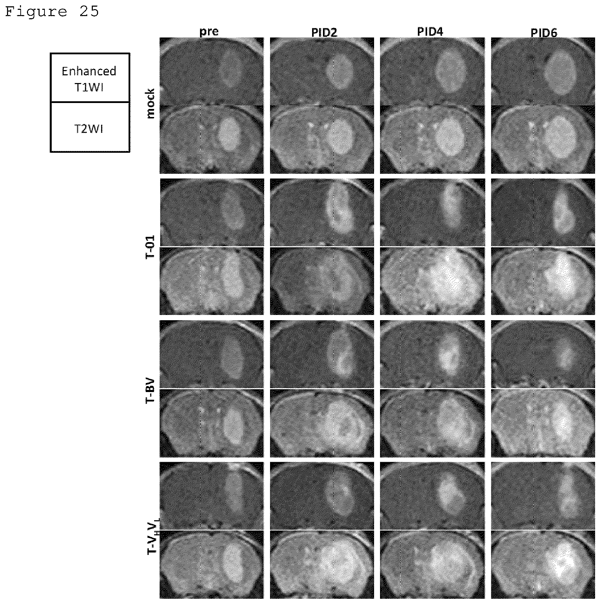

[Figure 25] A comparative study of swelling-suppressive effects of T-BV and T-VHVL in U87MG brain tumor models (representative images). 2 x 10⁵ cells of U87MG cells were implanted into the right frontal lobe of Balb/c nu/nu female mice with a stereotaxic brain operation device. On day 11 after implantation, MRI of pre-virus administration (pre) was taken. On day 12, all 20 mice were randomly grouped into mock, T-01, T-BV, and T-VHVL groups, then 2 x 10⁶ pfu viruses were administered in a single dose into the tumors. MRI (T2WI and T1WI (CE)) were taken at PIDs 2, 4, and 6.

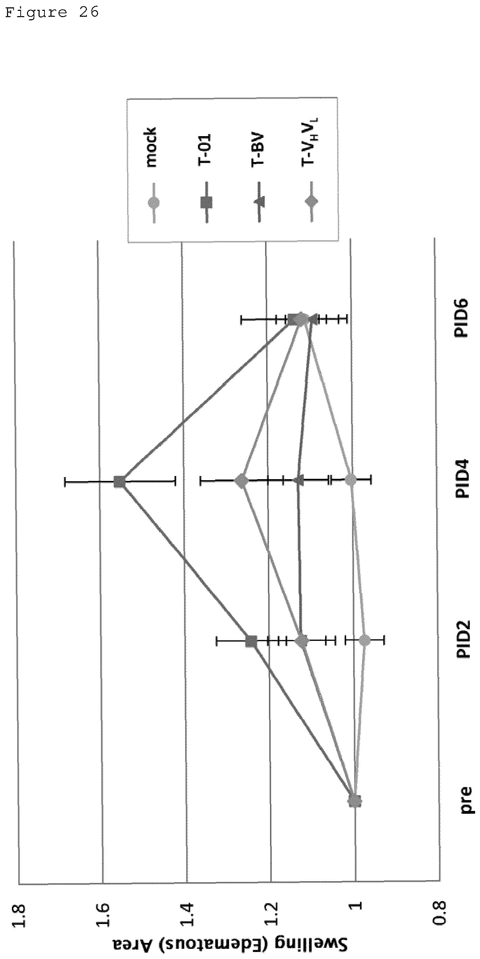

[Figure 26] A comparative study of swelling-suppressive effects of T-BV and T-VHVL in U87MG brain tumor models (quantification of edematous areas). Evaluation of swelling (edema) was performed in the same manner as the previous experiments for MRI data from U87MG brain tumor models. A significant reduction of the swelling areas was observed at PID 4 (p = 0.022) in T-BV relative to T-01. A reduction tendency of the swelling areas was also observed in T-VHVL relative to T-01. bar; SEM.

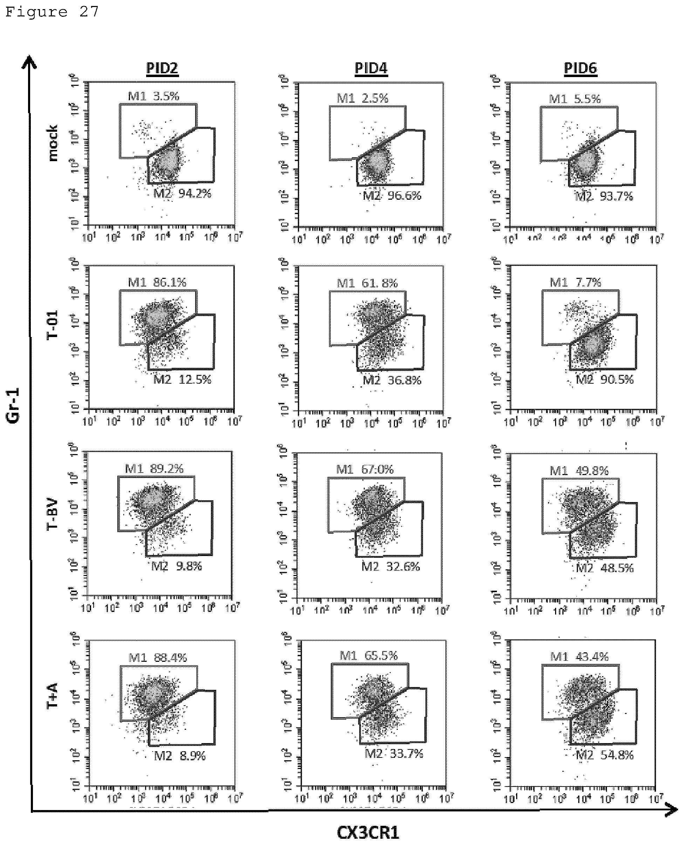

[Figure 27] An evaluation of macrophages by flow cytometry after virus administration to U87MG brain tumor models (representative scatter plot). Balb/c nu/nu female mice with U87MG brain tumors were grouped into T-01, T-BV, T+A, and mock groups by 9 mice each. 1 x 10⁶ pfu viruses were administered in a single dose into the tumors. The right frontal lobes were harvested at PIDs 2, 4, and 6, and single-cell suspensions were prepared. For the T+A group, 5 mg/kg Avastin(registered trademark) was administered intraperitoneally from the date of virus administration to euthanasia on a daily basis. Myelin fractions were removed by a two-dimensional plot of scattered light, doublets were removed, and dead cells were removed. Then, those gated by CD45-positive, CD11b-positive, F4/80-positive were plotted for Gr-1 and CX3CR1 to separate two cell populations of M1 (Gr-1highCX3CR1low) and M2 (Gr-1lowCX3CR1high). M2 fractions were consistently dominant in the mock administration group, whereas M1 fractions were dominant at PID2 in the virus administration group.

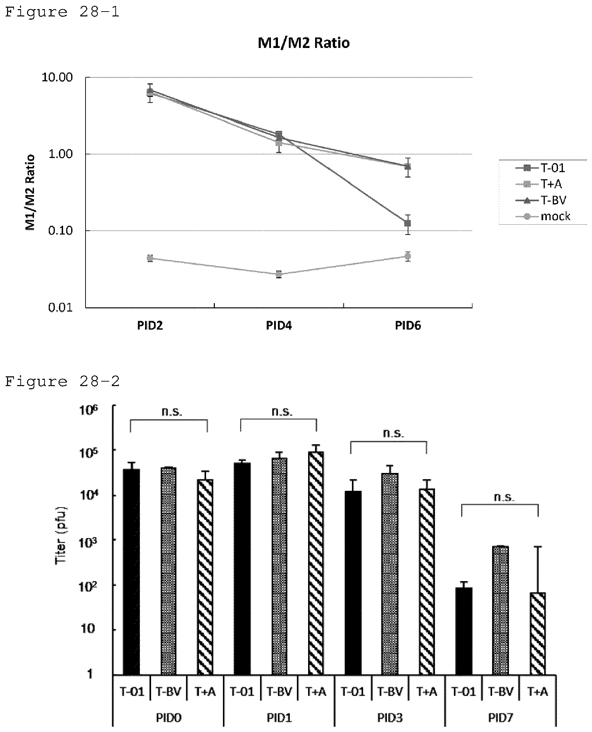

[Figure 28-1] Shift of M1/M2 ratio over time. For each of M1 and M2, the proportion in CD45-positive cells was calculated and the ratio of M1/M2 was calculated to evaluate which of M1 and M2 was dominant. In the mock group, M2 was consistently dominant as M1/M2 was shifted over time with 0.04-0.03-0.05, whereas, in the virus administration group, M1 was dominant as M1/M2 was > 1. In the T-01 group, the value of M1/M2 decreased over time with 6.21-1.81-0.13, and the state returned from M1 dominant to M2 dominant. While in the T-BV and T+A groups, although the values of M1/M2 decreased over time with 6.87-1.64-0.69 and 6.42-1.41-0.69, respectively, the proportion of M1 remained relatively high even at PID 6, and the respective M1/M2 ratios were significantly higher than that of T-01 (p < 0.05, p < 0.01). bar; SEM.
[Figure 28-2] The effect of persistent M1 dominance on viral titer was examined. The viral titers at PID1, PID3 and PID7 were examined for the T-01, T-BV and T+A groups, and no significant differences in viral titer were seen among the three groups at any measurement date.

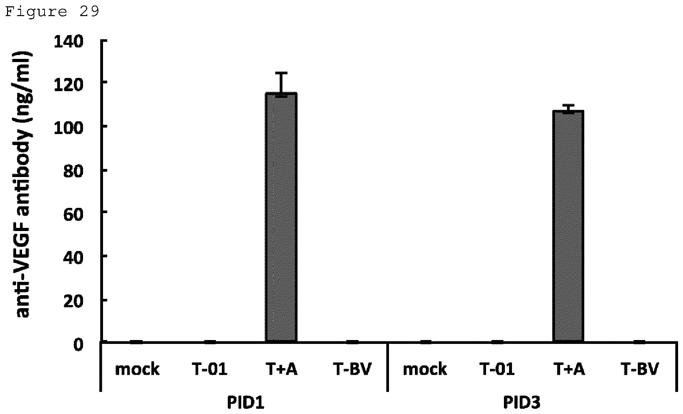

[Figure 29] Quantification of anti-VEGF antibodies in serum after T-BV administration to U87MG brain tumors. Balb/c nu/nu female mice with U87MG brain tumors were grouped into mock, T-01, T-BV and T+A groups by 6 mice each. 1 x 10⁶ pfu viruses were administered in a single dose into the tumors. Venous blood sampling was performed at PIDs 1 and 3 by 3 mice in each group, and anti-human VEGF antibodies contained in that serum were quantified by ELISA. For the T+A group, 5 mg/kg Avastin(registered trademark) was administered in a single dose intraperitoneally on the date of virus administration. The anti-human VEGF antibodies were detected in the T+A group at every time point, while the antibodies were below the detection limit in all other groups. bar; SEM.

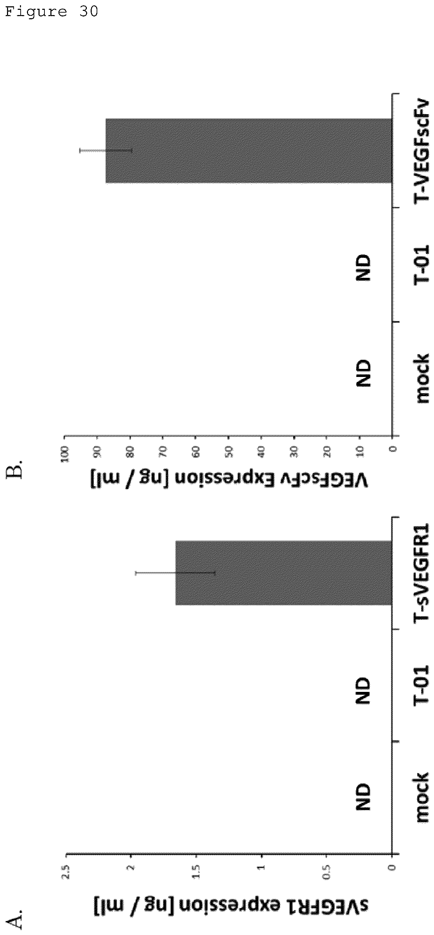

[Figure 30] A graph showing the confirmation by ELISA method for the expressions of VEGF inhibitor protein by T-sVEGFR1 and T-VEGFscFv. Vero was infected with T-01, T-sVEGFR1 and T-VEGFscFv, and the supernatant from the cells cultured for 48 hours was used to perform ELISA. A. In the culture supernatant of T-sVEGFR1, sVEGFR1 protein was detected. B. In the culture supernatant of T-VEGFscFv, VEGFscFv protein was detected. The proteins were not detected in the mock and the culture supernatant of T-01. n = 3; Error bar, standard deviation; ND, not detected.

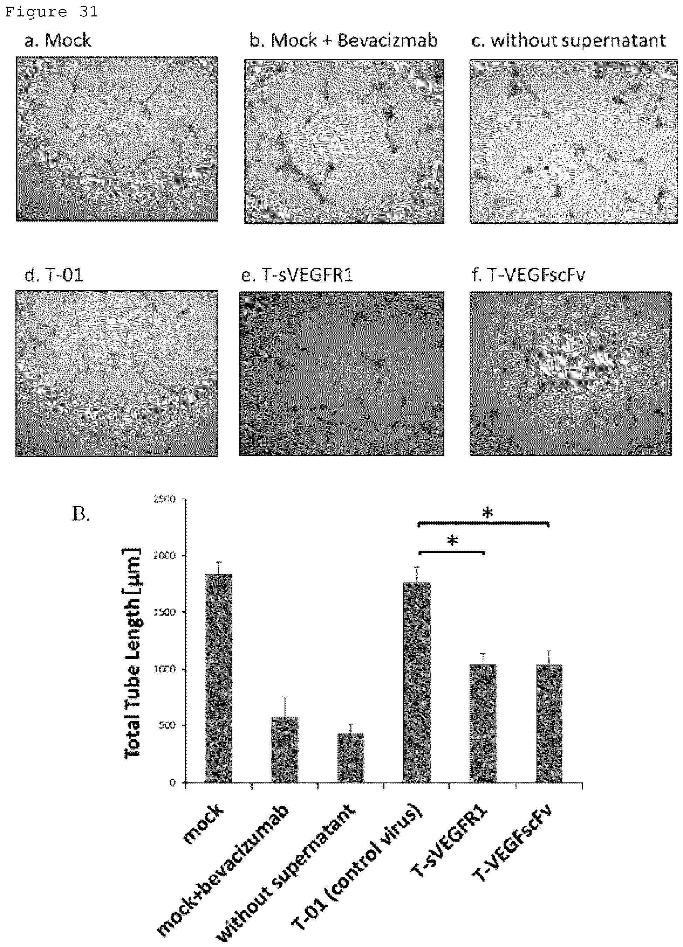

[Figure 31] A graph showing the results of a vascular endothelial cell tube formation test. Vero was infected with T-01, T-sVEGFR1 and T-VEGFscFv at MOI of 0.2, and cultured for 2 days. The culture supernatant was collected and concentrated 40-fold, and then added to the wells coated with Matrigel Matrix. HUVEC was further seeded thereto at 2 x 10⁴ cells/well. As a positive control, bevacizmab added to Medium 200/1%LSGS at 4 µg/ml was added. As a negative control, only Medium 200/1%LSGS was added. After the cells were cultured for 22 hours, the tube formation images of HUVEC were taken microscopically (Figure 31A), and the total tube length was calculated and compared. Tube Formation was significantly suppressed in the T-sVEGFR1 and T-VEGFscFv infected groups relative to the T-01 infected group (Figure 31B). n = 3; Error bar, standard deviation; *, p < 0.05.

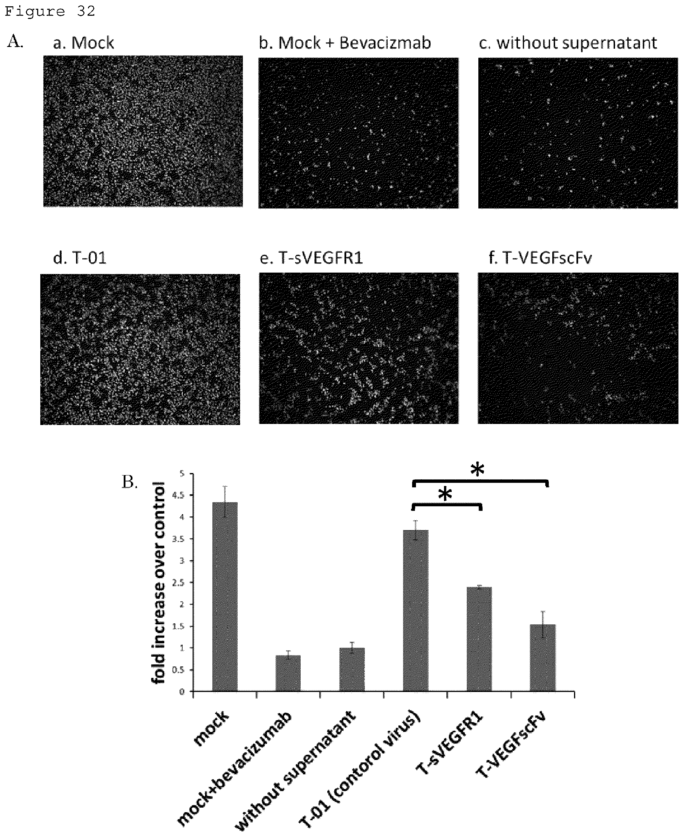

[Figure 32] A graph showing the results of a vascular endothelial cell migration test. HUVEC was seeded in the upper chamber at 1 x 10⁵ cells/well. In the lower wells, the culture supernatant which was prepared from cells infected with T-01, T-sVEGFR1 or T-VEGFscFv at MOI of 0.2 and cultured for 2 days, and concentrated 40-fold was added. HUVEC was also placed in the upper chamber at 1 x 10⁵ cells/well. As a positive control, bevacizmab was added to Medium 200/1%LSGS at 4 µg/ml. As a negative control, only Medium 200/1%LSGS was added. After cultured for 22 hours, HUVEC which passed through the membrane of the upper chamber and migrated to the lower well was fluorescently stained with calcein AM solution, and imaged by fluorescence microscopy (Figure 32A), and the fluorescence intensity was measured (Figure 32B). Migration of HUVEC was significantly suppressed in the T-sVEGFR1 and T-VEGFscFv infected groups relative to the T-01 infected group. n = 3; Error bar, standard deviation; *, p < 0.05.

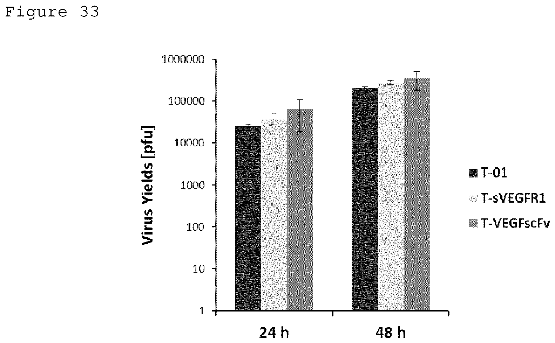

[Figure 33] A graph showing the results of in vitro viral replication assays with HT-29 for T-sVEGFR1, T-VEGFscFv. HT-29 was seeded in 6-well plates at 4 x 10⁵ cells/well, infected with T-01, T-sVEGFR1 and T-VEGFscFv at MOI of 0.01. After 24 and 48 hours of culture, the titer of the virus was measured. At both after 24 and 48 hours, the titer of replicated virus in the T-sVEGFR1 and T-VEGFscFv infected groups was approximately equivalent to that of the T-01 infected group. n = 3; Error bar, standard deviation.

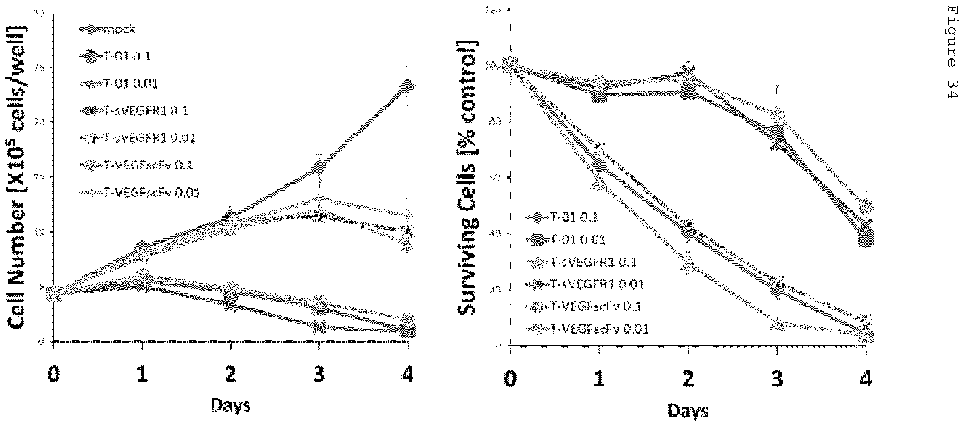

[Figure 34] A graph showing the results of in vitro cytopathic assays with HT-29 for T-sVEGFR1, T-VEGFscFv. HT-29 was seeded at 2 x 10⁵ cells/well in 6-well plates and infected with T-01, T-sVEGFR1 and T-VEGFscFv at MOI of 0.1 and 0.01. After infection, surviving cells were measured over 4 days, each after 24 hours, and the number of the surviving cells was calculated as a proportion relative to that in Mock. T-sVEGFR1 and T-VEGFscFv showed approximately equivalent cytopathic effects to T-01. n = 3; Error bar, standard deviation.

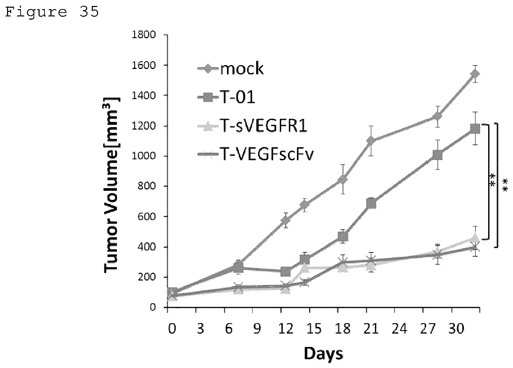

[Figure 35] A graph showing the anti-tumor effects of T-sVEGFR1 and T-VEGFscFv on HT-29 subcutaneous tumor models. HT-29 was subcutaneously administered at 1 x 10⁶ cells/50 µl to the left abdomen of BALB/c nu/nu to generate subcutaneous tumors. T-01, T-sVEGFR1, T-VEGFscFv and Mock were administered at 1 x 10⁶ pfu/20 µl into tumors on Day 0 and Day 3, twice. On Day 32, significantly strong anti-tumor effects were observed in the T-sVEGFR1 and T-VEGFscFv administration groups relative to the T-01 administration group. n = 8; Error bar, standard deviation; **, p < 0.01.

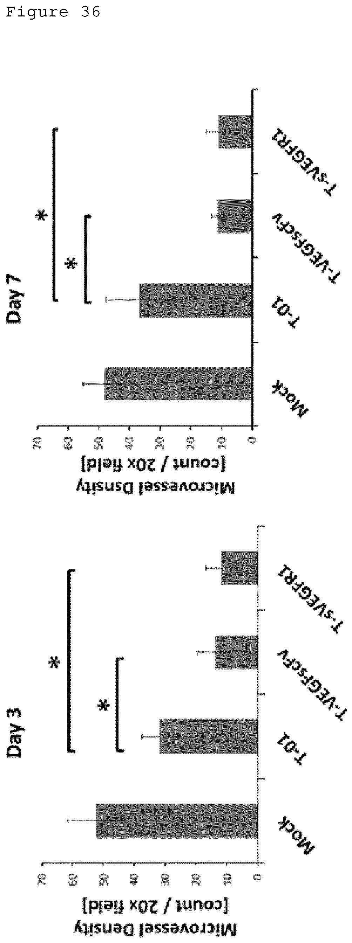

[Figure 36] A graph showing the intratumoral microvessel density of HT-29 subcutaneous tumors. CD31-positive cells of tissue sections of HT-29 subcutaneous tumors were counted and the intratumoral microvessel density was determined. As a result, intratumoral microvessels were significantly reduced in the T-sVEGFR1 and T-VEGFscFv administration groups relative to the T-01 administration group on both Day 3 and Day 7. n = 3; Error bar, standard deviation; *, p < 0.05.

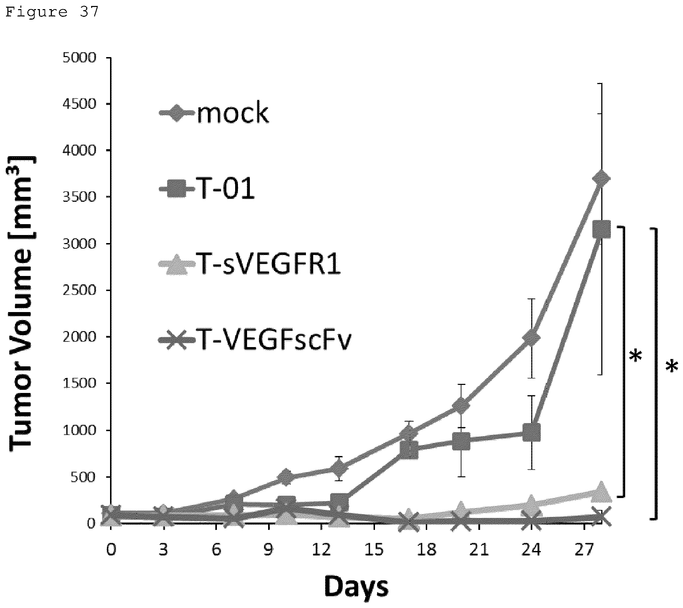

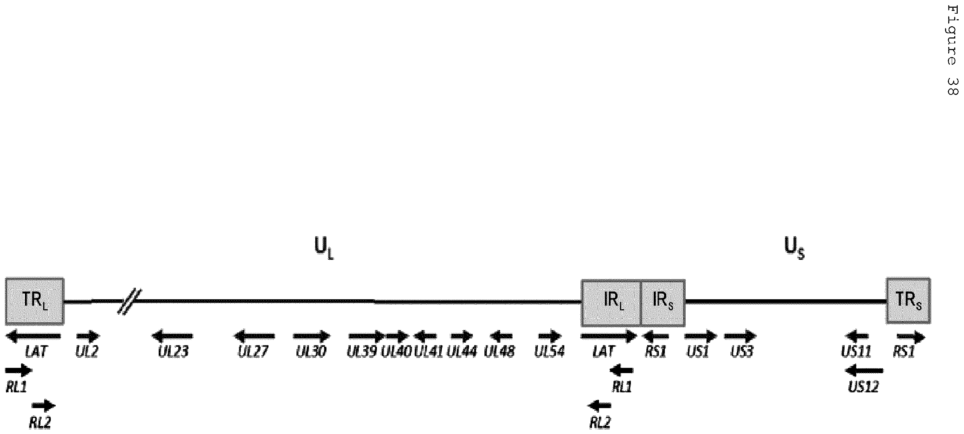

[Figure 37] A graph showing the anti-tumor effects of T-sVEGFR1 and T-VEGFscFv on CT26 subcutaneous tumors. CT26 was subcutaneously administered at 1 x 10⁵ cells/50 µl to the left abdomen of BALB/c mice to generate subcutaneous tumors. T-01, T-sVEGFR1, T-VEGFscFv and Mock were administered at 1 x 10⁶ pfu/20 µl into tumors on Day 0 and Day 3, twice. When the tumor volume was measured, significantly strong anti-tumor effects were observed on Day 28 in the T-sVEGFR1 and T-VEGFscFv administration groups relative to T-01. n = 7; Error bar, standard deviation; *, p < 0.05.

### Description of Embodiments

Hereinafter, the present invention is described in detail based on the embodiments of the present invention (also referred to as "the present embodiment"). The following embodiments are exemplifications to describe the present invention and there are no intentions to limit the present invention to the embodiments.

In one aspect, the present embodiment relates to an oncolytic virus containing a gene encoding a VEGF antagonist. As used herein, the VEGF antagonist refers to a molecule that neutralizes, blocks, reduces, or interferes with the biological activity of a vascular endothelial cell growth factor (VEGF), and includes a molecule that interferes with an interaction between VEGF and a VEGF receptor, for example, a molecule that inhibits the interaction between VEGF and a VEGF receptor by binding to the VEGF or the VEGF receptor, or otherwise interferes with the interaction. In one aspect, the VEGF antagonist of the present embodiment binds to the VEGF or VEGF receptor with a Kd of 1 nM to 1 µM, preferably 500 nM to 1 µM. Examples of the VEGF antagonist include an anti-VEGF antibody, an anti-VEGF receptor antibody, a VEGF receptor, a soluble VEGF receptor (sVEGFR), a peptide ligand, and a nucleic acid having a VEGF inhibitory effect. The VEGF antagonist is preferably an anti-VEGF antibody or sVEGFR.

In the present embodiment, "VEGF" includes not only human VEGF, but also non-human VEGF (e.g., VEGF of mouse, rat, and non-human primate). In one aspect, VEGF of the present embodiment is VEGF of an organism that is a subject of the virus administration of the present embodiment. For example, when the present embodiment is administered to a human, the VEGF is preferably human VEGF. In VEGF, there are seven types of growth factors, known as the VEGF family, namely VEGF-A, VEGF-B, VEGF-C, VEGF-D, VEGF-E, PLGF-1 and PLGF-2 (PLGF: placental growth factor), which have different physical and biological properties. In the present embodiment, VEGF is preferably VEGF-A that is involved in the proliferation of vascular endothelial cells, the promotion of tube formation, and the like.

In one aspect, the VEGF antagonist of the present embodiment is an anti-VEGF antibody that binds to VEGF with sufficient affinity and specificity. As the anti-VEGF antibody, a commercial product (e.g., bevacizumab, ranibizumab, ramucirumab) may be used, or other known anti-VEGF antibody may be used. Bevacizumab is an antibody that inhibits binding of VEGF-A to its receptors (VEGFR-1 and VEGFR-2) by specifically binding to VEGF-A. Ranibizumab is a Fab fragment of a monoclonal antibody against vascular endothelial growth factor-A (VEGF-A). Ramucirumab is an antibody to VEGFR-2, and inhibits signaling due to a VEGF ligand by inhibiting binding of VEGFR-2 to VEGF-A, and even to VEGF-C and D. An anti-VEGF antibody prepared by a method known to those skilled in the art using a VEGF antigen may also be used as the anti-VEGF antibody. In one aspect, the VEGF antagonist of the present embodiment is bevacizumab.

In one aspect, the anti-VEGF antibody of the present embodiment is preferably an antibody that binds to VEGF-A and/or an antibody that inhibits binding of VEGF to VEGFR-1.

In one aspect of the present embodiment, when the VEGF antagonist is an anti-VEGF antibody, the antibody may be a full-length antibody or a fragment thereof. The antibody may be a polyclonal antibody or a monoclonal antibody, preferably a monoclonal antibody. In one aspect, the type of the antibody is not particularly limited, and examples thereof include a human antibody, a humanized antibody, a chimeric antibody, an antibody derived from other animal (e.g., a mouse antibody, a rat antibody, a rabbit antibody, a sheep antibody, a camel antibody, a chicken antibody) and any other antibodies. The antibody against an organism that is the same as the tumor that the oncolytic virus containing a gene encoding the antibody targets to lyse can be preferably used. For example, when the oncolytic virus is a human oncolytic virus, a human antibody or a humanized antibody to human VEGF can be used. Any of the antibodies can be prepared using known methods. Alternatively, known antibodies can be used.

In one aspect of the present embodiment, the VEGF antagonist may be a fragment of an anti-VEGF antibody. As used herein, the fragment of an antibody means either an antibody fragment itself or one in which any molecule is attached to the antibody fragment, which recognizes the same epitope as the original antibody. The fragment of an antibody is not particularly limited as long as it includes the complementarity determining region (CDR) of the original antibody, and specific examples thereof include a Fab fragment consisting of V_{L}, V_{H}, C_{L}, and C_{H} regions; a Fab' fragment further including a hinge region; a F(ab')2 fragment in which two Fabs are linked by a disulfide bond at a hinge region; Fv consisting of V_{L} and V_{H}; and scFv, a single-stranded antibody in which V_{L} and V_{H} are linked by an artificial polypeptide linker. Further examples thereof include, but are not limited to, sdFv, a diabody and sc(Fv)2, and they are not particularly limited as long as they have two variable regions constituting the antigen binding site of the antibody, a heavy chain variable region (V_{H}) and a light chain variable region (V_{L}).

Any of these VEGF antagonists can be prepared by known methods. For example, scFv can be prepared by ligating V_{H} and V_{L} with a known linker (such as a GS linker (SEQ ID NO: 11)).

In one aspect, the antibody or a fragment thereof preferably has a long half-life in the tumor cell. For example, when compared with a single-stranded antibody, a double-stranded antibody has a longer half-life due to the presence of the Fc moiety, and is expected to have an antibody-dependent immune response.

In one aspect, the oncolytic virus of the present embodiment is a virus that contains a gene encoding a polypeptide of any one of the following (i) to (iii):
(i) a polypeptide of SEQ ID NO: 2;
(ii) a polypeptide consisting of an amino acid sequence in which one or several amino acids are deleted, substituted or added in the polypeptide of SEQ ID NO: 2, and having VEGF binding ability;
(iii) a polypeptide that has 75% or more, 80% or more, 85% or more, 90% or more, 95% or more, 98% or more, or 99% or more homology to the polypeptide of SEQ ID NO: 2, and has VEGF binding ability;
and a gene encoding a polypeptide of any one of the following (iv) to (vi):
(iv) a polypeptide of SEQ ID NO: 4;
(v) a polypeptide consisting of an amino acid sequence in which one or several amino acids are deleted, substituted or added in the polypeptide of SEQ ID NO: 4, and having VEGF binding ability;
(vi) a polypeptide that has 75% or more, 80% or more, 85% or more, 90% or more, 95% or more, 98% or more, or 99% or more homology to the polypeptide of SEQ ID NO: 4, and has VEGF binding ability,
and expresses the polypeptide.

In one aspect, the oncolytic virus of the present embodiment is a virus that contains a gene encoding a polypeptide of any one of the following (vii) to (ix):
(vii) a polypeptide of SEQ ID NO: 30;
(viii) a polypeptide consisting of an amino acid sequence in which one or several amino acids are deleted, substituted or added in the polypeptide of SEQ ID NO: 30, and having VEGF binding ability;
(ix) a polypeptide that has 75% or more, 80% or more, 85% or more, 90% or more, 95% or more, 98% or more, or 99% or more homology to the polypeptide of SEQ ID NO: 30, and has VEGF binding ability
and a gene encoding a polypeptide of any one of the following (x) to (xii):
(x) a polypeptide of SEQ ID NO: 31;
(xi) a polypeptide consisting of an amino acid sequence in which one or several amino acids are deleted, substituted or added in the polypeptide of SEQ ID NO: 31, and having VEGF binding ability;
(xii) a polypeptide that has 75% or more, 80% or more, 85% or more, 90% or more, 95% or more, 98% or more, or 99% or more homology to the polypeptide of SEQ ID NO: 31, and has VEGF binding ability,
and expresses the polypeptides.

In one aspect, it is preferable that the oncolytic virus of the present embodiment further contains genes encoding polypeptides of Furin recognition sequence (SEQ ID NO: 8) and FMDV-2A (SEQ ID NO: 9). It is more preferable that the oncolytic virus of the present embodiment contains the genes encoding polypeptides of SEQ ID NOs: 8 and 9 between the gene encoding a polypeptide of any one of (vii) to (ix) and the gene encoding a polypeptide of any one of (x) to (xii).

In one aspect, the VEGF antagonist of the present embodiment is a soluble VEGF receptor (sVEGFR). For sVEGFR, sVEGFR-1, -2 and -3 are known. In one aspect, sVEGFR is preferably sVEGFR-1 that has binding ability to VEGF-A and VEGF-B. As sVEGFR, a known sVEGFR can be used. For example, sVEGFR described in Park JE et al. (1994) J Biol Chem. Vol. 269(41): 25646-54 or a commercial product of sVEGFR can be used.

In one aspect, the oncolytic virus of the present embodiment is a virus that contains a gene having a polynucleotide of any one of the following (xiii) to (xv), and expresses the polypeptide encoded by the polynucleotide:
(xiii) a polynucleotide encoded by a nucleotide sequence of SEQ ID NO: 26;
(xiv) a polynucleotide consisting of a nucleotide sequence in which one or several nucleotides are deleted, substituted or added in the polynucleotide encoded by the nucleotide sequence of SEQ ID NO: 26, and encoding a polypeptide that has VEGF binding ability;
(xv) a polynucleotide that has 75% or more, 80% or more, 85% or more, 90% or more, 95% or more, 98% or more, or 99% or more homology to the polynucleotide encoded by the nucleotide sequence of SEQ ID NO: 26, and encodes a polypeptide that has VEGF binding ability.

When referring to "amino acid sequence in which one or several amino acids are deleted, substituted or added" herein, the number of amino acids deleted, substituted or added is not particularly limited as long as the polypeptide consisting of the amino acid sequence has VEGF binding ability, and examples of the number include 1 to 5, 1 to 3, or 1 to 2. The place where one or several amino acids are deleted, substituted or added may be at the end or at the intermediate of the peptide, and the number of the places may be one, or two or more.

Similarly, when referring to "nucleotide sequence in which one or several nucleotides are deleted, substituted or added" herein, the number of nucleotides deleted, substituted or added is not particularly limited as long as the polypeptide encoded by the nucleotide sequence has VEGF binding ability, and examples of the number include 1 to 5, 1 to 3, or 1 to 2. The place where one or several nucleotides are deleted, substituted or added may be at the end or at the intermediate of the polynucleotide, and the number of the places may be one, or two or more.

Whether the polypeptide has VEGF binding ability or not can be determined using methods known to those skilled in the art. For example, the ELISA described in the Examples below can be used to determine whether the polypeptide has VEGF binding ability or not.

In the present embodiment, the amino acid may be a natural amino acid, a derivative thereof, an artificial amino acid, or a non-natural amino acid. In one aspect, the amino acid of the present embodiment is preferably a natural amino acid.

The oncolytic virus of the present embodiment contains a gene encoding a VEGF antagonist. When the oncolytic virus is administered to a tumor cell, the VEGF antagonist is expressed along with the viral replication. To modify an oncolytic virus to express a gene encoding a VEGF antagonist, known methods can be used. Examples of the methods include a method including inserting a DNA encoding a VEGF antagonist into an expression vector by a known method (such as a method utilizing a restriction enzyme) and transfecting the expression vector into an oncolytic virus. The expression vector can further contain a promoter that modulates expression of the gene of interest, a replication origin, a selection marker gene, and the like. The promoter and the origin of replication can be appropriately selected depending on the types of the oncolytic virus and the vector to which the gene is introduced.

For example, when the VEGF antagonist is an anti-VEGF antibody or a fragment thereof, an amino acid sequence or a nucleotide sequence of the antibody to be expressed may be obtained and the region of interest may be expressed by a known method. For example, sequences encoding polypeptides corresponding to the heavy and light chains of an antibody may be introduced so that the heavy and light chains are expressed in equivalent amounts from cDNA to allow efficient antibody production, and preferably both a foot-and-mouth disease (FMDV)-2A sequence and an amino acid sequence encoding a furin cleavage site with self-cleaving activity may be co-introduced to modify the virus. Furthermore, each of the polypeptides corresponding to the heavy and light chains may have a signal sequence for secretion at the N-terminus. Figure 1 shows an example of a mechanism for expressing an antibody from such antibody expression gene.

The site of introduction of the above gene is not particularly limited as long as the oncolytic virus maintains the oncolytic ability, and a gene encoding a VEGF antagonist can be introduced, for example, at the site where a non-essential gene of the virus is deleted. When the virus is HSV-1, the non-essential gene shown in Table 1 below can be deleted to introduce a gene encoding a VEGF antagonist. The expression of the introduced gene can be confirmed by known methods, as shown in the Examples below.

In particular, when the oncolytic virus is a large genome-sized virus such as herpes simplex virus (HSV), T-BAC system described in Fukuhara, H. et al. (2005). Cancer Research, 65, 10663-10668 or WO 2005/103237, or an equivalent thereto can be used. When the T-BAC system is used for an HSV virus, the method including: a first step of inserting a BAC plasmid having a loxP site and an FRT site as well as at least one marker gene expression cassette inserted between the loxP site and the FRT site into an HSV genome; a second step of making a shuttle vector in which at least one expression cassette of a gene encoding a protein of interest, at least one marker gene, a loxP site and a FRT site are each inserted, and inserting the shuttle vector into a loxP site of an HSV genome using a Cre recombinase; and a third step of co-infecting a host with the HSV genome and a vector capable of expressing a Flp recombinase, excising a region sandwiched by the FRT sites on the genome, and generating a genetically modified HSV of interest, can be used to easily generate a virus that can express a VEGF antagonist of interest in tumor cells.

### (Oncolytic virus)

In the present embodiment, an oncolytic virus (also referred to as an anti-cancer virus) is a virus that can be used to treat a cancer, and is not particularly limited as long as the virus has the ability to infect tumor cells, replicate selectively in tumor cells, destroy tumor cells during the virus replication process, infect other surrounding tumor cells, and further replicate. As the oncolytic virus, a known virus can be used. In many cases, such a virus is a variant of a naturally occurring virus, which is genetically modified to increase tumor selectivity. The virus may be attenuated as needed, and modified to enhance anti-tumor activity (such as by incorporation of a suicide gene).

Examples of the oncolytic virus that may be used in the present embodiment include a virus or a viral variant selected from the group consisting of herpes simplex virus type I and type II (HSV-1 and HSV-2), adenovirus, poliovirus, measles virus, reovirus, vaccinia virus, seneca virus (seneca valley virus), vesicular stomatitis virus (VSV), Newcastle disease virus, coxsackievirus. In order to express the antibody gene, a virus that has a genome in a size in which the antibody can be inserted is preferred (because, in general, only about 1/10 of the genome can be modified), and HSV-1 and HSV-2 and vaccinia viruses can be preferably used.

Examples of the oncolytic virus which is an adenovirus variant include ONYX-015 (Khuri et al. (2000). Nat. Med 6(8): 879-85, etc.), H101 (Oncorine) (Xia et al. (2004). Ai Zheng 23(12): 1666-70), and Telomelysin (OBP-301) (Kawashima T. et al. (2004) Clin Cancer Res. 10: 285-292).

Examples of the oncolytic virus which is a poliovirus variant include those described in Goetz et al. (2010). Cytokine & Growth Factor Reviews 21 (2-3): 197, and in Lal, R. et al. (2009). Current opinion in molecular therapeutics 11 (5): 532-9.

Examples of the oncolytic virus which is a measles virus variant include MV-Edm (McDonald et al. (2006). Breast Cancer Treat. 99(2): 177-84) and MV-CEA, MV-NIS, etc., using MV-Edm, and HMWMAA (Kaufmann et al. (2013). J. Invest. Dermatol. 133(4): 1034-42).

Examples of the oncolytic virus which is a reovirus include Reolysin (Oncolytic Biotech).

Examples of the oncolytic virus which is a vaccinia virus variant include MDRVV002 (miRNA-dependent recombinant vaccinia virus 002) and MDRVV003 (MAPK-dependent recombinant vaccinia virus 003), and those described in D C Mansfield et al. (2016). Gene Therapy, 23, 357-368, and in Steve H. Thorne (2014). Frontiers in Oncology, 4:155.

Examples of the oncolytic virus which is a seneca virus include NTX-010 (Rudin et al., (2011). Clin. Cancer. Res. 17(4): 888-95).

Examples of the oncolytic virus which is a vesicular stomatitis virus (VSV) variant include viruses described in Stojdl et al. (2000). Nat. Med. 6(7): 821-5, and in Stojdl et al. (2003). Cancer Cell 4(4): 263-75.

Examples of the oncolytic virus which is a Newcastle disease virus include the strains 73-TPV701 and HDV-HUJ, and viruses described in Phuangsab et al. (2001). Cancer Lett. 172(1): 27-36, Lorence et al. (2007). Curr. Cancer Drug Targets 7(2): 157-67, and Freeman et al. (2006). Mol. Ther. 13(1): 221-8.

Examples of the oncolytic virus which is a coxsackievirus variant include CVA21 belonging to coxsackie A (CVA) and a variant thereof (Kelly EJ. Et al. (2008) Nat Med, 14: 1278-1283), and viruses belonging to coxsackievirus type B3 (CVB3) (Miyamoto S. et al. (2012) Cancer Res. 72: 2609-2621).

In one aspect of the present embodiment, the oncolytic virus is an HSV variant, preferably an HSV-1 variant. When the oncolytic virus is an HSV-1 variant, the oncolytic HSV-1 variant can be generated, for example, with reference to Patent Literatures 1 to 3. HSV-1 is classified as a double-stranded DNA virus with an envelope, and has the following characteristics in favor of cancer treatment: 1) it is infectious to all types of cells in human; 2) the life cycle and the genome sequence of the virus have been elucidated; 3) the functions of the majority of the viral genes have become clear and genetic manipulation can be applied; 4) the viral genome is large (about 152 kb), thus a large gene or multiple genes can be incorporated. Furthermore, HSV-1 has the following advantages suitable for clinical application: 5) it is possible to kill all cells with a relatively low multiplicity of infection (MOI); 6) there are antiviral drugs to suppress the proliferation; 7) anti-HSV-1 antibodies in blood do not affect the spread of virus infection from cell to cell and thus repeated administration thereof is possible; 8) preclinical evaluation of safety and effects can be performed in animals due to the existence of mice and monkeys that are sensitive to HSV-1; 9) viral DNA is not captured in the genome of the host cell and is present outside the chromosome.

The genome of HSV-1 consists of two unique sequence regions: 82% long unique-region (U_{L}) and 12% short unique-region (U_{S}), and inverted repeat sequences of terminal repeat (TR) and inverted repeat (IR) located at each end of U_{L} and U_{S} (Table 1, Todo, T. (2008). a journal and virtual library 13, 2060-2064). Since each of the two regions L and S can take two directions independently, the HSV-1 genomic DNA has four isomers. This genome contains a total of 84 genes encoded unidirectionally in RL1 and RL2 on TR_{L}, UL1-UL56 on U_{L}, RS1 on TR_{S}, and US1-US12 on U_{S}; about half of which are genes unnecessary for viral growth, and the deletion of these non-essential gene moieties can reduce pathogenicity and allow gene transduction (Carson, J. et al. (2010). Drugs of the Future 35, 183-195).

**Table 1**

| **Gene** | **Essential (E) or non-essential (N)** | **Protein** | **Function** |
|---|---|---|---|
| *LAT* | | LAT | Only gene that expresses during latent infection |
| *RL1* | N | ICP34.5 | Neuropathogenicity, apoptosis suppression |
| *RL2** | N | ICP0 | Promoting Transcription |
| *UL2* | E | UNG | Encoding uracil-DNA glycosylase required for DNA synthesis |
| *UL23* | N | ICP36 (Thymidine kinase) | Encoding thymidine kinase required for DNA synthesis |
| *UL27* | E | gB | Entry into cells |
| *UL30* | E | DNA polymerase | Catalytic subunit of DNA polymerase |
| *UL39* | N | ICP6 | Encoding large subunit of ribonucleotide reductase (RR) required for DNA synthesis |
| *UL40* | | | Encoding small subunit of RR |
| *UL41* | N | vhc (virion-induced host shutoff) | Degradation of host mRNA |
| *UL44* | N | gC | Adsorption to cells |
| *UL48* | E | VP16 | Promotion of immediate-early gene expression |
| *UL54** | E | ICP27 | Regulation of DNA synthesis and transcription, suppression of host protein expression by splicing suppression |
| *RS1** | E | ICP4 | Regulations of β and γ genes, apoptosis suppression |
| *US1** | N | ICP22 | Transcription regulation |
| *US3* | N | | Apoptosis suppression, capsid transportation from nucleus to cytoplasm, regulation of gB expression |
| *US11* | N | | Inhibition of protein kinase R activation under early promoter |
| *US12** | N | ICP47 | Inhibition of antigen presentation of host |

| | | | |
|---|---|---|---|
| * indicates an immediate-early gene | | | |

When the oncolytic virus in an HSV-1 variant, it may have one or more of the following characteristics:
- Acquisition of tumor-cell-specific replication ability by inactivation of enzymes involved in viral DNA synthesis, such as thymidine kinase (TK), ribonucleotide reductase (RR), and uracil-N-glycosylase (UNG or UDG).
- Acquisition of tumor-cell-specific replication ability by deleting gene γ34.5 encoding protein ICP 34.5 involved in the pathogenicity of HSV-1.
- Acquisition of an anti-tumor effect by deleting α47.
- Deletion or inactivation of a gene for preventing return to the wild type and increasing safety (e.g. deletion or inactivation of intrinsic γ34.5 gene, ICP47 gene, α0 gene (ICP0 gene), U_{L}41 gene (vhs gene), U_{L}56 gene) .
- Enhancement of anti-tumor immunity and prolonged survival by expressing an immunostimulatory gene (IL-4, IL-10, GM-CSF, IL-12, soluble B7.1, or the like).
- Enhancement of angiogenesis suppression and anti-tumor effects by expressing a gene expressing an angiogenesis inhibitory factor such as platelet factor IV, thrombospondin, endostatin, dominant negative FGF, or angiostatin.
- Enhancement of anti-tumor effect by expressing a metalloproteinase inhibitor involved in local infiltration of the tumor.
- Promotion of the spread of virus infection by overexpressing metalloproteinase.
- Enhancement of tumor-cell-specific viral replication ability by regulating a viral gene with a tumor- or tissue-specific promoter (such as calponin promoter, E2F-reactive cell cycle dependent promoter B-myb, Nestin promoter, a cancer fetal antigens (CEAs), alpha-fetoprotein (AFP), MUC-1, or Musashi enhancers/promoter).

In one aspect, the oncolytic virus of the present embodiment is preferably an HSV-1 having one or more characteristics of the following (a) to (c), more preferably having all characteristics of the following (a) to (c). HSV-1 variants with these characteristics can be generated with reference to Patent Literature 3, or the like.
(a) ICP6 gene is deleted or inactivated, or expressed under control of a tumor-specific promoter or a tissue-specific promoter.
(b) γ34.5 gene is deleted or inactivated.
(c) ICP47 gene is deleted or inactivated.

Regarding the characteristics of (a) and (b) above, the deletion of ICP6, a large subunit of RR, an enzyme important for nucleotide metabolism and viral DNA synthesis in non-dividing cells, and/or γ34.5, which antagonizes the function of phosphorylated PKR that occurs upon viral infection, results in a virus that cannot proliferate in normal cells but can only proliferate in tumor cells where cell division is frequent, RR activity is elevated and PKR phosphorylation is suppressed. Also with respect to the characteristic of (c) above, it is expected that, since the protein encoded by the α47 gene has the effect of escaping the host's immunosurveillance by inhibiting TAP to suppress expression of MHC class I on the surface of the host cell, α47 gene-deleted HSV-1 has an increased stimulation to the immune cells due to the retention of MHC class I expression of the host cells. Furthermore, in HSV-1 that has both of the characteristics of (b) and (c) above, the deletion of α47 results in simultaneous deletion of the US11 late promoter whose genome overlaps with α47, so that the expression of the US11 gene becomes earlier because the gene is under the control of the ICP47 immediate-early promoter, thereby HSV-1 has the effect of restoring the viral replication ability, which was attenuated by γ34.5 deletion, only in tumor cells.

As used herein, deletion or inactivation of a gene refers to deletion of all or a part of the gene or suppression of the gene expression by substituting or modifying some nucleotides or inserting an unnecessary sequence, or the like, and can be performed by known methods.

As used herein, a tumor-specific promoter or tissue-specific promoter means a promoter that specifically allows the expression of a gene under its control in a tumor cell or tissue of interest. A known promoter can be used as the tumor-specific promoter or tissue-specific promoter depending on the gene. For example, a telomerase reverse transcriptase promoter (hTERT promoter) or an E2F promoter can be used.

Examples of the HSV having deletion or inactivation of γ34.5 gene, ICP6 gene and/or ICP47 gene described in (a) to (c) above include G207 having deletion of two copies of γ34.5 genes and inactivation of ICP6 gene, and G47Δ having deletions of two copies of γ34.5 gene, inactivation of ICP6 gene, and deletion of ICP47 gene. Thus, the oncolytic virus of the present embodiment can also be generated by further modifying G207 or G47Δ.

In particular, G47Δ is an oncolytic HSV-1 that improves safety while significantly improves tumor-cell selective viral replication and the elicitation of anti-tumor immunity, and due to the broad therapeutic range, G47Δ can be administered safely to human brain at high doses (the phase II study is undergoing).

### (Pharmaceutical composition)

One aspect of the present embodiment relates to a pharmaceutical composition containing the oncolytic virus of the present embodiment described above. The pharmaceutical composition of the present embodiment specifically proliferates in tumor cells and also suppresses the swelling. Thus, repeated administration of the pharmaceutical composition can also be readily performed.

Also, in one aspect, according to the pharmaceutical composition of the present embodiment, as shown in the Examples below, a high tumor treatment effect is obtained because the dominant state of M1 macrophage having a strong anti-bacterial activity, an anti-viral activity, and an anti-tumor effect is maintained relative to the M2 macrophage, and the viral titer of the oncolytic virus in the pharmaceutical composition is not reduced even in the M1 dominant state. In one aspect, the virus of the present embodiment has a high tumor treatment effect relative to an oncolytic virus that has the same configuration but without a gene encoding a VEGF antagonist. Measurement of the viral titer, comparison of M1 and M2 macrophages, and confirmation of anti-tumor effects (including cytopathic effect in vitro, prolonged survival effect in vivo) can be performed by known methods, as shown in the Examples below.

Furthermore, in one aspect, according to the pharmaceutical composition of the present embodiment, each time the oncolytic virus replicates in the tumor, a VEGF antagonist (e.g., an anti-VEGF antibody) is expressed locally in the tumor, thus the risk of side effects, such as wound healing inhibition, is reduced relative to systemic administration of the VEGF antagonist. Confirmation of replication ability of the virus and expression amount of the VEGF antagonist can be performed by known methods, as shown in the Examples below.

Examples of tumors in which the pharmaceutical composition of the present embodiment is effective include, nervous system tumors (e.g., astrocytoma, oligodendroglioma, meningioma, neurofibroma, glioblastoma, ependymoma, neurilemmoma, neurofibrosarcoma, neuroblastoma), pituitary tumors (e.g., pituitary adenoma), medulloblastoma, melanoma, a brain tumor, a prostate cancer, a head and neck cancer, an esophageal cancer, a kidney cancer, a kidney cell cancer, a pancreatic cancer, a breast cancer, a lung cancer, a colorectal cancer, a colon cancer, a gastric cancer, a skin cancer, an ovarian cancer, a bladder cancer, sarcoma (e.g., osteosarcoma, chondrosarcoma, rhabdomyosarcoma, leiomyosarcoma, fibrosarcoma, liposarcoma, angiosarcoma), a squamous cell cancer, neuroectodermal tumor, a thyroid tumor, lymphoma, a hepatocellular carcinoma, mesothelioma, an epidermoid cancer, and benign tumors (such as a benign thyroid tumor, or a benign prostate tumor).

The pharmaceutical composition of the present embodiment suppresses the swelling and thus allows the treatment of the tumor without causing the swelling as well as the various symptoms associated with the swelling. For example, symptoms associated with swelling in the brain include not only minor symptoms, such as headaches, but also serious symptoms, such as paralysis, aphasia, and consciousness disorders due to compression to surrounding normal tissues and increased intracranial pressure. According to the pharmaceutical composition of the present embodiment, treatment of tumors can be performed without causing such symptoms, without causing increased intracranial pressure or temporary worsening of neurological symptoms due to the treatment of the tumor, and without lowering the patient's QOL. In one aspect, the pharmaceutical composition of the present embodiment is particularly effective in treating a tumor in the cranial cavity, including brain tumors, that have little extracranial metastasis and for which topical treatment are important.

In the present embodiment, swelling includes both swelling of the tumor itself and edema around the tumor, and refers to a condition in which the tumor and/or around the tumor has an increased volume and expanded. In the present embodiment, "swelling-suppressive" refers to one or more of suppressing the occurrence of swelling, reducing the size of swelling that occurs, and preventing the expansion of swelling. Although not bound by theory, the present inventor has found that the swelling upon administration of an oncolytic virus is particularly problematic when it is a swelling of the tumor itself. The virus of the present embodiment can suppress swelling upon administration of the oncolytic virus, including such swelling of the tumor itself. When the oncolytic virus is administered topically to the affected area by injection or the like, swelling occurs immediately after administration. Repeated topical administration to the same site while this swelling persists causes further expansion of the swelling. In contrast, the virus of the present embodiment suppresses the swelling, thereby allowing for safer and easier treatment of the tumor.

Along with the swelling, edema (a condition in which fluid is accumulated to cause an expansion) may also occur. Since edema cannot be always clearly distinguished from swelling in appearance, the extent of swelling can also be confirmed by measuring the extent of edema in the present embodiment. That is, the extent of swelling can be measured by methods known to those skilled in the art in the measurements of swelling and edema. As shown in the Examples below, the extent of swelling (edema) can be confirmed, for example, by comparison of the high intensity area in T2-enhanced MRI. The extent of swelling and/or edema can also be confirmed by quantifying substances which has been confirmed to be related to swelling and/or edema. For example, with respect to swelling in the brain, the extent of swelling and/or edema can be confirmed by quantifying aquaporin (AQP)1, AQP3, AQP4, AQP5, AQP8, AQP9, AQP11, AQP12 (in particular AQP4) which are known to be related to brain edema.

The method of administering the pharmaceutical composition of the present embodiment is not particularly limited, and the pharmaceutical composition of the present embodiment may be administered, for example, intravenously, intraarterially, intraventricularly, intraperitoneally, intrathoracically, intraspinal cavity, subcutaneously, intradermally, intraepidermally, intramuscularly, or into a mucosal surface (e.g., eye, intranasal, lung, oral, intestinal, rectal, vaginal, urinary tract surface). Preferably, it is administered topically directly to the tumor tissue by injection, endoscopy or surgical method. Topical administration reduces the risk of side effects, such as wound healing inhibition, relative to systemic administration of the VEGF antagonist, because the VEGF antagonist is expressed locally in the tumor, and has the advantage that, even when an oncolytic virus is administered by a surgical method, the healing of wounds associated with the surgical method is not inhibited.

The pharmaceutical composition of the present embodiment can be formulated by a known formulation method for introducing a virus into a body of mammals including human. For example, the pharmaceutical composition may contain an adjuvant or any carrier, or may be one simply diluted with a physiologically acceptable solution such as sterile saline or sterile buffered saline without addition of an adjuvant or carrier. It may be formulated in a frozen formulation, a dried formulation, a lyophilized formulation, or the like which are suitable for long-term storage.

The pharmaceutical composition of the present embodiment contains a therapeutically effective amount of the oncolytic virus of the present embodiment. As used herein, a therapeutically effective amount means an amount of an agent in which one or more symptoms of the tumor to be treated are thereby relieved to some extent. More specifically, a therapeutically effective amount means an amount causing at least one of reduction of tumor size, inhibition (delay or arrest) of tumor metastasis, inhibition (delay or arrest) of tumor growth, and relief of one or more symptoms associated with the tumor. The specific dosage can be appropriately determined by those skilled in the art depending on the degree of symptoms; age, sex, weight, difference in susceptibility of the subject to be administered; method of administration; timing of administration; dosing interval; properties of formulation; strength of promoter; and the like.

The pharmaceutical composition of the present embodiment can be administered, for example, at about 10¹ to about 10¹² plaque forming units (pfu), preferably at about 10⁷ to about 10¹⁰ pfu, still more preferably at about 10⁸ pfu to about 5 x 10⁹, in one or several portions, respectively, by injection. The number of administrations of the pharmaceutical composition of the present embodiment can be appropriately set depending on the patient to be administered and the target disease. For example, the pharmaceutical composition may be administered within two weeks for the first two times, and thereafter, administered with intervals of 3-5 weeks. Since the pharmaceutical composition of the present embodiment is a swelling-suppressive composition, it has advantages that, even when administered multiple times, there are no side effects such as swelling increase at each time of administration, and no inconvenience such as refraining from the next administration until the swelling upon administration is reduced.

The pharmaceutical composition of the present embodiment can further contain other active ingredient, and can also be used in combination with a pharmaceutical composition containing other active ingredient. Furthermore, the pharmaceutical composition of the present embodiment can be used in combination with other tumor therapies, for example, a surgery (tumor resection, or the like), a chemotherapy, a radiation therapy, an immunotherapy, a hormone therapy, and a combination thereof. Since the viral therapy by the pharmaceutical composition of the present embodiment has a different mechanism from the existing tumor therapy, the combination use thereof with other therapy such as a radiation therapy or a chemotherapy can enhance the tumor treatment effect.

The present embodiment also relates to a method for treating a tumor, using the swelling-suppressive oncolytic virus. The present embodiment also relates to a use of the swelling-suppressive oncolytic virus in the manufacture of a medicament for treating a tumor. The method and use can be performed with reference to the descriptions of the pharmaceutical composition above.

Unless otherwise specified, all terms used herein (including technical and scientific terms) has the same meaning as widely understood by those skilled in the art to which the present invention belongs. The terms used herein are, unless otherwise specifically defined, to be construed as having consistent meanings with the meanings in the present description and in the related art, and are not to be interpreted in an idealized or excessively formatted sense.

### Examples

### A. Materials

### 1. Cell line and medium

African green monkey kidney cell line Vero, human colorectal cancer cell line HT-29, mouse colorectal cancer cell line CT26, human glioma cell line U87MG, U251 MG, NMC-G1, human embryonic kidney cell line HEK293T, human umbilical vein endothelial cell HUVEC (BD™ HUVEC-2), human umbilical vein endothelial cell HUVEC-2, and human glioma stem cell lines TGS-01, TGS-04 and 1123/M were used. Vero, HT-29, CT26 and U87MG were all purchased from the American Type Culture Collection (ATCC). HEK293T was purchased from the Riken BioResource Research Center. HUVEC was purchased from BD Bioscience (USA). HUVEC-2 was purchased from Corning Life Sciences. NMC-G1 was purchased from JCRB cell bank. TGS-01 and TGS-04 are glioma stem cell lines established from the excision tissues of glioblastoma patients who had undergone tumor removal by craniotomy at the Neurosurgery Department of the University of Tokyo Hospital, by culturing the tissues in serum-free medium and separating the cells with cancer stem cell markers and evaluation of sphere (floating cell mass) forming capacity by limiting dilution method. The TGS-01 and TGS-04 cell lines stored in the inventor's laboratory were used. 1123/M is a stem cell line established from the excision tissue of oligodendroglioma patients. A 1123/M cell line transferred from Ohio State University was used. G47Δ is an oncolytic HSV-1 virus, and the virus stored in the inventor's laboratory was used.

Vero, U87MG, U251MG and NMC-G1 were cultured in a Dulbecco's Modified Eagle Medium (DMEM) (Gibco) supplemented with 10% fetal bovine serum (FBS) (Sigma-Aldrich Co. LLC.). HT-29 was cultured in a McCoy's 5A (Modified) Medium supplemented with 10% FBS, and CT26 was cultured in a RPMI 1640 Medium supplemented with 10% FBS. HUVEC and HUVEC-2 were cultured in a Medium 200PRF (Gibco) supplemented with 1% Low Serum Growth Supplement (LSGS) (Gibco). TGS-01 and TGS-04 were cultured in a medium prepared from 500 mL of DMEM/F12 (1:1) (Gibco) supplemented with 10 mL of B-27 (Gibco), 7 mL of 45% D-Glucose (Sigma-Aldrich Co. LLC.), 5 mL of 200 mM L-glutamin (Gibco), and 1.875 mL of Insulin (Gibco), with the addition of 0.001 volume of EGF (Peprotech) (20 ng/mL) and 0.001 volume of bFGF (Peprotech) (20 ng/mL) before use (hereinafter, "SCM"). 1123/M was cultured in a medium prepared from 500 mL of DMEM/F12 (1:1) supplemented with 10 mL of B-27, 5 mL of 200 mM L-glutamin, and 550 µL of Heparin (5 mg/mL) (Sigma-Aldrich Co. LLC.), with the addition of 0.001 volume of EGF (20 ng/mL) and 0.001 volume of bFGF (Peprotech) (20 ng/mL) before use. The EGF and bFGF were added into the medium once every three days. All cells were cultured in an incubator at 5% CO₂ and 37°C. A VP-SFM (Gibco) supplemented with 1% L-glutamin (Gibco) was used as a serum-free medium for virus culture and an Opti-MEM (Sigma-Aldrich Co. LLC.) was used as a co-transfection medium.

### 2. Virus

The representative structures of the viruses generated and used in this Example are shown in Figure 2. Both viruses were generated using G47Δ, a genetically modified HSV-1, as a basic backbone. T-01 is a control virus in which a foreign gene is not introduced into the 894 bp deletion site of the ICP6 gene, and only LacZ and a cytomegalovirus (CMV) promoter are inserted. T-BV and T-V_{H}V_{L} generated in this study contain anti-human VEGF antibody expression genes and anti-VEGF single-stranded antibody (VEGF scFv) expression genes inserted downstream of the CMV promoter, respectively. Details of the inserted antibody expression genes and the methods for generating the viruses are described later.

### 3. E. coli, medium for E. coli and antibiotics

One Shot DH5α-T1 Competent Cells (Thermo Fisher Scientific) and One Shot PIR1 Competent Cells (Thermo Fisher Scientific) were used as E. coli for transfection, and ElectroMax DH10B Electrocompetent Cells (Thermo Fisher Scientific) were used as E. coli for electroporation. An SOC Medium (Thermo Fisher Scientific) was used as a transfection medium. An LB (Thermo Fishier Scientific) Medium was used for culture of E. coli. Kanamycin sulfate (Sigma-Aldrich Co. LLC.) and chloramphenicol (Sigma-Aldrich Co. LLC.) were used as antibiotics added to the medium.

### 4. Serum

Fetal Bovine Serum (FBS) (Sigma-Aldrich Co. LLC.) was used as a serum. Thermal deactivation treatment was performed at 56°C for 30 minutes.

### 5. Antibodies

An anti-human VEGF antibody, Bevacizumab (trade name: Avastin(registered trademark)), was purchased from Chugai Pharmaceutical Co., Ltd.

As antibodies for western blotting, a goat anti-human IgG- heavy and light chain monkey-absorbed antibody was purchased from Bethyl Laboratories, Inc., and a donkey anti-goat IgG-HRP conjugated was purchased from Santa Cruz.

As antibodies for ELISA, a Human VEGF (100-20), a Murine VEGF (450-32) and a Rabbit Anti-Murine VEGF Antibody (500-P131) were purchased from PeproTech, and a Goat anti-Human IgG-Fc Fragment Antibody HRP conjugated (A80-104P) and a Goat anti-Rabbit IgG-Fc Fragment Antibody HRP conjugated (A120-111P) were purchased from Bethyl Laboratories, Inc.

As antibodies for immunohistochemical staining, a rabbit anti-HSV-1 antibody (B0114, polyclonal) and an HRP-labeled anti-rabbit polymer (K4003) were purchased from Dako, a rat anti-mouse CD31 antibody (DIA-310, monoclonal) was purchased from Dianova, a rat anti-mouse F4/80 antibody (ab6640, monoclonal) was purchased from Abcam, and a Mouse MAX PO (Rat) (414311) was purchased from Nichirei.

As antibodies for flow cytometry analysis, a Purified Anti-Mouse CD16/32 Antibody (101301), a Pacific Blue Anti-Mouse CD45 Antibody (103126), an APC Anti-Mouse/Human CD11b Antibody (101212), a Brilliant Violet 570 Anti-Mouse Ly-6G/Ly-6C (Gr-1) Antibody (108431), and a PE/Cy7 Anti-Mouse CX3CR1 Antibody (149015) were purchased from BioLegend, and a Rat Anti-Mouse F4/80: RPE Antibody (MCA497PE) was purchased from Bio-Rad Laboratories, Inc.

### 6. Laboratory animals

Females of BALB/cnu/nu mice (nude mice) at 5-6 weeks of age were used. Mice were purchased from Charles River, Japan, or Japan SLC, Inc., and raised in specific pathogen free environments. Animal experiments were complied with the "Act on Welfare and Management of Animals", and followed the University of Tokyo Animal Experiment Implementation Manual of the University of Tokyo Bioscience Committee.

### B. Experimental method

### Example 1: Generation of viruses

T-BV, T-V_{H}V_{L} and T-sVEGFR1 were generated using T-BAC system. The T-BAC system is a genetically modified HSV-1 generation technique that facilitates maintenance and amplification of the G47Δ genome by inserting bacterial artificial chromosome (BAC), a replicon-based cloning vector of Factor F plasmid capable of stably holding a large DNA fragment up to 1 million bp in E. coli by a single copy, into the ICP6 deletion site of the G47Δ genome (Fukuhara, H. et al. (2005). Cancer Research, 65, 10663-10668). The T-BAC contains loxP and FRT sequences. Thus, by mixing the T-BAC with a shuttle vector plasmid SV-01 that similarly contains loxP and FRT sequences, and utilizing two recombinase systems, Cre/loxP and FLP/FRT, a foreign gene loaded on SV-01 is inserted and the BAC is excised, thereby G47Δ to which the foreign gene is inserted can be generated (Figure 3).

### Example 1.1: Generation of antibody-expressing cDNA

For cDNA of anti-human VEGF antibody expression genes, the amino acid sequence of the Fab region of bevacizumab and the amino acid sequence of the human IgG1 Fc region were obtained from the databases of the International ImMunoGeneTics information system (IMGT) and National Center for Biotechnology Information (NCBI), respectively. The polypeptides corresponding to the heavy and light chains, which harbored an Ig kappa leader sequence and a signal peptidase recognition site as a secretory signal at their N terminal side, were ligated using the foot-and-mouth-disease virus (FMDV)-2A sequence and the furin recognition sequence. The amino acid sequence and the overall structure are shown in Figure 4. Finally, the Kozak sequence and the start codon were inserted into the transcription start point, and both ends of the sequence were sandwiched with restriction enzyme sites BamHI and NotI, then conversion to a nucleotide sequence optimized for codons in humans was outsourced to Eurofins Genomics K.K. to provide one cDNA.

For cDNA for VEGF scFv expression, based on the amino acid sequences of the heavy and light chains of G6-31, one clone of the anti-VEGF antibody having binding ability to VEGF of both human and mice described in a literature (Liang, W. C. et al. (2006). Journal of Biological Chemistry, 281, 951-961), cDNAs corresponding to each were generated using a genetic information processing software GENETYX (GENETYX CORPORATION). The cDNAs corresponding to the heavy chain and the light chain were ligated with triple repeats of a GS linker composed of four glycine and one serine (Gly-Gly-Gly-Gly-Ser (SEQ ID NO: 12)), the basic structure of scFv. An Ig kappa leader sequence and a signal peptidase recognition site were ligated at their N terminal side. The amino acid sequence and the overall structure are shown in Figure 5. Finally, the Kozak sequence and the start codon were inserted into the transcription start point, and both ends of the sequence were sandwiched with restriction enzyme sites BamHI and NotI to provide one cDNA. The procedures in the following Examples 1.2 to 1.7 only show those related to T-BV. T-V_{H}V_{L} and T-sVEGFR1 were generated as described in Example 1.8.

### Example 1.2: Amplification of anti-human VEGF antibody expression gene-loaded plasmid (pEX-K-BV)

The pEX-K-BV (manufactured by Eurofins Genomics K.K.) (Figure 6), an anti-human VEGF antibody expression gene-loaded plasmid, was dissolved in TE buffer (at equivalent to 1 ng), added to One shot DH5α-T1 Competent Cell, and the mixture was left on ice for 30 minutes. After heat treatment at 42°C for 45 seconds, SOC medium was added to the mixture, and after incubation at 37°C for 1 hour, the whole amount of the culture solution was seeded in a kanamycin-added LB agar medium and cultured at 37°C overnight. Colonies were harvested, and cultured in a kanamycin-added LB medium at 37°C for 8 hours. Pellets were collected centrifugally, and the plasmid DNA was extracted using QIAprep Spin Miniprep Kit (Qiagen).

### Example 1.3: Insertion of anti-human VEGF antibody expression gene into shuttle vector SV-01

SV-01 was treated with restriction enzymes BamHI and NotI, and treated with CIAP. pEX-K-BV was also treated with BglII in combination with them, because the size of pEX-K obtained when treated with restriction enzymes BamHI and NotI, 2,507 bp, and the size of the anti-human VEGF antibody expression gene, 2,261 bp, are close in electrophoresis bands; thus BglII, which has no recognition site within the anti-human VEGF antibody expression gene and cleaves pEX-K almost at the middle, was used for the purpose of improving separation. After electrophoresis on 1% agarose gel at 50 V for 80 minutes, the band of interest was cut out within the UV transilluminator (UVP), and the DNA fragments of the size of interest were extracted with QIAquick Gel Extraction Kit (Qiagen). The extracted DNA fragments were mixed with DNA Ligation Kit Mighty Mix (Takara Bio Inc.), then mixed with One shot PIR1 Competent Cell. After subjected to a heat treatment, the mixture was cultured at 37°C for 1 hour, and the mixture was seeded in a kanamycin-added LB agar medium, and cultured at 37°C overnight. Colonies were harvested and cultured in a kanamycin-added LB medium at 37°C for 24 hours, then pellets were collected centrifugally, and DNA was extracted with QIAprep Spin Miniprep Kit. Extracted DNA was treated with restriction enzymes BamHI and NotI, then electrophoresed on 1% agarose gel to confirm insertion of anti-human VEGF antibody expression gene into SV-01.

### Example 1.4: Insertion of SV-01 into T-BAC

SV-01 in which the insertion of anti-human VEGF antibody expression gene was confirmed (hereinafter referred to as "BV/SV-01") was mixed with T-BAC and Cre-recombinase to react at 37°C for 1 hour, thereby BV/SV-01 was inserted into T-BAC by Cre-recombination. After ethanol precipitation, the resultant was mixed with ElectroMax DH10B Electrocompetent Cell. The mixture was placed in a cuvette, and electroporated at 1.2 kV, 25 mF, 200 Ω using a Gene Pulse Xcell electroporation system (Bio-Rad Laboratories, Inc.), and then cultured in a kanamycin chloramphenicol-added LB agar medium at 37°C overnight. Colonies were harvested and cultured in a kanamycin-added LB medium at 37°C for 24 hours, and then pellets were collected centrifugally, and BV/SV-01/T-BAC was extracted with QIAprep Spin Miniprep Kit.

### Example 1.5: Confirmation of recombination of BV/SV-01/T-BAC

Structural confirmation was performed by PCR and restriction enzyme treatment. PCR was performed using BV/SV-01/T-BAC as templates and setting forward/reverse primers in ICP6 (ICP6-f1)/LacZ (SV01-r1) or SV-01 (SV01-f1)/LacZ (SV01-r1), respectively, to confirm insertion of BV/SV-01 as single. T-BAC was used as a negative control for insertion, and mock (without template DNA) was used as a negative control for PCR. Both PCR was performed using a Veriti thermal cycler (Thermo Fisher Scientific) by treatments of denaturation at 94°C for 2 minutes, then 25 cycles of "98°C for 10 seconds, 57°C for 30 seconds, and 68°C for 1 minute and 30 seconds", and finally at 68°C for 7 minutes. The PCR product was electrophoresed on 1% agarose gel at 100 V for 25 minutes, and the electrophoresis pattern was confirmed.

BV/SV-01/T-BAC was treated with restriction enzymes HindIII, KpnI as the restriction enzyme treatment, and then electrophoresed on 0.6% agarose gel at 50 V overnight. T-BAC was used as a negative control.

### Example 1.6: Co-transfection of BV/SV-01/T-BAC, pOG44 to Vero

To BV/SV-01/T-BAC and FLP recombinase-expressing plasmid pOG44 (Thermo Fisher Scientific), a transfection reagent FuGENE HD (Promega Corporation) was added. The mixture was left at room temperature for 15 minutes, then added to Vero cells replaced with Opti-MEM, and cultured at 5%CO₂, 37°C for 3 hours. Subsequently, the culture was cultured in DMEM supplemented with 10% v/v FBS overnight, then in DMEM supplemented with 1% v/v heat-inactivated FBS at 37°C, 5%CO₂ for 72 hours.

### Example 1.7: Isolation of single clone by limit dilution method, and viral purification

After co-transfection, 50% cytopathic effect (CPE) was confirmed. Then, fluorescence of Green Fluorescence Protein (GFP) was observed with a fluorescence microscope BIOREVO (KEYENCE CORPORATION), and disappearance of fluorescence of 70-80% of the plaque formed by the virus, i.e., excision of BAC by FLP, was confirmed. Vero cells were then stripped with a cell scraper and recovered together with the culture solution. After centrifugation, pellets were collected, then phosphate buffer saline (PBS) was added. The mixture was repeatedly frozen and thawed 3 times, and subjected to an ultrasound crushing procedure to extract the virus in the cells. Subsequently, isolation of a single clone of the virus was performed by limit dilution method. Vero cells were seeded in 96-well plates at 1 x 10⁴ cells per well, then the cells were infected at a viral titer of 0.3 pfu/well. After shaking at room temperature for 5 minutes, the cells were cultured at 5%CO₂, 37°C for 90 minutes. Then the viral solution was removed, DMEM supplemented with 1% v/v heat-inactivated FBS was added, then the cells were cultured at 5%CO₂, 34.5°C, for 6 days. The wells in which GFP-negative single plaque was observed were selected by 3 wells per three 96-well plates, and the viruses were recovered together with the culture solution. This viral solution was amplified with 6-well plates, and then the limit dilution method was performed again with 96-well plates. These series of procedures were repeated a total of three times, and finally, the viruses that could be considered a single clone were isolated.

The isolated viruses were then purified. The isolated viruses were infected at MOI of 0.01 against 16 sheets of T150 flask in which Vero cells were placed at 1 x 10⁷ cells per sheet, and the flasks were shaken at room temperature for 5 minutes. After culturing at 5%CO₂, 37°C for 90 minutes, the viral solution was removed, VP-SFM supplemented with L-glutamine was added at 25 mL/flask, and the cells were cultured at 5%CO₂, 34.5°C. After confirmation of CPE, the cells were peeled off with a cell scraper, and recovered together with the culture solution. After centrifugation at 800 x g, pellets were collected, then 16 mL of PBS was added, and the mixture was suspended. The mixture was repeatedly frozen and thawed 3 times, and subjected to an ultrasound crushing procedure of 2 minutes. The suspension was filtered sequentially with a 0.8 µm filter and a 0.45 µm filter, then layered slowly and quietly in a distal sinking tube in which a 4 mL of 30% v/v sucrose (Sigma-Aldrich Co. LLC.)/PBS was placed. The tubes were centrifuged at 4°C, 24,000 x g for 90 minutes with an ultra-centrifuge Avanti HP-26XP (Beckman Coulter, Inc.). The pellets were collected and rinsed with 5 mL of cold PBS, then 1.6 mL of 10% glycerol (Merck Millipore)/PBS was added, and the pellets were dissolved by repeated brief ultrasonic crushing. After dispensed at 150 µL each, the solution was frozen with dry ice ethanol and stored at -80°C, then used for the following evaluation experiments.

### Example 1.8: Generation of T-V_{H}V_{L} and T-sVEGFR1

T-sVEGFR1 and T-V_{H}-V_{L}, the VEGF antagonist-expressing HSV-1, were generated by inserting cDNA of the gene of soluble VEGF receptor 1 (sVEGFR1), a VEGF inhibitor, (Park JE et al. (1994) J Biol Chem. Vol. 269 (41): 25646-54) (Figure 7), or a single-stranded anti-VEGF antibody VEGFscFv (VH-VL) (16, 17, 18) into the ICP6 deletion site of the basic backbone of G47Δ, using T-BAC system, in accordance with the generation procedures of T-BV described in the Examples above. Since VEGFscFv proteins secreted in the culture supernatant of T-VH-VL cannot be detected by ELISA method, T-VEGFscFv (VH-VLCL) having insertion of VEGFscFv (VH-VLCL) in which κ chain was fused to the C-terminus of VEGFscFv (VH-VL) was also generated to be detected by ELISA method for κ chain (Figure 8).

### Example 2: Structural Confirmation of T-BV by Southern Blot Method

For the final product, T-BV, structural confirmation was performed by Southern blot method. Viral DNA was extracted with QIAamp MinElute Virus Spin Kit (Qiagen) from 150 µL of the purified virus (titer 8.4 x 10⁸ pfu/mL) and concentrated with ethanol precipitation. The concentrated DNA was treated with a restriction enzyme HindIII at 37°C over 16 hours, and electrophoresed on 0.6% agarose gel at 45 V for 13 hours. After confirmation of the electrophoresis pattern with a UV transilluminator (UVP), the gel was immersed in 0.25 M hydrochloric acid at room temperature for 20 minutes to depurinate. Subsequently, the gel was immersed in a denaturing buffer (3 M NaCl, 0.4 M NaOH) at room temperature for 30 minutes to denature DNA. Viral DNA fragments were transcribed from the gel into a Nytran SPC nylon membrane (GE Healthcare) using a Turbo Blotter system (GE Healthcare). UV irradiation was performed at 254 nm, 120 mJ/cm² for 2 min with UV CrossLinker (UVP), and DNA was immobilized on the Nytran SPC nylon membrane. For probes, BV/SV-01 was treated with restriction enzymes BamHI, NotI, and SV-01 was treated with a restriction enzyme BglI, respectively, and electrophoresed on 0.7% agarose gel at 100 V for 30 minutes, and the respective bands were cut out to extract DNA with QIA Quick Gel Extraction Kit. DNAs were then labeled using an AlkPhos Direct Labelling and Detection System with CDP-Star (GE Healthcare) to provide a BV probe and a LacZ probe, respectively.

A hybridization buffer (GE Healthcare), warmed to 55°C in advance with a hybridization incubator (TAITEC CORPORATION), was placed and sealed in a hybridization bag with the Nytran SPC nylon membrane, and reacted at 55°C for 30 minutes. The labeled probe was then added, and hybridization was performed at 55°C overnight. The Nytran SPC nylon membrane was washed with a primary wash solution (2 M urea, 0.1% v/v SDS, 50mM Na phosphate (pH 7.0), 150 mM NaCl, 1 mM MgCl₂, 0.2% v/v Blocking Reagent) at 55°C for 20 minutes, then with a secondary wash solution (50 mM Tris base, 100 mM NaCl) at room temperature for 10 minutes. After treated with a detection solution (GE Healthcare) at room temperature for 4 minutes, the membrane was imaged with ImageQuant LAS-4000 (GE Healthcare).

### Example 3: Measurement of viral titer

In this Example, the titer of the virus used was expressed by plaque forming unit (pfu), and measured as follows. The measurement of the titer was performed each time a virus was used in the experiment to check whether the actual titer was significantly different from the expected titer. Vero cells were seeded in 6-well plates at 3.6 x 10⁵ cells/2 mL of DMEM supplemented with 10% FBS/well, and cultured at 5%CO₂, 37°C overnight. On the following day, three serial dilutions of viruses were prepared with Dulbecco's phosphate buffered saline (DPBS) (Sigma-Aldrich Co. LLC.) supplemented with 1% v/v heat-inactivated FBS, based on the expected titer. After washing the plates, in which Vero cells were seeded, with 1 mL/well DPBS supplemented with 1% v/v heat-inactivated FBS, the three serial dilutions of viruses were added at each concentration to 2 wells, a total of 6 wells, by 700 µL/well. The 6-well plates to which the viral solution was added was shaken at room temperature for 5 minutes, and the cells were cultured at 5%CO₂, 37°C for 1 hour. The viral solution was removed, the medium in which 5% human immunoglobulin G (Japan Blood Products Organization) was added to DMEM supplemented with 1% v/v heat-inactivated FBS at 0.1% v/v was added at 2 mL/well, and the cells were cultured at 5%CO₂, 34.5°C for 72 hours. The medium was removed, the plate was washed once with PBS at 1 mL/well, then methanol was added at 1 mL/well, and the plate was left at room temperature for 5 minutes for immobilization. Methanol was removed and the plate was dried, then the cells were stained with one-twentieth diluted Giemsa stain solution (Merck Millipore), and washed with distilled water. After drying, the plaque number was measured with microscope, and the titer was calculated as pfu/mL based on the dilution fold.

### Example 4: Study of cytopathic effects of virus in vitro Example 4.1: Study of cytopathic effects in U87MG cells

U87MG cells were seeded in 6-well plates at 2 x 10⁵ cells/2 mL of DMEM supplemented with 10% v/v FBS/well, and cultured at 5%CO₂, 37°C overnight. Viral dilutions of T-01 and T-BV at MOI of 0.1 and 0.01 were prepared with DPBS supplemented with 1% v/v heat-inactivated FBS, and the viral solution or mock (DPBS supplemented with 1% v/v heat-inactivated FBS) was added by each virus at each concentration to 3 wells at 700 µL/well. After shaking at room temperature for 5 minutes, the cells were cultured at 5%CO₂, 37°C for 1 hour. The viral solution was removed, DMEM supplemented with 1% v/v heat-inactivated FBS was added at 2 mL/well, and the cells were cultured at 5%CO₂, 34.5°C. The number of surviving cells was measured by Coulter Counter (Beckman Coulter, Inc.) for 4 consecutive days from infection, and calculated as a proportion relative to the surviving cell number in mock.

### Example 4.2: Study of cytopathic effects in TGS-01 and TGS-04

TGS-01 and TGS-04 need to be cultured in serum-free medium to maintain stem cellularity. Since floating cell masses (spheres) are formed in culture in serum-free medium, an evaluation different from that for adhesion-based cells is required for TGS-01 and TGS-04. Thus, the evaluation was performed by an MTS test in which the number of surviving cells is measured by a colorimetric method using a tetrazolium compound ([3-(4,5-dimethylthiazol-2-yl)-5-(3-carboxymethoxyphenyl)-2-(4-sulfophenyl)-2H-tetrazolium, inner salt; MTS]). The MTS test is an evaluation method in which MTS is converted by NADPH or NADH to a chromogenic formazan product in cells having metabolic activity, and quantification of the number of surviving cells is performed by measuring the absorbance of the obtained colored solution at 490 nm. In this experiment, measurements were performed using CellTiter 96 AQueous Non-Radioactive Cell Proliferation Assay Kit (Promega Corporation). TGS cells were seeded in non-adhesion-treated 96-well plates (NUNC) at 4,000 cells/SCM 50 µL/well. Viral dilutions of T-01 and T-BV at MOI of 1, 0.1, and 0.01 were prepared with DPBS supplemented with 1% v/v heat-inactivated FBS. The viral solution or mock (DPBS supplemented with 1% v/v heat-inactivated FBS) was added by each virus at each concentration to 3 wells at 10 µL/well. After shaking at room temperature for 5 minutes, the cells were cultured at 5%CO₂, 37°C for 1 hour. Then, 50 µL of SCM was added, and the cells were cultured at 5%CO₂, 37°C. One day after infection, a mixture of MTS + phenazine methosulfate (PMS) (20 : 1) was added at 20 µL/well, and the cells were further cultured at 5%CO₂, 37°C for 24 hours, and then absorbance was measured with a 96-well plate reader AD200 (Beckman Coulter, Inc.) at 490 nm. This addition of the reagent and the measurement at 24 hours later were performed for 4 consecutive days from infection, and the proportion relative to the absorbance in mock was calculated.

### Example 5: Viral Replication Test in vitro

Vero cells and U87MG cells were seeded in 6-well plates at 3 x 10⁵ cells/2 mL of DMEM supplemented with 10% v/v FBS/well, and the cells were cultured at 5%CO₂, 37°C, overnight. Viral dilutions of T-01 and T-BV at MOI of 0.01 were prepared with DPBS supplemented with 1% v/v heat-inactivated FBS. The viral solution or mock (DPBS supplemented with 1% v/v heat-inactivated FBS) was added by each virus at each concentration to 3 wells at 700 µL/well. After shaking at room temperature for 5 minutes, the cells were cultured at 5%CO₂, 37°C for 1 hour. The viral solution was removed, DMEM supplemented with 1% v/v heat-inactivated FBS was added at 2 mL/well, and the cells were cultured at 5%CO₂, 37°C. The cells were peeled off with a cell scraper 24 and 48 hours after infection, and recovered by each well together with the culture solution. The recovered cells were subjected to 3 cycles of freezing and thawing with dry ice ethanol, then a 1-minute ultrasound cell crushing treatment. The titer of the resultant was measured in the same manner as previously described.

### Example 6: Confirmation of expressed proteins

### Example 6.1: Confirmation of expressed proteins through western blotting

The specimen was prepared first. HEK293T cells were seeded in a 6-well plate at 8 x 10⁶ cells with 2 mL of DMEM supplemented with 10% v/v FBS per well, and cultured at 5%CO₂ and 37°C for 8 hours. After that, 1.6 µg of BV/SV-01 and SV-01 as a control were mixed with transfection reagents FuGENE HD and OptiMEM, and dripped over the HEK293T cells. The resulting cells were cultured at 5%CO₂ and 37°C for 48 hours so that the HEK293T cells were transfected with BV/SV-01 and SV-01. The supernatants were then collected, filtered with a 0.2 µm filter, and concentrated under ultrafiltration using an Amicon Ultra-15, 10K (Merck Millipore), and the obtained product was stored as a specimen. Avastin(registered trademark) (Chugai Pharmaceuticals, Co., Ltd.) was used as a positive control.

Polyacrylamide gel electrophoresis (PAGE) was performed in the presence of sodium dodecyl sulphate (SDS) (SDS-PAGE) and without SDS (native PAGE).

### Example 6.1.1: SDS-PAGE

2.4 mL of 40% w/v-Acrylamide/Bis Mixed Solution (29:1), 3 mL of 1 M Tris/HCl, 2.47 mL of distilled water, 80 µL of SDS (10% v/v), 40 µL of APS (10% v/v), and 8 µL of TEMED were mixed to prepare a 12% separate gel. The obtained gel was poured in between the gel preparation glass plates, over which distilled water was overlaid, and the gel was left for about 20 minutes until it became solid. The overlying distilled water was then removed and a 5% stacking gel, which was prepared by mixing 312.5 µL of 40% w/v-Acrylamide/Bis Mixed Solution (29:1), 312.5 µL of 1 M Tris/HCl, 1.835 mL of distilled water, 25 µL of SDS (10% v/v), 12.5 µL of APS (10% v/v), and 2.5 µL of TEMED, was stacked to thereby prepare the gel. The obtained gel was set in the electrophoresis tank, and an electrophoresis buffer (0.12 M Tris·HCl, 0.192 M glycine, and 3.47 mM SDS) was thereafter poured into the anode and cathode tanks. Distilled water and a 4x loading buffer (0.25 M Tris·HCl (pH 6.8), 8% v/v SDS, 40% v/v glycerol, 20% v/v 2-mercaptoethanol, and 0.2% v/v bromophenol blue) were mixed in order to control the amount of loaded protein so as to be constantly 500 ng, boiled at 100°C for 10 minutes, and then rapidly cooled on ice. The specimen was loaded into the wells and electrophoresis was performed at 10 mA, 250 V, 10 W in the stacking gel and at 20 mA, 250 V, 10 W in the separating gel, with both gels at room temperature. After a polyvinylidene difluoride (PVDF) membrane (Merck Millipore) was immersed with methanol, proteins were transferred to the PVDF membrane at 100 mA for 60 minutes using a Trans-Blot SD Cell (Bio-Rad Laboratories, Inc.), with the membrane being immersed with a transfer buffer (0.048 M Tris, 0.039 M Glysin, 20% v/v methanol, and SDS (10%) 100 µL). The membrane was blocked with a Blocking One at room temperature for 50 minutes and then reacted with a primary antibody, a goat anti-human IgG-heavy and light chain monkey-absorbed antibody (1/2000 dilution) at 4°C overnight. The PVDF membrane was washed and then reacted with a secondary antibody, a donkey anti-goat IgG-horseradish peroxidase (HRP) conjugated (1/2000 dilution) at room temperature for two hours. The resulting membrane was washed and then reacted with a Pierce Western Blotting Substrate (1:1) (Thermo Fisher Scientific) at room temperature for about 5 minutes, and images were captured with LAS4000.

### Example 6.1.2: Native PAGE

The gel used was a precast gel, NativePAGE Novex 3-12% Bis Tris Gels (Thermo Fisher Scientific). After the gel was set in the electrophoresis tank, an anode buffer (0.05 M BisTris and 0.05 M Tricine) was poured into the anode tank and a cathode buffer (an electrophoresis buffer (0.05 M BisTris, 0.05 M Tricine, and NativePAGE Cathode Additive (Thermo Fisher Scientific)) was poured into the cathode tank. Distilled water and a 4x NativePAGE Sample Buffer (Thermo Fisher Scientific) were mixed, in order to control the amount of loaded protein so as to be constantly 500 ng. The specimen was loaded into the wells and electrophoresis was performed at 150 V, 8-10 mA for the first 60 minutes and at 250 V, 2-4 mA for the remaining 30-90 minutes, the entire process of electrophoresis being performed in a low-temperature room at 4°C in order to prevent thermal denaturation. Proteins were transferred to the PVDF membrane at 25V for one hour, using a Xcell2 Blot Module (Thermo Fisher Scientific), with an inner chamber filled with a transfer buffer (0.5 M Tricine, 0.5 M Bis-Tris, and 0.02 M EDTA) and an outer chamber filled with distilled water. The membrane was blocked with a Blocking One at room temperature for 50 minutes and reacted with a primary antibody, a goat anti-human IgG-heavy and light chain monkey-absorbed antibody (1/2000 dilution), at 4°C overnight. The PVDF membrane was washed and then reacted with a secondary antibody, a donkey anti-goat IgG-HRP conjugated (1/2000 dilution), at room temperature for two hours. The resulting membrane was washed and then reacted with a Pierce Western Blotting Substrate (40:1) (Thermo Fisher Scientific) at room temperature for 5 minutes, and images were captured with LAS4000.

### Example 6.2: ELISA quantification of expressed anti-human VEGF antibodies in vitro

U87MG cells were seeded in a 6-well plate at 4 x 10⁵ cells with 2 mL of DMEM supplemented with 10% v/v FBS per well, and cultured at 5% CO₂ and 37°C for 12 hours. After that, viral dilutions of T-01 and T-BV at MOI of 0.2 were prepared using DPBS supplemented with 1% v/v heat-inactivated FBS. The viral solution or mock (DPBS supplemented with 1% v/v heat-inactivated FBS) was added to three wells for each virus at 700 µL/well. After shaking at room temperature for 5 minutes, the cells were cultured at 5% CO₂ and 37°C for 1 hour. The viral solution was then removed, DMEM was added at 2 mL/well, and the cells were cultured at 5% CO₂ and 37°C for 72 hours. The supernatants were then collected and concentrated 2.7-fold under ultrafiltration using an Amicon Ultra-15, 10K, to thereby obtain a specimen. Avastin(registered trademark) was used as a standard for drawing a calibration curve. Eight serial dilutions were prepared based on the stock solution (25 mg/mL) by repeating one-third dilution from 90 ng/mL. A Sample/conjugate diluent (Bethyl Laboratories, Inc.) was used for dilution of the standard, specimen and detection antibodies. ELISA was conducted using an ELISA Starter Accessory Kit (Bethyl Laboratories, Inc.). Recombinant Human VEGF 165 (Peprotech) was placed in a micro titer well at 50 ng/100µL and reacted at room temperature for one hour to perform immobilization of the antigen. After washing five times with 100 µL/well ELISA Wash Solution, a Blocking Solution was added at 200 µL/well so that blocking was performed at room temperature for 30 minutes. After washing five times with 200 µL/well ELISA Wash Solution, the standard and the specimen were each added at 100 µL/well and reacted at room temperature for one hour. After washing five times with 100 µL/well ELISA Wash Solution, a goat anti-human IgG-Fc fragment antibody HRP conjugated (1/100000 dilution) was added as a detection antibody at 100 µL/well, and reacted at room temperature for one hour. After washing five times, a TMB Substrate was added at 100 µL/well and reacted for 15 minutes in dark, a Stop Solution (0.18 M H₂SO₄) was then added at 100 µL/well, and an absorbance at 450 nm was measured using a 96-well plate reader AD200. A calibration curve was created based on the absorbance obtained for the standard, and the concentration of anti-human VEGF antibodies in the specimen was calculated. The calibration curve was prepared using the free image processing software "Image J."

### Example 7: Confirmation of functions of antibodies expressed by T-BV

A vascular endothelial cell tube formation test and a vascular endothelial cell migration test were performed using HUVEC-2 to confirm the VEGF inhibitory effects of proteins expressed by T-BV. The specimen was prepared first. Vero cells were used for the preparation of the specimen after confirming that an expression amount of VEGF from such Vero cells was almost the same as that of U87MG cells. The Vero cells were seeded in a T150 flask at 8 x 10⁶ cells and cultured at 37°C and 5% CO₂ for 8 hours. The cells were then infected with T-01, T-BV, or mock (DPBS supplemented with 1% v/v heat-inactivated FBS) at MOI of 0.2, shaken at room temperature for 5 minutes, and cultured at 37°C and 5% CO₂ for one hour. The viral solution was removed and replaced with 15 mL of DMEM, and culture was further performed at 37°C and 5% CO₂ for three days. The supernatants were then collected and concentrated 40-fold under ultrafiltration using an Amicon Ultra-15, 10K.

### Example 7.1: Vascular endothelial cell tube formation test

The vascular endothelial cell tube formation test was performed using a BD BioCoat Angiogenesis System-Endothelial Cell Tube Formation (BD Bioscience). A BD BioCoat Angiogenesis Plate was thawed at 4°C 24 hours before cell seeding, and HUVEC-2 was seeded at 2 x 10⁴ cells per well. The prepared specimens were added at 2.5 µL per well to prepare three groups, and, in addition to this, two groups were prepared as positive controls by adding 2.5 µL of mock with 10 pg/2.5 µL or 10 ng/2.5 µL of Avastin(registered trademark) (p.c.1 and p.c.2) and one group was prepared as a negative control in which no cell culture supernatant was added. These six groups in total were assessed. The test was conducted with three wells for each group. A Medium 200 supplemented with 1% v/v LSGS was added to each well so that the total volume of culture solution in the well was 50 µL, and then, culture was performed at 37°C and 5% CO₂ for 17 hours. After removing the culture solution and washing twice with 100 µL/well Hanks' Balanced Salt Solution (HBSS) (Thermo Fisher Scientific), 8 µg/mL Calcein AM (BD Bioscience) was added at 50 µL/well for labelling at 37°C for 30 minutes. After removing the Calcein AM and washing twice with 100 µL/well HBSS, images of each well were captured with a fluorescence microscope BIOREVO, to measure the length of the formed tubes and thereby calculate the average of the total tube lengths of the three wells for each group.

### Example 7.2: Vascular endothelial cell migration test

The vascular endothelial cell migration test was performed using a BD BioCoat Angiogenesis System-Endothelial Cell Migration (BD Bioscience). HUVEC-2 was seeded in an upper chamber at 1 x 10⁵ cells per well and the prepared specimens were added to lower wells at 19 µL per well, to thereby prepare three groups. Further, two groups were prepared as positive controls by adding 19 µL of mock with 380 pg/19 µL or 380 ng/19 µL of Avastin(registered trademark) (p.c.1 and p.c.2) and one group was prepared as a negative control in which no cell culture supernatant was added. These six groups in total were assessed. The test was conducted with three wells for each group. A Medium 200 supplemented with 1% v/v LSGS was added to each well so that the total volume of culture solution in the well was 750 µL, and then, culture was performed at 37°C and 5% CO₂ for 22 hours. After the culture solution within the upper chamber and the medium attached to the undersurface of the membrane were removed, the cells were transferred to a 24-well plate, which was separately prepared by dispensing 500 µL of 8 µg/mL Calcein AM into each well, so as to label the cells at 37°C for 90 minutes. Using a fluorescence microscope BIOREVO, a fluorescence intensity of each well at 494 nm/517 nm was measured to calculate the average of the three wells in each group.

### Example 8: Confirmation of interspecies cross-reactivity of expressed antibodies of T-BV

The specimen was prepared first. HEK293T cells were seeded in a T150 flask at 6 x 10⁶ cells, cultured at 37°C, 5%CO₂ for 8 hours. Then, the cells were infected with T-BV at MOI of 3. The plate was shaken at room temperature for 5 minutes, and then the cells were cultured at 37°C, 5%CO₂ for 1 hour. The viral solution was removed, 15 mL of VP-SFM was added, and the cells were further cultured for 15 hours. The supernatant was then collected, and concentrated 20-fold with ultrafiltration to prepare a specimen. The concentrated T-BV infected supernatant solution, Avastin(registered trademark) and a rabbit anti-mouse VEGF antibody were used as primary antibodies. Then, as the secondary antibodies, anti-human IgG-Fc antibodies were used for the concentrated T-BV infected supernatant solution and Avastin(registered trademark) since the Fc moieties thereof derived from human, and an anti-rabbit IgG-Fc antibody was used for the rabbit anti-mouse VEGF antibody, and each detection was performed. In the ELISA performed for the concentrated cell supernatant solution made above, the concentration of the anti-human VEGF antibody was 8.85 ng/mL. Based on this, the concentrations of Avastin(registered trademark) and the anti-mouse VEGF antibody, which were positive controls, were set to two levels of 8.85 ng/mL and 0.885 ng/mL. As a negative control, VP-SFM was used. In the negative control, a crossover reaction system in which two antibodies were reacted with each of the wells immobilized with each VEGF was set up, with the consideration of possibility of false positives due to cross-reactions between two immobilized VEGFs and two secondary antibodies for detection.

ELISA Starter Accessory Kit (Bethyl Laboratories, Inc.) was used as the ELISA kit. Human VEGF or mouse VEGF was placed in the micro titer well at 50 ng/100 µL/well each and reacted at room temperature for 1 hour to perform immobilization of the antigen. After washing five times with 100 µL/well ELISA Wash Solution, 200 µL/well ELISA Blocking Solution was added, and blocking was performed at room temperature for 30 minutes. After washing five times with 200 µL/well ELISA Wash Solution, the specimens were placed at 100 µL/well, and reacted at room temperature for 1 hour. After washing five times with 100 µL/well ELISA Wash Solution, 100 µL/well of each secondary antibody (1/100000 dilution) was added, and reacted at room temperature for 1 hour. After washing five times with ELISA Wash Solution, TMB Substrate was added at 100 µL/well, and the mixture was reacted for 15 minutes under shading. Stop Solution was then added by 100 µL/well, and absorbance of 450 nm was measured with a 96-well plate reader AD200.

### Example 9: Animal Experiments

For the viral solutions used for treatment in animal experiments, all filter sterilized, 10% glycerol in PBS were used. For the cell suspension, DMEM/F12 (1:1) was used for TGS, and DMEM was used for other cell lines. In the mock group, DPBS supplemented with 1% v/v heat-inactivated FBS, in place of the viral solution, was added to Vero cells upon purification, and those purified were administered at the subsequent procedures, as the virus. General anesthesia was performed by intraperitoneal injection of ketamine (DAIICHI SANKYO COMPANY, LIMITED) and xylazine (Bayer Holding Ltd.) or by inhalation of isoflurane (Pfizer Inc.).

### Example 9.1: Study of anti-tumor effects

### Example 9.1.1: Intratumoral administration experiment of virus in a subcutaneous tumor model

2 x 10⁶ cells of U87MG suspended in 50 µL of DMEM were subcutaneously implanted with 26 G needles into the left abdomen of BALB/c nu/nu mice under general anesthesia by intraperitoneal administration of ketamine and xylazine. The tumors were measured at a frequency of 2-3 times a week with a caliper, and calculated by tumor volume (mm³) = long diameter x short diameter x thickness

After confirmation that the tumor diameter reached 6 mm, the mice were randomly grouped into each treatment group by 10 mice, and intratumoral administration of the virus was performed. 2 x 10⁵ pfu/20 µL of T-01, T-BV or mock diluted with PBS supplemented with 10% v/v glycerol were administered using a microsyringe #1710 (Hamilton Company) fitted with a 30 G needle to the mice under general anesthesia with intraperitoneal administration of ketamine and xylazine. The day of first administration of the virus was set as day 0, and the same amount of virus was intratumorally administered on day 3. Tumor diameters were measured 2-3 times a week still after virus administration to calculate the tumor volume.

### Example 9.1.2: Survival experiment by intratumoral administration of virus in brain tumor models

Under general anesthesia with intraperitoneal administration of ketamine and xylazine, the heads of BALB/c nu/nu mice were immobilized with a small animal brain stereotactic immobilizer MODEL 900 (DAVID KOPF INSTRUMENTS), and 2 x 10⁵ cells of U87MG suspended in 2 µL of DMEM were injected with a microsyringe #1701N (Hamilton Company) into the right frontal lobe. The puncture site was set to be 2 mm from bregma to the right outer side and 1 mm to the front. A longitudinal skin incision of about 4 mm in length was made just above this, and the periosteum was stripped, and then the head was punctured. The puncture needle was then passed from the brain surface to a depth of 3 mm. The cell suspension was injected over 2 minutes and the needle was held for 5 minutes, then the puncture needle was removed over 2 minutes. The skin was sutured with 5-0 vicryl (Johnson & Johnson). For TGS-01 and TGS-04, 1 x 10⁵ cells suspended in 2 µL of DMEM were implanted into the right frontal lobe in the same procedures as described above.

When the tumor size reached 2-3 mm large, the mice were randomly grouped into each treatment group by 10 mice, and then the viruses were administered into the tumor on day 10 from tumor implantation for U87MG and TGS-01, and on day 20 from tumor implantation for TGS-04. The heads of mice were immobilized with a small animal brain stereotactic immobilizer under general anesthesia by intraperitoneal administration of ketamine and xylazine, and the cutaneous incision site set upon the tumor implantation was incised again. 1 x 10⁶ pfu/5 µL of T-01, T-BV or mock diluted with 10% v/v glycerol added PBS were injected into U87MG, and 2 x 10⁶ pfu/5 µL of T-01, T-BV or mock were injected into TGS-01 and TGS-04, with a microsyringe #1701N, respectively. To prevent contamination of different viruses, a fresh microsyringe was used for each virus. The puncture site and depth were set to the same as when the tumor was implanted. The viral solution was injected over 2 minutes and the needle was held for 5 minutes, then the puncture needle was removed over 2 minutes. The skin was sutured with 5-0 vicryl. The virus was administered in a single dose, and the date of administration was set as day 0. The survival of each mouse was recorded.

### Example 9.2: Analysis of swelling-suppressive effects

### Example 9.2.1: Magnetic resonance imaging (MRI)

### Example 9.2.1.1: Preparation of brain tumor models using various human brain tumor cell lines, and intratumoral administration of virus

In order to analyze the swelling-suppressive effects of T-BV on various human brain tumors, BALB/c nu/nu mouse brain tumor models were prepared using U87MG, U251MG, NMC-G1, TGS-01 and 1123/M. U87MG, which were the most easily depictable tumors by MRI, was used for testing. Number of implanted cells: 2 x 10⁵ (cells); Date of virus administration: 12 days after tumor implantation; Amount of virus administration: 1 x 10⁶ (pfu). In a similar manner to that previously described, tumor cells were implanted into the right frontal lobe. After confirming by MRI that the tumor had a suitable size for virus administration, the virus was intratumorally administered in a similar manner to that previously described. As a positive control, a group using T-01 and Avastin(registered trademark) in combination (a T+A group for which 5 mg/kg of Avastin(registered trademark) was intraperitoneally administered for 5 consecutive days after the intratumoral administration of T-01) was established.

### Example 9.2.1.2: Imaging by MRI

Imaging by MRI was performed under inhalational anesthesia of isoflurane and respiration monitoring. MRI ICON 1T™ (Brucker Biospin) for small animals was used for imaging. The following parameters were set for imaging by MRI.

T2 weighted image (T2WI): repetition time (TR) = 2,000 msec; echo time (TE) = 85 msec; 6 slices; thickness = 0.7 mm; field of view (FOV) = 20 x 20 mm; matrix = 96 x 96; and number of averages (NA) = 8.

T1 weighted image (T1WI): TR = 400 msec; TE = 12 msec; 6 slices; thickness = 0.7 mm; FOV = 20 x 20 mm; matrix = 96 x 96; and NA = 10.

A contrast-enhanced T1WI (T1WI (CE)) was captured after the imaging of T2W1, by diluting an MRI contrast medium Magnevist(registered trademark) (Bayer Holding Ltd.) 1/20-fold with PBS and intravenously administering 100 µL of such diluted contrast medium via the eye orbit.

After the tumor implantation, T2WI and T1WI (CE) were captured as needed, to confirm the development of the tumor. After confirming that the tumor was developed, the date of virus administration was determined. T2WI and T1WI (CE) were captured for all individuals the day before the determined administration date, to select individuals whose tumor size and shape were suitable for intratumoral administration of the virus. The selected individuals were randomly divided into the mock group and the viral treatment group. Images were also captured by MRI 2, 4 and 6 days after the virus administration (post inoculation day (PID) 2, 4 and 6) under the same imaging phase.

### Example 9.2.1.3: Evaluation of swelling area

For captured images, the high intensity area in T2WI and the contrast area in T1WI (CE) were measured using DICOM image processing free software OsiriX. For the areas (mm²) of the high intensity area in T2WI and T1WI (CE), respectively, the images taken the day before virus administration were set as S₂ₚ and S₁ₚ, respectively, and the images taken on day n after virus administration were set as S₂ₙ and S₁ₙ, respectively, and the swelling (edema) around the tumor was calculated by (S₂ₙ/S₂ₚ)/(S₁ₙ/S₁ₚ) as the relative value.

### Example 9.2.2: Quantification of mouse aquaporin (AQP) 4 by RT-qPCR

### Example 9.2.2.1: Generation of U87MG brain tumor model and intratumor administration of virus

In a similar manner as previously described, 2 x 10⁵ cells of U87MG suspended in 2 µL of DMEM was implanted into the right frontal lobe of BALB/c nu/nu mice. The mice were randomly grouped into each treatment group by 6 mice after 10 days of tumor implantation. Then, 1 x 10⁶ pfu/5 µL of T-01, T-BV or mock were administered intratumorally in the same manner as previously described. T+A group was set as a positive control.

### Example 9.2.2.2: RT-qPCR

All mice were euthanized at PID 6. The tumor-bearing right frontal lobes were harvested, and RNA was extracted from the lobes using an RNeasy Plus Mini Kit (Qiagen). After that, cDNA was generated from the extracted RNA using a reverse transcription enzyme ReverTra Ace qPCR RT Master Mix (Toyobo). As PCR primers, primers of murine AQP4 (Mm_00802131_m1) and murine β-actin (Mm_02619580_g1) in Taqman Gene Expression Assays were used, and a 7500 Fast Real-Time PCR System (Thermo Fisher Scientific) was used. In data analysis, murine β-actin was used as a reference gene and data was calculated using a comparative cycle method.

### Example 9.2.3: Comparison of swelling-suppressive effects of T-BV and T-V_{H}V_{L}

In a similar manner as previously described, 2 x 10⁵ cells of U87MG suspended in 2 µL of DMEM were implanted into the right frontal lobe of BALB/c nu/nu mice. For virus administration, on 10 days after tumor implantation, when the tumor was confirmed to be the right size for virus administration by MRI, 1 x 10⁶ pfu/5 µL of T-01, T-BV, T-V_{H}V_{L} or mock were administered intratumorally in the same manner as previously described. The method for imaging by MRI, the timing, and the evaluation of swelling were performed in the same manner as the swelling-suppressive effect confirmation experiment of T-BV.

### Example 9.3: Effects on the tumor environment after virus administration

### Example 9.3.1: Pathological study after virus administration to brain tumor models (immunohistochemical staining)

### Example 9.3.1.1: Generation of U87MG brain tumor model, intratumor administration of virus, and slide preparation

In a similar manner as previously described, 2 x 10⁵ cells of U87MG suspended in 2 µL of DMEM were implanted into the right frontal lobe of BALB/c nu/nu mice. The mice were grouped into treatment groups by 6 mice each 10 days after tumor implantation. Then, 1 x 10⁶ pfu/5 µL of T-01, T-BV or mock were administered intratumorally in the same manner as previously described. T+A group was set as a positive control. Avastin(registered trademark) was administered at 5 mg/kg daily from the date of virus administration to euthanasia. The mice were euthanized at PIDs 2 and 4 by 3 mice in each group. The brain was then removed, and immobilized by penetration of neutral buffered formaldehyde (10) (Sigma-Aldrich Co. LLC.) for 24 hours. The immobilized brain was paraffin-embedded with a fixed paraffin-embedded device Tissue-Tek VIP-5-Jr (SAKURA SEIKI Co., Ltd.), and a paraffin-block was prepared with a paraffin-embedded block production device Tissue-Tek TEC Plus (SAKURA SEIKI Co., Ltd.). The tumor center site of the block was thinly sliced with a microtome (Leica Biosystems Nussloch GmbH) in thickness of 4 µm to provide slides of continuous sections.

Furthermore, for the purpose of evaluating angiogenesis upon administration of T-BV and T-V_{H}V_{L}, 2 x 10⁵ cells of U87MG suspended in 2 µL of DMEM were implanted into the right frontal lobe of BALB/c nu/nu mice in a similar manner as previously described to prepare brain tumor models. The mice were grouped into treatment groups by 6 mice each 10 days after tumor implantation. Then, 1 x 10⁶ pfu/5 µL of T-01, T-BV, T-V_{H}V_{L} or mock were administered intratumorally in the same manner as previously described. Then, slides of paraffin sections were prepared in the same manner as described above.

### Example 9.3.1.2: Immunohistochemical staining

The slides were stretched and dried on a stretcher at 43°C overnight, then the slide was immersed in xylene for 30 minutes for deparaffinization treatment, and then immersed in 100% ethanol, 90% v/v ethanol and 70% v/v ethanol for 10 minutes each for hydrophilic treatment. After washing with distilled water, antigen activation treatment was performed by microwave method, and blocking of endogenous peroxidase was performed with Peroxidase-Blocking Solution (Dako). After washing with Tris-HCl Buffer Saline (TBS) for 10 minutes, and then blocking was performed by immersion in 1.5% v/v Normal Goat Serum (VECTOR LABORATORIES, INC.) at room temperature for 15 minutes. For the primary antibody reaction, the following antibodies at the indicated dilution fold were used to react at room temperature for 1 hour. Anti-HSV-1 antibody (1:2000), anti-mouse CD31 antibody (1:40), anti-mouse F4/80 antibody (1:2000). For dilution, 1.5% v/v normal goat serum was used. After the primary antibody reaction, washing was performed with TBS for 10 minutes. For the secondary antibody reaction, HRP-labeled anti-rabbit polymer or Mouse MAX PO (Rat) was used to react at room temperature for 30 minutes. After washing with TBS for 10 minutes, the detection was performed by immersing in DAB Substrate Kit (VECTOR LABORATORIES, INC.). After detection, contrast staining was performed with hematoxylin for 7 minutes. The slide was sequentially immersed in 70% v/v ethanol, 90% v/v ethanol and 100% ethanol for 10 minutes each, then dehydrated, and immersed in xylene for 10 minutes for penetration, and then enclosed with a non-fat-soluble encapsulant mount quick (Daido Sangyo Co.,Ltd.).

### Example 9.3.1.3: Evaluation of tumor angiogenesis

Tumor angiogenesis was evaluated by measuring blood vessel counts in tumors using the slide that underwent immunohistochemical staining of mouse CD31 against U87MG brain tumor models after virus administration. The measurement of blood vessel counts was performed in accordance with the method by Weidner et al. (Weidner, N. et al. (1991) The New England Journal of medicine 324, 1-8). The portion having the largest vessel density was imaged at a magnification of 200 fold regarding the specimen slides each day in each group at n = 3, using a virtual slide scanner Nanozoomer (Hamamatsu Photonics K.K.). The CD31-positive cells were counted to calculate the mean blood vessel count.

### Example 9.3.2: Changes in macrophages after virus administration to brain tumor models

### Example 9.3.2.1: Generation of U87MG brain tumor models, and intratumor administration of virus

In a similar manner as previously described, 2 x 10⁵ cells of U87MG suspended in 2 µL of DMEM were implanted into the right frontal lobe of BALB/c nu/nu mice. The mice were grouped into treatment groups by 9 mice each 10 days after tumor implantation. Then, for virus administration, 1 x 10⁶ pfu/5 µL of T-01, T-BV or mock were administered intratumorally in the same manner as previously described. T+A group was set as a positive control. Avastin(registered trademark) was administered at 5 mg/kg daily from the date of virus administration to euthanasia, for 5 days at the longest.

### Example 9.3.2.2: Evaluation of macrophages by flow cytometry

At PIDs 2, 4 and 6, three mice were euthanized in each group and, immediately after that, their tumor-bearing right frontal lobes were harvested. The tissues were dissociated using a Naural Tissue Dissociation Kit (Miltenyi Biotec) and a gentleMACS Octo Dissociator (Miltenyi Biotec), to prepare a suspension of single cells. The obtained cell suspension was placed into an Eppendorf tube, suspended with 1 mL of PBS, vortexed under the addition of 1 µL of Fixable Viability Dye eFluor 780 (Affymetrix), and reacted at 4°C for 30 minutes with light shielded, so as to stain the dead cells. After washing twice using a Flow Cytometry Staining Buffer (eBioscience), the suspension was adjusted to a 1 mL suspension to be used as a specimen. First, 2 µL of anti-mouse CD16/32 antibody was added for reaction on ice for 5-10 minutes so that non-specific binding of antibodies via an Fc receptor was blocked. As a result of studying the conditions, an amount of each antibody to be added per 100 µL of the specimen was determined as follows: anti-CD45 antibody (0.5 µL); anti-CD11b antibody (1 µL); anti-F4/80 antibody (5 µL); anti-Gr-1 antibody (0.5 µL); and anti-CX3CR1 antibody (0.5 µL). Stained samples were prepared by adding all of the above antibodies to each tube containing the specimen, and a single-stained sample was also prepared by adding only a single type of antibody for making corrections for fluorescence, as well as a non-stained sample for sensitivity adjustment. The samples were tapped for mixing, reacted at 4°C for 30 minutes with light shielded, and thereafter washed twice using a Flow Cytometry Staining Buffer. The samples were then suspended in 100 µL of Flow Cytometry Staining Buffer, immobilized with the addition of 10 µL of 4% v/v formalin/PBS, and analyzed using a flow cytometer CytoFLEX (Beckman Coulter, Inc.). The non-stained specimen was first used to adjust the sensitivity of each fluorescence and the single-stained sample was then used to make a correction for fluorescence. After that, each stained sample was analyzed. Macrophages are a population which is CD45 positive, CD11b positive and F4/80 positive, in which M1 can be separated as Gr-1^{high}CX3CR1^{low} while M2 can be separated as Gr-1^{low}CX3CR1^{high}. After myelin fractions were gated out by two-dimensional plotting of scattered light and dead cells were eliminated by dead cell staining, CD45-positive fractions were plotted in a two-dimensional manner based on CD11b and F4/80, and CD11b-positive and F4/80-positive cells were further plotted in a two-dimensional manner based on Gr-1 and CX3CR1, so as to thereby separate M1 and M2 macrophages. The respective proportions of M1 and M2 in the CD45-positive cells were calculated and the M1/M2 ratio was accordingly calculated to thereby evaluate a shift in the M1/M2 ratio of macrophages.

### Example 9.3.2.3: Virus replication test and measurement of viral titer (in vivo)

In a similar manner to that previously described, mice were subcutaneously implanted with U87MG cells, to develop a tumor. After confirming that the tumor reached 6 mm in diameter 20 days after the tumor implantation, the mice were randomly divided into the respective treatment groups, with 12 mice for each group. After that, virus administration was performed in a similar manner to that previously described. The administration was performed by 1 x 10⁶ pfu of T-01, T-BV, or mock, or T-01 along with intraperitoneal administration of Avastin(registered trademark) (T+A). In the T+A group, Avastin(registered trademark) was intraperitoneally administered at 5 mg/kg of body weight for 5 days (starting just before virus inoculation). Tumors were collected immediately after virus inoculation, or 1, 3 or 7 days after the inoculation, and homogenized in 300 µL of ice-cold PBS. After repeating three cycles of freezing and thawing, titers were measured using Vero cells in the same way as in Example 3 (n=3 per group, each measurement day).

### Example 9.4: Confirmation of antibody expression of T-BV in vivo

### Example 9.4.1: Confirmation of antibody expression at the local site of administration after T-BV administration to a subcutaneous tumor model

In a similar manner as previously described, 2 x 10⁶ cells of U87MG suspended in 50 µL of DMEM were implanted into the left abdomen of BALB/c nu/nu mice. When the tumor diameter reached 5 mm, the mice were randomly divided into T-01, T-BV, and mock groups by 9 mice each. 2 x 10⁶ pfu/20 µL of T-01, T-BV were administered in a single dose into tumors. Mice were euthanized at PIDs 2, 4, and 6 by 3 mice in each group, and subcutaneous tumors were removed. The tumors were crushed in 500 µL of ice-cold Cell Lysis Buffer (FUJIFILM Wako Pure Chemical Corporation) in which 1/100 amount of Complete Protease inhibitor cocktail (F. Hoffmann-La Roche Ltd.) and 1/1000 amount of 1M dithiothreitol (FUJIFILM Wako Pure Chemical Corporation) were added, and then concentrated 2-fold with Amicon Ultra-15, 10K. Since the in vivo specimen of mice contains a lot of mouse IgG and reduces non-specific responses to ELISA, the immunoprecipitation method was used to remove the mouse IgG. Specifically, anti-mouse IgG (H&L), F(ab')2 Fragment (Sepharose Bead Conjugate) (Cell Signaling Technology, Inc.) were added to the specimen at 10 µL each, and the mixture was invert-mixed at 4°C for 1 hour and centrifuged. The obtained supernatant was used for ELISA. ELISA was performed in the same manner as previously described with wells in which human VEGF was immobilized. As the standard, Avastin(registered trademark) serially diluted was used. Absorbance at 450 nm was measured with a 96-well plate reader. A calibration curve was drawn using an image-processing free software ImageJ based on the standard, and the anti-human VEGF antibody concentration in the specimen was calculated.

### Example 9.4.2: Confirmation of blood concentrations of expressed antibodies after virus administration in brain tumor models

Blood concentrations of anti-human VEGF antibodies expressed upon administration of T-BV to brain tumor models were measured by ELISA. 2 x 10⁵ cells of U87MG suspended in 2 µL of DMEM were implanted into the right frontal lobe of BALB/c nu/nu mice in a similar manner as previously described. 10 days after tumor implantation, the mice were grouped into T-01, T-BV, T+A, and mock groups by 6 mice each. Then, for virus administration, 1 x 10⁶ pfu/5 µL of T-01, T-BV or mock were administered intratumorally. For the T+A group, 5 mg/kg Avastin(registered trademark) was administered in a single dose intraperitoneally in a single dose on the date of virus administration. Venous blood sampling was performed at PIDs 1 and 3 by 3 mice in each group, and VEGF concentrations in the serum were measured by ELISA.

### Example 10: Evaluation of T-V_{H}V_{L} and T-sVEGFR1

In Example 10, T-V_{H}V_{L} and T-sVEGFR1 generated in Example 1.8 were evaluated as follows.

### Example 10.1: Measurement of virus titer

Vero cells were seeded into 6-well plates at 3.6 x 10⁵ cells/well, and cultured at 37°C, 5%CO₂. After 16 hours, the cells were washed with PBS supplemented with 1% heat-inactivated FBS (1% IFBS). Then, a viral solution of 10-fold serial dilutions was added, and the cells were cultured under conditions of 37°C, 5%CO₂. After 1 hour, the viral solution was removed, DMEM supplemented with 1% IFBS, to which 0.1% human immunoglobulin was added, was added as medium, and the cells were cultured under conditions of 34.5°C, 5%CO₂. After 3 days, the cells were washed with PBS and immobilized with methanol. After dried, the cells were stained with Giemsa solution diluted 20 times with distilled water, then washed and dried. The plaque number was counted under microscope, and the virus titer (pfu/ml) was calculated.

### Example 10.2: Structural confirmation of viral DNA by Southern blotting method

Viral DNA was extracted from the purified virus using QIAamp DNA Mini Kit (QIAGEN, Netherlands) according to the manufacturer's protocol, and then treated with a restriction enzyme KpnI. After electrophoresis on a 0.6% agarose gel at a voltage of 35 V for 16 hours, viral DNA was transcribed onto a nylon membrane (Turbo Blotter, Whatman, US). The probes were prepared by labeling the DNA fragments of sVEGFR1 and VEGFscFv with alkaline phosphatases, respectively (AlkPhos Direct with CDP-Star, GE Healthcare, USA). The probes were hybridized with the DNA-transcribed membrane, and detected using AlkPhos Direct with CDP-Star (GE Healthcare, USA).

### Example 10.3: Confirmation of VEGF inhibitor protein expression of T-sVEGFR1 and T-VEGFscFv (in vitro)

To confirm the soluble VEGFR1 protein expressed upon infection of cells with T-sVEGFR1, Human sVEGF-R1/FLT-1 ELISA Kit (BioVendor, USA) was used. To confirm the single-stranded anti-VEGF antibody expressed upon infection of cells with T-VEGFscFv, Human Kappa ELISA Kit (Bethyl Laboratories, USA) was used.

Vero cells were seeded into 6-well plates at 3 x 10⁵ cells/well, and cultured at 37°C, 5%CO₂. After 16 hours, the plates were washed with PBS supplemented with 1% IFBS. The cells were infected with T-01, T-sVEGFR1 or T-VEGFscFv at multiplicity of infection (MOI) of 0.5, and cultured at 37°C, 5%CO₂. In mock (negative control), PBS supplemented with 1%IFBS was added instead of the viral solution. After 1 hour, the viral solution was removed, DMEM supplemented with 1% IFBS was added, and the cells were cultured at 37°C, 5%CO₂. After 48 hours, the culture supernatant was collected and measured by ELISA method in accordance with the manufacturer's protocol.

### Example 10.4: Vascular endothelial cell tube formation test (in vitro)

The vascular endothelial cell tube formation test was performed using the BD BioCoat(TM) Angiogenesis System-Endothelial Cell Tube Formation (BD Bioscience, USA). Vero was seeded into a T-150 flask at 1 x 10⁷ cells, and cultured at 37°C, 5%CO₂. After 16 hours, the cells were infected with T-01, T-sVEGFR1 or T-VEGFscFv at MOI of 0.2, and cultured at 37°C, 5%CO₂. After 90 minutes, the viral solution was removed, DMEM (without serum) was added, and the cells were cultured at 37°C, 5%CO₂. After 2 days, the culture supernatant was collected, and human immunoglobulin was added at 50 µg/ml, and the virus was inactivated. The collected supernatant was ultrafiltered with Amicon(R) Ultra-15 10K (Merck Millipore, Ireland), and concentrated 40-fold. HUVEC were seeded at 2 x 10⁴ cells/well in 96-well plates coated with Matrigel Matrix. 2.5 µl of the concentrated culture supernatant and 47.5 µl of Medium 200 supplemented with 1%LSGS were added thereto, and the cells were cultured at 37°C, 5%CO₂. As a positive control, 50 µl of Medium 200 supplemented with 1%LSGS to which bevacizmab (Chugai Pharmaceutical Co., Ltd., Tokyo), an anti-VEGF humanized monoclonal antibody, was added at 4 µg/ml was added to each well. As a negative control, 50 µl of Medium 200 supplemented with 1%LSGS alone was added. After 18 hours, each well was imaged with BIOREVO (KEYENCE CORPORATION, Osaka), the length of the tube was measured by Image J software, and the total lengths was calculated and compared.

### Example 10.5: Vascular endothelial cell migration test (in vitro)

The vascular endothelial cell migration test was performed using the BD BioCoat(TM) Angiogenesis System-Endothelial Cell Migration (BD Bioscience, USA). Vero cells were seeded into T-150 flasks at 1 x 10⁷ cells, and cultured at 37°C, 5%CO₂. After 16 hours, the cells were infected with T-01, T-sVEGFR1 or T-VEGFscFv at MOI of 0.2, and cultured at 37°C, 5%CO₂. After 90 minutes, the viral solution was removed, DMEM (without serum) was added, and the cells were cultured at 37°C, 5%CO₂. After 2 days, the culture supernatant was collected, and human immunoglobulin was added at 50 µg/ml, and the virus was inactivated. The collected culture supernatant was ultrafiltered with Amicon(R) Ultra-15 10K, and concentrated 40-fold. Into the upper chamber, HUVEC was seeded at 1 x 10⁵ cells/well. To the lower 24-well plates, 19 µl of the culture supernatant concentrated and 731 µl of Medium 200 supplemented with 1% LSGS were added per well. As a positive control, 750 µl of Medium 200 supplemented with 1% LSGS, to which bevacizmab was added at 4 µg/ml, was added to each well. As a negative control, 750 µl of Medium 200 supplemented with 1%LSGS alone was added. After cultured at 37°C, 5%CO₂ for 22 hours, HUVEC was fluorescently stained with calcein AM solution. Each well was imaged with BIOREVO (KEYENCE CORPORATION, Osaka), then fluorescence intensity was measured at 494/517 nm, thereby HUVEC which passed through the membrane of the upper chamber and migrated to the lower well was evaluated.

### Example 10.6: Virus replication test (in vitro)

HT-29 was seeded into 6-well plates at 4 x 10⁵ cells/well, and cultured in McCoy's 5A (Modified) supplemented with 10% FBS under conditions of 37°C, 5%CO₂. After 16 hours, the cells were washed with PBS supplemented with 1% IFBS, and were infected with T-01, T-sVEGFR1 or T-VEGFscFv at MOI of 0.01, and cultured at 37°C, 5%CO₂. After 1 hour, the viral solution was removed, McCoy's 5A (Modified) supplemented with 1% IFBS was added, and the cells were cultured under conditions of 37°C, 5%CO₂. After 24 and 48 hours, the cells were peeled off with a cell scraper, and the titer of the virus recovered together with the supernatant was measured.

### Example 10.7: Study of cytopathic effects of virus (in vitro)

HT-29 was seeded into 6-well plates at 2 x 10⁵ cells/well, and cultured under conditions of 37°C, 5%CO₂. After 16 hours, the cells were washed with PBS supplemented with 1% IFBS, and were infected with T-01, T-sVEGFR1 or T-VEGFscFv at MOI of 0.1 and 0.01, and cultured at 37°C, 5%CO₂. In mock, PBS supplemented with 1% IFBS was added instead of the viral solution. After 1 hour, the viral solution was removed, McCoy's 5A (Modified) supplemented with 1%IFBS was added, and the cells were cultured under conditions of 34.5°C, 5%CO₂. After infection, the surviving cells were measured over 4 days, each after 24 hours, with a Coulter counter (Beckman Coulter, Inc., CA, USA). The number of the cells was calculated as a proportion relative to mock.

### Example 10.8: Study of VEGF expression amounts in HT-29 cell lines and CT26 cell lines (in vitro)

HT-29 or CT26 was seeded into 6-well plates at 3 x 10⁵ cells/well, and cultured under condition of 37°C, 5%CO₂, in McCoy's 5A (Modified) supplemented with 1% IFBS for HT-29, or in RPMI 1640 supplemented with 1%IFBS for CT 26. After 48 hours, the culture supernatant was collected. VEGF in the supernatant was quantified using Human VEGF Quantikine ELISA Kit (R&D Systems, Inc., USA) for HT-29, and using Mouse VEGF Quantikine ELISA Kit (R&D Systems, Inc., USA) for CT26.

### Example 10.9: Study of anti-tumor effects on HT-29 subcutaneous tumor models

Xylazine and ketamine were intraperitoneally administered to BALB/c nu/nu (5 weeks of age, females, Japan SLC, Inc., Shizuoka) for general anesthesia. HT-29 was then subcutaneously administered to the left abdomen of mice at 1 x 10⁶ cells/50 µl McCoy's 5A (Modified) (without serum) per mouse. On Day 0, when tumor diameter reached 5 mm after 14 days, the mice were randomly grouped into 4 groups, and T-01, T-sVEGFR1, T-VEGFscFv or Mock were administered into the tumors at 1 x 10⁶ pfu/20 µl per tumor. Viruses were diluted with 10% glycerol-added PBS. For mock, as a negative control, those prepared through the same culture and purification steps but without infecting Vero with a virus were used. On Day 3, 3 days after the first administration of the virus, the virus was administered again in a similar manner. The subcutaneous tumors were measured in long diameter, short diameter, and thickness at a frequency of twice a week, and calculated by tumor volume (mm³) = long diameter x short diameter x thickness.

### Example 10.10: CD31 immunohistochemical staining of HT-29 subcutaneous tumor models

General anesthesia was applied to BALB/c nu/nu (5 weeks of age, females, Japan SLC, Inc., Shizuoka). HT-29 was then subcutaneously administered to the left abdomen of mice at 1 x 10⁶ cells/50 µl McCoy's 5A (Modified) (without serum) per mouse. After 14 days, which was set as Day 0, mice were randomly grouped into 4 groups, and T-01, T-sVEGFR1, T-VEGFscFv or Mock was administered into the tumors at 1 x 10⁶ pfu/20 µl. The virus was administered on Day 3 in a similar manner. Mice were euthanized on Day 3 (prior to the second dose of the virus) and Day 7 by cervical dislocation. The subcutaneous tumor tissue was excised and immobilized with formalin. After 24 hours, the immobilized tissue was washed with PBS and paraffin-embedded. The paraffin sections of 4 µm thick were treated with a 200-fold diluted immunosaver (Nisshin EM Co., Ltd., Tokyo) at 98°C for 45 minutes to activate antigens. After blocking with 2% Normal Goat Serum/TBS, the tissue sections were reacted with rabbit anti-CD31 antibodies (Abcam plc., UK) diluted 50-fold with 2% BSA/TBS, as a primary antibody, for 1 hour. The sections were then reacted with EnVision+System-HRP Labeled Polymer Anti-Rbbit (Dako, Denmark) for 30 minutes, and antibodies were detected with DAB Substrate kit (VECTOR LABORATORIES, INC., Burlingame, CA). Tumor angiogenesis was evaluated by microvessel density in tumors. The microvessel density was set, according to the article by Weidner N et al. (19), as the average of microvessels of the tissue section per field of view under a microscope (x 20). The average number was calculated by imaging tissue sections randomly at 10 locations with Nanozoomer (HAMAMATSU, Shizuoka), counting CD31-positive cells, and averaging the results of counts.

### Example 10.11: Quantification of viral DNA by Real Time Quantitative Polymerase Chain Reaction (PCR)

General anesthesia was applied to BALB/c nu/nu (5 weeks of age, females, Japan SLC, Inc., Shizuoka). HT-29 cells were then subcutaneously administered to the left abdomen of mice at 1 x 10⁶ cells/50 µl McCoy's 5A (Modified) (without serum) per mouse. After 14 days, which was set as Day 0, the mice were randomly grouped into 4 groups, and T-01, T-sVEGFR1, T-VEGFscFv or Mock was administered intratumorally at 1 x 10⁶ pfu/20 µl. Mice were euthanized on Day 3 by cervical dislocation. The subcutaneous tumors were then excised, and stored frozen at -80°C. After thawing at a later date, PBS was added, and the tumors were homogenized, and then the viral DNA was extracted using QIAamp DNA Mini Kit according to the manufacturer's protocol. As primers and probes for detecting DNA of HSV-1, those shown below, which were designed for the region of the gene encoding DNA polymerase of HSV-1, were used:
HSV DNApoly-F: 5'-GGCACGCGGCAGTACTTT-3' (SEQ ID NO: 27)
HSV DNApoly-R: 5'-CCATGCGCTCGCAGAGA-3' (SEQ ID NO: 28)
HSV DNApoly-T: 5'-AGGTCGACAGGCACCTACAATGCCG-3' TAMRA (Reporter dye: FAM)(SEQ ID NO: 29)

TaqMan Fast Universal PCR Master Mix (2x) (Applied Biosystems, USA) was used for Real Time Quantitative PCR. The quantification of the copy number of virus genome was performed by preparing a plasmid containing a sequence of the gene encoding DNA polymerase of HSV-1, preparing serial dilutions of the DNA, generating a calibration curve, and measuring the copy number based on the curve.

### Example 10.12: Study of anti-tumor effects on CT26 subcutaneous tumor models

Xylazine and ketamine were intraperitoneally administered to BALB/c (5 weeks of age, females, Japan SLC, Inc., Shizuoka) for general anesthesia. CT26 from BALB/c was subcutaneously administered to the left abdomen of mice at 1 x 10⁵ cells/50 µl RPMI 1640 (without serum) per mouse. On Day 0, when the tumor diameter reached 5 mm after 10 days, the mice were randomly grouped into 4 groups, and T-01, T-sVEGFR1, T-VEGFscFv or Mock was administered intratumorally at 1 x 10⁶ pfu/20 µl per mouse. The virus was also administered on Day 3, 3 days after the first administration of the virus. The subcutaneous tumors were measured in long diameter, short diameter, and thickness at a frequency of twice a week, and calculated by tumor volume (mm³) = long diameter x short diameter x thickness.

### C. Statistical analysis

Statistical analysis was performed, unless otherwise noted, by student t test using Excel Statistics (Microsoft Corporation) for two-group tests, while by multiple test by Sidak method using SPSS Statistics version 22 (IBM Corporation) for multi-group tests. In the evaluation experiment for swelling by MRI, repeated ANOVA was performed with the consideration of repeating measurements on the same individual. In the experiments for those with alternating actions, multiple tests by Sidak method were performed. For survival of brain tumor models, a cumulative survival curve was drawn by Kaplan Meier method using JMP12 (SAS Institute Inc.), and Logrank or Wilcoxon test was performed for statistical test. A significant difference between groups was defined when the p-value was less than 0.05.

### D. Results

### Result I: Generating T-BV

### Result Ia: Generating anti-VEGF antibody-expressing cDNA (Result of Example 1.1)

The design of amino acid sequences for generating an oncolytic virus that expresses an anti-VEGF antibody is as shown in Figures 4 and 5. Bevacizumab, the anti-VEGF antibody used in this Example, is a humanized anti-human VEGF monoclonal antibody produced by, based on the antibody gene of a mouse anti-human VEGF monoclonal antibody muMAb A4.6.1-producing line selected from a hybridoma cell line obtained by immunizing mice with 165 residues of human VEGF (VEGF₁₆₅), remaining the complementarity determining region (CDR) involved in antigen specificity as it is, and humanizing the framework region (FR). For efficient antibody production from cDNA, it is necessary that the heavy and light chains are expressed in equal amounts. Thus, in designing the amino acid sequence, a method of binding polypeptides each corresponding to the heavy and light chains with the FMDV-2A sequence and an amino acid sequence of the furin cleavage site was employed (Figure 4, Figure 1).

Sequence information of cDNA of an anti-human VEGF antibody expression gene prepared from the amino acid sequence, and cDNA of a VEGF scFv expression gene is shown in Figures 9 and 10, respectively.

### Result Ib: Insertion of gene of interest into SV-01 and confirmation of protein expressed from cDNA (Results of Example 1.2, Example 1.3, and Example 6.1)

From plasmid pEX-K-BV containing the generated cDNA, cDNA of interest was excised by restriction enzyme treatment, and inserted into SV-01 to obtain BV/SV-01. Subsequently, for the purpose of confirming protein expression from the cDNA of interest, western blotting was performed on proteins contained in the culture supernatant obtained by transfecting BV/SV-01 into HEK293T cells. In general, two types of electrophoresis, SDS-PAGE and native PAGE, are used in analysis by western blotting for antibodies. In SDS-PAGE, two bands corresponding to heavy chain (50 kDa) and light chain (25 kDa) are detected because electrophoresis is performed after the protein is reduced and the disulfide bonds are cleaved, and this process results in the cleavages of the disulfide bond that connects the heavy chains of the antibody to each other and the disulfide bond that connects the heavy chain and the light chain of the antibody. Meanwhile, in native PAGE, the protein is electrophoresed without denaturation, thus it is possible to perform electrophoresis while maintaining the higher order structure of the protein, and only one band equivalent to the tetramer (150 kDa) is detected. Analysis of the protein expressed from the generated cDNA in this Example was performed by these two types of PAGE.

In SDS-PAGE, no band was detected in a specimen transfected with a negative control, SV-01, whereas in a specimen transfected with BV/SV-01 and a positive control, Avastin(registered trademark), two bands corresponding to molecular weights 25 kDa and 50 kDa were detected (Figure 11A). In native PAGE, no band was detected in specimens transfected with SV-01, whereas in a specimen transfected with BV/SV-01 and Avastin(registered trademark), one band corresponding to molecular weight 150 kDa was detected (Figure 11B). These results showed that the expressed protein from the anti-human VEGF antibody expression gene used for gene recombination in this experiment is a double-stranded antibody without contradiction.

### Result Ic: Insertion of gene of interest into G47Δ and viral purification (Results of Examples 1.4-1.7)

Subsequently, BV/SV-01 was inserted into T-BAC. SV-01 and T-BAC contain loxP sites, and thus BV/SV-01/T-BAC was generated by Cre-recombination. PCR was performed at this stage for structural confirmation. The insertion of BV/SV-01 was observed in all 10 clones harvested, and double insertion was observed in one of the clones. Two clones were selected from the clones in which single insertion was observed in this PCR, and each DNA was treated with restriction enzymes HindIII or KpnI and the electrophoresis patterns were confirmed. In those treated with HindIII, when BV/SV-01 was inserted, 2,261 bp of the anti-human VEGF antibody expression gene was additionally confirmed, and a band of 14,986 bp was disappeared due to two more sites cleaved by HindIII, thereby bands of 13,151 bp, 7,958 bp, and 6,233 bp each were appeared. In those treated with KpnI, it was expected that, only when BV/SV-01 be inserted, 2,261 bp of the anti-human VEGF antibody expression gene be additionally appeared, and a band of 3,853 bp be disappeared due to five more sites cleaved by KpnI, thereby bands of 9,010 bp, 5,122 bp, 175 bp, 1,040 bp, 677 bp, 728 bp each be appeared. In fact, the expected electrophoresis pattern was obtained. From the above results, it was confirmed that the two clones were recombinant as planned. Then, the clones were mixed with an FLP recombinase-expressing plasmid pOG44, co-transfected into Vero cells, and BAC sequences were removed. Since BAC contains GFP expression genes, removal of BAC was confirmed by confirming disappearance of GFP expression by fluorescence microscopy. Viruses were collected, and extraction of a single clone by limit dilution method was performed.

### Result Id: Structural confirmation of T-BV (Result of Example 2)

For the final product, T-BV, the structural confirmation was performed by Southern blot method using DNA extracted from the virus. As the probes, a DNA probe corresponding to an inserted anti-human VEGF antibody expression gene (BV probe) and a probe corresponding to a LacZ gene region (LacZ probe) were used. When hybridized with the BV probe, no band was detected in T-01, while a band of 13,151 bp was detected in T-BV, as expected. When hybridized with the LacZ probe, a band of 13,515 bp was detected in T-BV, instead of a band of 10,890 bp detected in T-01, confirming that the anti-human VEGF antibody expression gene was inserted at the site as planned.

From the above processes, it was confirmed that the T-BV of interest was generated.

### Result II: Evaluation of T-BV-expressing antibodies

### Result IIa: Quantification of T-BV-expressing antibodies (Result of Example 6.2)

First, for quantification of antibody expression of T-BV in vitro, ELISA against anti-human VEGF antibodies was performed using culture supernatants of virus-infected cells of Vero cells and U87MG cells. In Vero cells and U87MG cells, anti-VEGF antibodies were below the detection limits from the culture supernatants infected with T-01 or mock, whereas 256 pg/mL and 69 pg/mL anti-VEGF antibodies were respectively detected from the culture supernatant infected with T-BV (Figure 12) .

Subsequently, quantification of antibody expression of T-BV in vivo was performed. U87MG subcutaneous tumors were subcutaneously generated in the left abdomen of BALB/c nu/nu mice. The mice were randomly divided into the T-01, T-BV, and mock groups by 9 mice each. Then, 2 x 10⁶ pfu viruses were administered into the tumors in a single dose. Subcutaneous tumors were removed from 3 mice in each group at PIDs 2, 4, and 6, and anti-human VEGF antibodies contained in the tumors were quantified by ELISA. For the T-BV group, the T-01 group, and the mock group, the amount of the antibody was: 186.2 pg/mL, 44.0 pg/mL, and 38.8 pg/mL at PID 2, respectively; 93.4 pg/mL, 19.6 pg/mL, and 41.4 pg/mL at PID 4, respectively; and 137.8 pg/mL, 44.1 pg/mL, and 55.7 pg/mL at PID 6, respectively (Figure 13). Although non-specific reactions were seen in mock and T-01 due to high protein concentrations of tissue suspensions used in ELISA with subcutaneous tumors, measurements were significantly higher in the T-BV group than in mock and T-01 at any time point, suggesting expression of anti-human VEGF antibody in vivo.

### Result IIb: Confirmation of functions of T-BV expressing antibodies (Result of Example 7)

To examine the VEGF inhibitory effect of the proteins expressed by T-BV, a vascular endothelial cell tube formation test and a vascular endothelial cell migration test were performed.

The vascular endothelial cell tube formation test is an experimental method which utilizes the phenomenon of tube formation of vascular endothelial cells being induced by angiogenic stimulating factors such as VEGF in a Matrigel basement membrane matrix uniformly coated in a plate, and is used for screening angiogenic stimulating factors by quantifying the length of the tubes formed. In the present example, the total tube length (µm) was 817, 26,558, 16,408, 1,117, 24,267, and 7,358 in the order of n.c., mock, p.c.1, p.c.2, T-01, and T-BV, respectively, showing that T-BV significantly suppressed tube formation relative to T-01 (p = 0.002) (Figure 14).

The vascular endothelial cell migration test is an experimental method which utilizes the phenomenon of vascular endothelial cells migrating towards angiogenic stimulating factors such as VEGF and is used for screening angiogenic stimulating factors by placing angiogenic stimulating factors such as VEGF in one of the two compartments separated by membrane uniformly coated with human fibronectin and placing vascular endothelial cells in the other, and quantifying vascular endothelial cells migrated by stimulation of angiogenic stimulating factors through pores of the membrane. In this example, fluorescence intensity relative to n.c. was 2.76, 2.63, 0.62, 2.85, 1.77 in the order of mock, p.c.1, p.c.2, T-01, T-BV, showing that T-BV significantly suppressed HUVEC-2 migration relative to T-01 (p = 0.001) (Figure 15) .

These experimental results showed that the expressed antibody of T-BV has a VEGF inhibitory effect.

### Result IIc: Study of interspecies cross-reactivity of expressed antibodies of T-BV (Result of Example 8)

It is generally known that VEGF has interspecies cross-reactivity, whereas bevacizumab, an anti-human VEGF antibody, is highly specific for human VEGF and has little response to mouse VEGF (Yu, L., et al. (2008). Investigative ophthalmology & visual science 49, 522-527). When, as like this example, a mouse model in which a human brain tumor cell line is implanted is used, it is expected that both human and mouse VEGF act at the tumor sites. Thus, it was considered that it is important to confirm the binding ability of the expressed antibody of T-BV to human and mouse VEGFs in discussing the mechanism of the effects of T-BV. Thus an evaluation experiment was performed by ELISA for binding ability of the T-BV-expressing antibody to VEGFs of human and mice each.

The anti-mouse VEGF antibody bound to both mouse VEGF and human VEGF, while the T-BV-expressing antibody did not bind to mouse VEGF and only bound to human VEGF, indicating the same result as Avastin(registered trademark) (Figure 16). From this result, it was considered that the expressed anti-human VEGF antibody of T-BV has high species-specificity for human VEGF as Avastin(registered trademark).

### Result III: Evaluation of T-BV as a virus

### Result IIIa: Study of cytopathic effects of T-BV in vitro (Result of Example 4)

To confirm whether T-BV inserted with the gene of interest by gene recombination has cytopathic effects in vitro equivalent to T-01, in vitro cytotoxicity assays were performed on U87MG cells. Furthermore, to confirm the cytopathic effect on a glioma stem cell that is considered as a factor of treatment difficulties in glioblastoma because of its resistance to both chemotherapy and radiation therapy, in vitro cytotoxicity assays using TGS-01 cells and TGS-04 cells were also performed.

For U87MG cells, T-01 and T-BV had cell viability (ratio relative to surviving cell number in the mock group) after 4 days of infection of 60.9% and 67.9%, respectively, at MOI of 0.01; and 20.8% and 19.6%, respectively, at MOI of 0.1; showing equivalent high cytopathic effects (p = 0.18 and p = 0.15, respectively).

For TGS-01 cells, T-01 and T-BV had cell viability of 25.6% and 43.6%, respectively, at MOI of 0.01; 4.1% and 6.6%, respectively, at MOI of 0.1; and 2.0% and 1.8%, respectively, at MOI of 1 (p < 0.01, p = 0.10, p = 0.84, respectively).

For TGS-04 cells, T-01 and T-BV had cell viability of 12.5% and 25.8%, respectively, at MOI of 0.01; 5.4% and 7.1%, respectively, at MOI of 0.1; and 6.1% and 5.5%, respectively, at MOI of 1 (p = 0.08, p < 0.05, p = 0.33, respectively).

T-BV had the almost same shape of graph as T-01 in any cell line, and thus showed cytopathic effects equivalent to T-01 (Figure 17).

### Result IIIb: Study of replicability of T-BV in vitro (Result of Example 5)

Subsequently, viral replication tests of T-01, T-BV in Vero cells and U87MG cells were performed. In Vero cells, the viral titers of T-01 and T-BV was 9.71 x 10⁴ pfu/mL and 1.22 x 10⁵ pfu/mL, respectively, after 24 hours; and 1.32 x 10⁶ pfu/mL and 1.24 x 10⁶ pfu/mL, respectively, after 48 hours (p = 0.06, and 0.43, respectively). In U87MG cells, the viral titers of T-01 and T-BV were 6.02 x 10³ pfu/mL and 7.81 x 10³ pfu/mL, respectively, after 24 hours; and 2 x 10⁵ pfu/mL and 2.16 x 10⁵ pfu/mL, respectively, after 48 hours (p = 0.29, and 0.53, respectively). In both cells, T-BV was considered to have approximately comparable replicability to T-01 (Figure 18). This is important in exhibiting sufficient cytopathic effects and function by protein expression, with the consideration that the virus destroys tumor cells during the process of replication and that the protein of interest is expressed from the therapeutic gene along with the replication.

### Result IV: Study of anti-tumor effects of T-BV in vivo

### Result Iva: Study of anti-tumor effects of T-BV in U87MG subcutaneous tumor models (Result of Example 9.1.1)

U87MG subcutaneous tumor models were generated, then, when tumor diameters reached 6 mm, the mice were randomly divided into 3 groups of T-01, T-BV and mock by 10 mice each. 2 x 10⁵ pfu viruses were administered intratumorally twice on day 0 and day 3. In comparison by independent t-test using Excel statistics (Microsoft Corporation), at the time point 20 days after the administration of viruses, a significant tumor growth suppressive effect was observed in the T-01 group relative to the mock group (p = 0.00749). Furthermore, a significant tumor growth suppressive effect was observed in the T-BV group relative to the T-01 group (p = 0.0211) (Figure 19).

### Result IVb: Study of anti-tumor effects of T-BV in U87MG brain tumor models (Result of Example 9.1.2)

A study of anti-tumor effects using a U87MG brain tumor model was performed. Brain tumor models were randomly divided on day 10 into 3 groups of T-01, T-BV and mock by 10 mice each, and 1 x 10⁶ pfu viruses were administered in a single dose into tumors. Significant prolonged survival was observed in the T-01 and T-BV administered groups relative to the mock group (p < 0.01 in both). T-BV showed more prolonged survival tendency relative to T-01 (p = 0.163) (Figure 20).

The above results showed that T-BV has a higher anti-tumor effect in vivo relative to T-01.

### Result IVc: Study of anti-tumor effects of T-BV in TGS-01 and TGS-04 brain tumor models (Result of Example 9.1.2)

Brain tumor models with glioma stem cells, TGS-01 and TGS-04, form borderless tumors that show invasive images to the opposite side with low cell counts, and show pathological images closer to glioblastoma in real clinical practice. Glioma stem cells show resistance to radiation therapy and chemotherapy, which is a factor that makes glioma refractory. For the purpose of examining the effect in vivo on glioma stem cells, brain tumor models of TGS-01 and TGS-04 were prepared, and treatment experiments in which 2 x 10⁶ pfu of T-01, T-BV or mock were administered in a single dose into the tumors by 10 mice in each group were performed. In the TGS-01 brain tumor models, significant prolonged survival was observed in both T-BV and T-01 relative to mock (p < 0.01 in both), and further, significant prolonged survival was observed in T-BV relative to T-01 (p = 0.0475). In the TGS-04 models, no prolonged survival was observed in T-01 relative to mock, while significant prolonged survival was observed in T-BV (p = 0.0495) (Figure 21).

This showed that T-BV has a higher anti-tumor effect on glioma stem cells relative to T-01 in vivo.

### Result V: Study of swelling-suppressive effects of T-BV

For U87MG brain tumor models, the changes that occur in the high intensity area in T2WI when T-BV is administered relative to when T-01 is administered was evaluated by MRI for small animals.

Furthermore, since there have been reports that AQP4 has a strong association with brain edema development, quantification of mouse AQP4 expression by RT-qPCR was also performed to verify the swelling-suppressive effects of T-BV.

### Result Va: Quantification of swelling by MRI

In MRI, a vascular tumor is enhanced and depicted as a high intensity area in T1WI due to reduction of the longitudinal relaxation time by paramagnetic contrast agent-administration. On the other hand, a tissue containing free water, such as edema, is depicted as a high intensity area in T2WI due to its long lateral relaxation time. In clinical trials of viral therapy against glioblastoma, the tumor is depicted as a high intensity area in T1WI (CE), and edema occurred after virus administration is depicted as a high intensity area in T2WI. Thus, in this Example, the swelling-suppressive effects of T-BV was evaluated by taking MRI imaging over time after virus administration into the tumors of the brain tumor models, and measuring the high intensity area around the tumor in T1WI (CE) and T2WI.

### Result Vb: Study of animal models

For each of the U87MG, U251MG, NMC-G1, TGS-01, and 1123/M cell lines, the cells were implanted into the right frontal lobe of BALB/c nu/nu mice using a stereotaxic brain operation device, and the MRI images were taken over time. U87MG, which was the easiest to depict tumors by MRI, was used (Figure 22). It should be noted that U251MG and NMC-G1 had slow swelling formation rates, and thus had difficulties to obtain contrast enhancement stable as U87MG, TGS-01 and 1123/M tumors.

### Result Vc: MRI evaluation after virus administration in U87MG brain tumor models (Result of Example 9.2.1)

U87MG brain tumor models were imaged by MRI on day 11 after tumor implantation, and the tumor size and shape were examined. The mice were then randomly grouped into the T-01, T-BV, T+A, and mock groups by 6 mice each. Single doses of 1 x 10⁶ pfu were administered into the tumors in a single dose on day 12, and MRI images were taken at PIDs 2, 4, and 6. The area was measured based on the image, and the area ratio was calculated based on the aforementioned calculation formula. The area ratio over PID 2-4-6 was shifted by 1.32-1.32-1.20 in the T-01 group, while shifted by 1.12-1.06-1.04 and 1.01-1.01-1.04 in the T-BV group and the T+A group, respectively, resulting in significantly low in the T-BV group and the T+A group relative to the T-01 group (p = 0.015, and 0.002, respectively) (Figure 23). This result showed that brain swelling is reduced by T-BV administration or T-01 plus Avastin(registered trademark) systemic administration.

### Result Vd: Quantification of AQP4 after virus administration by RT-qPCR (Result of Example 9.2.2)

AQP was discovered in 1992 as a membrane protein that selectively transmits water molecules. Thirteen types from AQP0 to AQP12 have been reported in mammals. Structurally, many of them are six transmembrane proteins composed of no more than 300 amino acids, characterized by the presence of a highly conservative moiety of asparagine (N)-proline (P)-alanine (A), called NPA box, in two locations. The moiety is highly hydrophobic, and is folded into the membrane without penetrating the membrane to form a passage of water molecules. Many types of AQP including AQP1, AQP3, AQP4, AQP5, AQP8, AQP9, AQP11, and AQP12 are reported to be expressed in brain. Among them, AQP4 is highly expressed in brain relative to other organs, and from its distribution, it is believed that AQP4 is involved in the movement of water in the blood-brain barrier. AQP4 has also been reported to be strongly associated with the development of brain edema, and is likely involved in the early stages of brain edema development. Quantification on mouse AQP4 by RT-qPCR was thus performed using the right frontal lobe of U87MG brain tumor models after virus administration for the purpose of quantifying swelling changes after virus administration.

For U87MG brain tumor models, the T-01, T-BV, T+A, and mock groups were used. The right frontal lobes were removed at PID6 to extract RNA, then cDNA was prepared with reverse transcriptase, and then quantitative PCR on mouse AQP and mouse β-actin was performed with TaqMan primers. AQP4 expression was significantly increased in the T-01 group relative to the mock group (p < 0.001), and AQP4 expression was significantly decreased in the T-BV group and the T+A group relative to the T-01 group (p < 0.001) (Figure 25). From this result, it was also considered that brain swelling is reduced by T-BV administration or T-01 plus Avastin(registered trademark) systemic administration.

From the results of MRI and RT-qPCR on AQP4, it was shown that T-BV has the same swelling-suppressive effect as the systemic administration of Avastin(registered trademark) for swelling after virus administration to brain tumors.

### Result VI: Comparative study of swelling-suppressive effects of T-BV and T-V_{H}V_{L} (Result of Example 9.2.3)

To U87MG brain tumor models, 1 x 10⁶ pfu of T-01, T-BV, T-V_{H}V_{L} or mock was administered, and then MRI images were taken over time (Figure 25). Significant decreases of swelling were observed at PID 4 in T-BV relative to T-01 (p = 0.039). A tendency of swelling suppression was also observed in T-V_{H}V_{L} (p = 0.397) (Figure 26).

### Result VIII: Effects on the tumor environment after virus administration

It has been reported that anti-VEGF antibodies have effects on immunity as well as angiogenesis suppression effects (Roland, C. L. et al. (2009) PloS one 4, e7669, (2009)). Thus, evaluations of angiogenesis and macrophages by immunohistochemical staining were performed using U87MG brain tumor models administered with various viruses.

### Result VIIIa: Immunohistochemical staining (Result of Example 9.3.1)

To U87MG brain tumor models, T-01, T-BV, T+A or mock was administered. The brain was removed at PIDs 2 and 4, and then paraffin sections were made, and a study by immunohistochemical staining was performed. To evaluate changes due to virus administration, the virus infection of the tumor was confirmed by HSV-1 staining, and then mouse CD31 staining was performed for an evaluation of angiogenesis and mouse F4/80 staining was performed for an evaluation of infiltration macrophages, respectively.

In the mouse CD31 staining, the vascular shape and distribution were uniform in the mock group, whereas the vascular diameter was thick and the vascular distribution was non-uniform in the virus administration group. The vessel density tended to be higher in the virus administration groups relative to the mock group, but neither significant difference nor tendency were observed among the virus administration groups. No obvious suppression of angiogenesis was observed in T-V_{H}V_{L}, either.

For the evaluation of infiltration macrophages, the infiltration of macrophages into tumors was observed in the mock group, but the distribution overall was sparse and uniform. While, in the virus administration group, the pathological images showing that the infiltration macrophages were densely clustered mainly at viral infected sites were obtained, and no apparent differences were observed among the viral groups.

### Result VIIIb: Flow cytometry (Results of Example 9.3.2)

In the immunohistochemical staining of the U87MG brain tumor models, pathological image showing the macrophages clustered mainly at viral infected sites were obtained in the virus administration group. Thus, quantification by flow cytometry was then performed. Macrophages are categorized into M1 macrophages and M2 macrophages. M1 macrophages are induced by bacterial or viral infections, and exert a strong antibacterial or antiviral activity, and an anti-tumor effect. Meanwhile, M2 macrophages have functions of tissue repair and angiogenesis, tumor growth promotion, and immunosuppression. It is believed that, depending on the effects of various cytokines associated with tumor progression, tumor-associated macrophages (TAMs) infiltrating tumor tissue shifts from M1 to M2. Thus, flow cytometry was performed to examine how TAMs changes when viruses are administered to the tumors and what differences are there in the changes depending on the viruses administered, by using the right frontal lobes at PIDs 2, 4, and 6 for U87MG brain tumors. A representative scatter diagram is shown in Figure 27. In the mock group, M2 dominance was consistently observed at any time point. In contrast, in any virus administration group, M1 dominant states were observed at PID2. Thereafter, in the T-01 group, the M1 dominant state shifted toward the original M2 dominant state at PID6, while in the T-BV group and the T+A group, the states where the proportions of M1 and M2 were approximately equal were maintained at PID 6, and the M1 dominant state relative to that in the mock group was maintained (Figure 28-1).

As noted above, M1 macrophage has antiviral activity. Thus, it was examined how the persistent M1 dominance affects viral titer. No significant differences were seen in viral titer of the T-01, T-BV and T+A groups at any of PID 1, PID 3 and PID 7 (Figure 28-2). From these results, it was suggested that expression of anti-human VEGF antibody can confer anti-tumor activity on HSV-1 by making macrophages M1 dominant, without adversely affecting viral titer.

Based on the results of conventional studies on M1 macrophages, the maintenance of the M1 dominant state is considered to be a factor of the high anti-tumor effect of T-BV, as confirmed in this experiment. Since M1 macrophages have phagocytosis activity, it was considered that viral titer may be reduced by M1 dominance, but surprisingly, no negative effects on viral replication were seen, and the high anti-tumor effects of T-BV did not affect the viral titer.

### Result X: Confirmation of antibody expression of T-BV in vivo (Result of Example 9.4)

Concerning safety, it is important to confirm the distribution of proteins expressed upon administration of functionalized viruses to organisms. Thus, the detectable amount in blood of the anti-human VEGF antibody expressed upon administration of T-BV to brain tumors in vivo was evaluated.

U87MG brain tumors were generated in the right frontal lobes of BALB/c nu/nu mice. The mice were randomly divided into the T-01, T-BV, T+A, and mock groups by 6 mice each. Then, 1 x 10⁶ pfu viruses were administered in a single dose into the tumors. Venous blood sampling was performed at PIDs 1 and 3 by 3 mice in each group, and anti-human VEGF antibodies in the isolated serum were quantified by ELISA. Only in the T+A group, where a single dose of Avastin(registered trademark) was administered intraperitoneally, 114.5 ng/mL and 106.9 ng/mL anti-VEGF antibodies were detected at PID1 and PID3, respectively. However, in the other groups, including the T-BV group, the antibodies were below the detection limit (Figure 29).

### Result XI: Result of evaluation of T-sVEGFR1 and T-VEGFscFv (Result of Example 10)

### (1) Confirmation by Southern blotting method of viral genomic structure of T-sVEGFR1, T-VEGFscFv (VH-VL) and T-VEGFscFv (VH-VLCL)

First, in conducting experiments using T-sVEGFR1, T-VEGFscFv (VH-VL) and T-VEGFscFv (VH-VLCL), DNA was extracted from each of the purified viruses and Southern blotting was performed to check whether the genes of VEGF inhibitors were correctly incorporated into the respective viral DNA. With VEGFscFv (VH-VL) probes, 5543 bp and 5868 bp DNA fragments were detected in T-VEGFscFv (VH-VL) and T-VEGFscFv (VH-VLCL), respectively, confirming the insertion of genes VEGFscFv (VH-VL) and VEGFscFv (VH-VLCL) (not illustrated). Then, in Southern blotting with sVEGFR1 probes, 6798 bp DNA fragments were detected in T-sVEGFR1, confirming the insertion of a gene sVEGFR1 (not illustrated). From the above, it was confirmed that the genes of VEGF inhibitors were correctly inserted in T-sVEGFR1, T-VEGFscFv (VH-VL) and T-VEGFscFv (VH-VLCL) at the ICP6 deletion site of the basic backbone of G47Δ.

### (2) Confirmation of VEGF inhibitor protein expression in T-sVEGFR1 and T-VEGFscFv (in vitro)

To confirm whether sVEGFR1 and VEGFscFv proteins were secreted in the culture supernatant upon infection of cells with T-sVEGFR1 and T-VEGFscFv, respectively, measurements were made by ELISA method. As a result, sVEGFR1 was detected at a concentration of 1.66 ± 0.30 ng/ml in the T-sVEGFR1 infected group (Figure 30A), and VEGFscFv protein was detected at a concentration of 87.30 ± 7.99 ng/ml in the T-VEGFscFv infected group (Figure 30B), respectively.

### (3) Study of anti-VEGF functions of T-sVEGFR1 and T-VEGFscFv (in vitro)

Since the expressions of VEGF inhibitory factors could be confirmed in T-sVEGFR1 and T-VEGFscFv, respectively, a vascular endothelial cell tube formation test and a vascular endothelial cell migration test were then performed to examine whether the factors actually have anti-VEGF functions, and comparison to T-01 and evaluation were performed.

### a. Vascular endothelial cell tube formation test

A vascular endothelial cell tube formation test was performed to examine the effect of T-sVEGFR1 and T-VEGFscFv on the tube formation of HUVEC, a vascular endothelial cell. The cultured supernatant of the virus and HUVEC were co-cultured in Matrigel Matrix, and the effect on tube formation of the virus cultured supernatant was compared in the total tube length of HUVEC after 22 hours. As a result, the tube formation was significantly suppressed in the T-sVEGFR1 and T-VEGFscFv infected groups relative to the T-01 infected group (Figure 31).

### b. Vascular endothelial cell migration test

Next, a vascular endothelial cell migration test was performed to examine the effect of T-sVEGFR1 and T-VEGFscFv on the migration of HUVEC. The cultured supernatant of the virus and HUVEC were co-cultured. After 16 hours, the migrated HUVEC was fluorescently stained. The fluorescence intensity was then measured, and the effect of the virus cultured supernatant on the migration of HUVEC was compared. As a result, migration was significantly suppressed in the T-sVEGFR1 and T-VEGFscFv infected groups relative to the T-01 infected group (Figure 32).

These experimental results showed that T-sVEGFR1 and T-VEGFscFv have anti-VEGF functions in vitro.

### (4) Virus replication test (in vitro)

It is known that there is a possibility that viral replication is impaired when a gene is inserted into a virus. Thus, in vitro viral replication assays were performed with HT-29 to examine whether T-sVEGFR1 and T-VEGFscFv had replicability equivalent to T-01. As a result of each measurement of the titer of the replicated virus, the viral titers of T-01, T-sVEGFR1 and T-VEGFscFv after 24 hours were 6.43 ± 0.47 times, 9.94 ± 2.95 times, and 16.07 ± 11.28-fold the amount of virus infected, respectively. Also the viral titers of T-01, T-sVEGFR1 and T-VEGFscFv after 48 hours were 51.79 ± 3.09 times, 69.04 ± 8.25 times, and 88.10 ± 41.70 times, respectively (Figure 33).

From the above, T-sVEGFR1 and T-VEGFscFv were shown to have approximately equivalent replicability to T-01 in vitro.

### (5) Study of cytopathic effects of virus (in vitro)

To examine whether T-sVEGFR1 and T-VEGFscFv had cytopathic effects equivalent to T-01, in vitro cytotoxicity assay was performed with HT-29. As a result, cell viability after 4 days was 4.1 ± 0.25% in the T-01 infected group, 3.9 ± 2.34% in the T-sVEGFR1 infected group, and 8.4 ± 0.78% in the T-VEGFscFv infected group, at MOI of 0.1. Also, it was 38 ± 7.65% in the T-01 infected group, 43 ± 0.71% in the T-sVEGFR1 infected group, and 49 ± 6.68% in the T-VEGFscFv infected group, at MOI of 0.01 (Figure 34).

From the above, T-sVEGFR1 and T-VEGFscFv were shown to have approximately equivalent cytopathic effects to T-01 in vitro.

### (6) Study of VEGF expression amount in HT-29 cell lines and CT26 cell lines (in vitro)

Next, ELISA was performed to confirm whether HT-29 and CT26 secrete VEGF proteins in in vivo experiments, upon studying the anti-tumor effects of T-sVEGFR1 and T-VEGFscFv expressing VEGF inhibitory factors with comparison to that of T-01 in vivo.

When VEGF protein quantity in the culture supernatant of HT-29 or CT26 was measured, VEGF proteins were detected (human VEGF: 709.5 ± 129.0 [pg/ml], mouse VEGF: 682.7 ± 3.3 [pg/ml]), confirming that HT-29 and CT26 were cell lines that produce VEGF protein. These cell lines were thus considered suitable for the anti-tumor effect study of T-sVEGFR1 and T-VEGFscFv in vivo.

### (7) Study of the anti-tumor effects of T-sVEGFR1 and T-VEGFscFv on HT-29 subcutaneous tumor models

To study the anti-tumor effects of T-sVEGFR1 and T-VEGFscFv in vivo, a nude mouse subcutaneous tumor model was generated using HT-29 which had been confirmed to produce VEGF, and a therapeutic effect was observed.

HT-29 was subcutaneously administered to the left abdomen of nude mice to generate subcutaneous tumors. On Day 0, when tumor diameter reached 5 mm after 14 days, and Day 3, thereafter 3 days, T-01, T-sVEGFR1, T-VEGFscFv and Mock were administered into the subcutaneous tumors.

On Day 32, significantly strong anti-tumor effects were observed in the T-sVEGFR1 and T-VEGFscFv administration groups relative to the T-01 administration group (Figure 35). It should be noted that anti-tumor effects were comparable between the T-sVEGFR1 administration group and the T-VEGFscFv administration group.

From this result, it was shown that T-sVEGFR1 and T-VEGFscFv have significantly strong anti-tumor effects relative to T-01 in HT-29 subcutaneous tumor models.

### (8) Study of intratumoral angiogenesis in HT-29 subcutaneous tumor models by CD31 immunohistochemical staining

In HT-29 subcutaneous tumor models, T-sVEGFR1 and T-VEGFscFv exhibited significantly higher anti-tumor effects than T-01. Thus, the effect of the virus on intratumoral angiogenesis was studied to clarify the mechanism for enhancing the anti-tumor effects of T-sVEGFR1 and T-VEGFscFv.

In the intratumoral vessels study, HT-29 was subcutaneously administered to the left abdomen of nude mice to generate subcutaneous tumors. On Day 0, when tumor diameter reached 5 mm after 14 days, and Day 3, T-01, T-sVEGFR1, T-VEGFscFv and Mock were administered into the subcutaneous tumors. Subcutaneous tumor tissues were harvested on Day 3 (prior to the second dose of the virus) and Day 7, and the tissues were immobilized with formalin. The paraffin sections were then made, and immunohistochemical staining on vascular endothelial cell marker CD31 was performed (Figure 10A).

Microvasculatures in immunostained tissue sections were counted, and the intratumoral microvessel density was determined. As a result, on Day 3, the count under microscope (x 20) was 52.3 ± 9.2 counts in the Mock infection group, 31.6 ± 5.9 counts in the T-01 infection group, 13.7 ± 5.8 counts in the T-sVEGFR1 infection group, and 11.8 ± 4.9 counts in the T-VEGFscFv infected group. Also, on Day 7, the count was 48.0 ± 7.0 counts in the Mock infection group, 36.4 ± 11.1 counts in the T-01 infection group, 11.3 ± 1.7 counts in the T-sVEGFR1 infection group, and was 11.0 ± 3.8 counts in the T-VEGFscFv infection group. As a result, intratumoral microvessels were significantly reduced in the T-sVEGFR1 and T-VEGFscFv administration group relative to the T-01 administration group on both Day 3 and Day 7 (Figure 36).

This showed that T-sVEGFR1 and T-VEGFscFv have the function of suppressing intratumoral angiogenesis. The intratumoral angiogenesis suppression function was considered as one of the mechanisms of the enhanced anti-tumor effects of these two viruses relative to T-01.

### (9) Quantification of viral DNA by Real Time Quantitative PCR

The intratumoral angiogenesis suppression function of T-sVEGFR1 and T-VEGFscFv suggested from the results of CD31 immunostaining was considered as one of the mechanisms of the enhanced anti-tumor effects of these two viruses relative to T-01. However, it has been reported that VEGF has not only a function to promote angiogenesis, but also a function to facilitate the migration of VEGF receptor-positive immune responsible cells such as macrophages. Meanwhile, it has been reported that macrophages reduce the replication efficiency of therapeutic viruses by phagocytosing the therapeutic viruses. It has also been reported that when bevacizmab, an anti-VEGF antibody, is used in combination with therapeutic HSV in order to overcome the reduced replication efficiency of the therapeutic virus due to the phagocytosis of macrophages, the anti-tumor effect is enhanced by reducing intratumoral infiltration of macrophages and improving the replication efficiency of viruses.

It was thus considered that another mechanism of anti-tumor effect enhancement of T-sVEGFR1 and T-VEGFscFv relative to T-01 could be an improvement of the efficiency of virus infection by suppressing intratumoral infiltration of macrophages. First, to study the efficiency of virus infection in HT-29 subcutaneous tumor models, HT-29 was subcutaneously administered to the left abdomen of nude mice to generate subcutaneous tumors. On Day 0, when tumor diameter reached 5 mm after 14 days, T-01, T-sVEGFR1, T-VEGFscFv and Mock were administered into the subcutaneous tumors. Subcutaneous tumors were harvested on Day 3, and DNA was extracted. Real-time PCR was performed targeting the gene encoding DNA polymerase of HSV-1, and intratumoral viral DNA was quantified.

DNA was extracted from the tumor 3 days after virus administration, and real-time PCR was performed. As a result, in both the T-sVEGFR1 and the T-VEGFscFv administration groups, the amounts of viral DNA approximately equal to that in the T-01 administration group were detected. It should be noted that no viral DNA was detected in the Mock administration group.

From this, there was no significant difference in the replication efficiency of viral DNA in T-sVEGFR1 and T-VEGFscFv relative to T-01, and it was thus considered that the replication efficiency was not a major factor in the anti-tumor effect enhancement.

### (10) Study of the anti-tumor effects of T-sVEGFR1 and T-VEGFscFv on CT26 subcutaneous tumor models

From the results of real-time PCR, it was found that the replication efficiency of the virus was not a factor in the enhancement of anti-tumor effects of T-sVEGFR1 and T-VEGFscFv relative to T-01 in HT-29 subcutaneous tumor models. Then, a further study was performed to investigate whether there were any factors that enhance the anti-tumor effects of T-sVEGFR1 and T-VEGFscFv, in addition to the aforementioned tumor angiogenesis inhibitory function. To examine the effects of T-sVEGFR1 and T-VEGFscFv on anti-tumor immunity, experiments were performed using BALB/c mice with normal immune function and colorectal cancer cell line CT26 from BALB/c which was confirmed to produce VEGF. First, the anti-tumor effects of T-sVEGFR1 and T-VEGFscFv on CT26 subcutaneous tumors were examined. CT26 was subcutaneously administered to the left abdomen of BALB/c to generate subcutaneous tumors, and T-01, T-sVEGFR1, T-VEGFscFv and Mock were administered into subcutaneous tumors on Day 0, when tumor diameter reached 5 mm after 10 days, and Day 3.

The tumor volume was measured with continuous observation, then on Day 28, 28 days after the first virus administration significantly strong anti-tumor effects were observed, in the T-sVEGFR1 and T-VEGFscFv administration groups relative to the T-01 administration group (Figure 37).

This result showed that T-sVEGFR1 and T-VEGFscFv had significantly high anti-tumor effects relative to T-01 in normal immune mice, as in the study with nude mice. There was also a tendency that higher therapeutic effects were obtained than those in the study with nude mice. For example, on Day 28, 4 out of 7 tumors disappeared in the T-sVEGFR1 administration group, and 6 out of 7 tumors disappeared in the T-VEGFscFv administration group. It was also considered there is a possibility that T-sVEGFR1 and T-VEGFscFv had increased anti-tumor effects as a result that they were able to induce anti-tumor immunity more efficiently than T-01.

### E. Discussion

### (1) Production of an anti-VEGF antibody expressing oncolytic virus

Oncolytic virus expresses proteins locally in the tumor each time the virus is replicated in the tumor. Thus, although the expression amount of protein is exceptionally high relative to that in non-proliferative viral vectors, protein expression remains localized. Consequently, the risk of side effects is reduced relative to systemic administration. As shown in Result X, bevacizumab was detected at high concentrations in the serum when intraperitoneal administration of Avastin(registered trademark) was used in combination, whereas expressed antibodies were only detected from the tumor locally and were below the detection limit in the serum when T-BV was administered.

As anti-VEGF, when an oncolytic virus to which a single-stranded antibody was introduced and an oncolytic virus to which a double-stranded antibody was introduced were compared, both viruses suppressed the development of swelling. The virus to which a double-stranded antibody is introduced is expected to have more antibody-dependent immune response, because the double-stranded antibody has a longer half-life due to the presence of the Fc moiety.

### (2) Mechanisms of swelling after virus administration

As far as we searched, there have been no reports on swelling observed in the viral therapy to the tumor. However, the swelling is a phenomenon often found as a high intensity area around the tumor in MRI (T2WI) after virus administration in clinical trials of G47Δ to glioblastoma.

In the evaluation of swelling of brain after virus administration by MRI in the present example, the combined use of T-BV and Avastin(registered trademark) showed swelling-suppressive effects, and T-V_{H}V_{L} showed swelling-suppressive tendencies, suggesting that VEGF was involved in swelling of brain upon virus administration (Figures 22 and 25). HSV-1 infection and angiogenesis were observed by immunohistochemical staining in individuals who had a high intensity area around the tumor in MRI (T2WI) after virus administration in U87MG brain tumor models. From this, it was considered that the high intensity area around the tumor in T2WI resulting from virus administration appears when the tumor cells are infected with virus. It was also considered that, since the development is suppressed by the effect of the anti-VEGF antibody, VEGF is involved in the development of swelling.

### Industrial Applicability

According to the present invention, oncology treatment with an oncolytic virus can be performed without causing swelling. The present invention has industrial applicability in the field of medicine.

This application claims priority based on Japanese Patent Application No. 2018-068847, filed on March 30, 2018, the contents of which are hereby incorporated by reference.

### Sequence Listing Free Text

SEQ ID NO: 1 Entire amino acid sequence for anti-human VEGF antibody expression shown in Figure 4;
SEQ ID NO: 2 V_{H} chain (amino acid sequence) in Figure 4;
SEQ ID NO: 3 C_{H} chain (amino acid sequence) in Figure 4;
SEQ ID NO: 4 V_{L} chain (amino acid sequence) in Figure 4;
SEQ ID NO: 5 C_{L} chain (amino acid sequence) in Figure 4;
SEQ ID NO: 6 Ig kappa leader sequence (amino acid sequence) in Figure 4;
SEQ ID NO: 7 Signal peptidase recognition site sequence (amino acid sequence) in Figure 4;
SEQ ID NO: 8 Furin recognition site sequence (amino acid sequence) in Figure 4;
SEQ ID NO: 9 FMDV-2A sequence (amino acid sequence) in Figure 4;
SEQ ID NO: 11 GS linker in Figure 5;
SEQ ID NO: 10 Entire amino acid sequence for anti-VEGF scFv expression shown in Figure 5;
SEQ ID NO: 12 One unit of the GS linker in Figure 5;
SEQ ID NO: 13 cDNA of anti-human VEGF antibody expression gene in Figure 9;
SEQ ID NO: 14 cDNA of V_{H} in Figure 9;
SEQ ID NO: 15 cDNA of C_{H} in Figure 9;
SEQ ID NO: 16 cDNA of V_{L} in Figure 9;
SEQ ID NO: 17 cDNA of C_{L} in Figure 9;
SEQ ID NO: 18 Ig kappa leader sequence (cDNA) in Figure 9;
SEQ ID NO: 19 Signal peptidase recognition site sequence (cDNA) in Figure 9;
SEQ ID NO: 20 Furin recognition site sequence (cDNA) in Figure 9;
SEQ ID NO: 21 FMDV-2A sequence (cDNA) in Figure 9;
SEQ ID NO: 22 cDNA of anti-VEGF scFv expression gene in Figure 10;
SEQ ID NO: 23 Gs linker (cDNA) in Figure 10;
SEQ ID NO: 24 cDNA of T-VEGFscFV (V_{H}V_{L}C_{L}) in Figure 8;
SEQ ID NO: 25 cDNA of CL in Figure 8;
SEQ ID NO: 26 cDNA of sVEGFR1 in Figure 7;
SEQ ID NO: 27 HSV DNApoly-F;
SEQ ID NO: 28 HSV DNApoly-R;
SEQ ID NO: 29 HSV DNApoly-T;
SEQ ID NO: 30 V_{H} chain + C_{H} chain (amino acid sequence) in Figure 4; and
SEQ ID NO: 31 V_{L} chain + C_{L} chain (amino acid sequence) in Figure 4

## Claims

1. An oncolytic virus comprising a gene encoding a vascular endothelial cell growth factor (VEGF) antagonist.

2. The oncolytic virus according to claim 1, wherein the VEGF antagonist is an anti-VEGF antibody or a fragment thereof.

3. The oncolytic virus according to claim 2, wherein the VEGF antagonist is an anti-VEGF antibody or a single-stranded anti-VEGF antibody comprising a V_{H} chain and a V_{L} chain.

4. The oncolytic virus according to claim 2 or 3, wherein the oncolytic virus comprises:
a gene encoding a polypeptide of any one of following (i) to (iii):
(i) a polypeptide of SEQ ID NO: 2;
(ii) a polypeptide that consists of an amino acid sequence in which one or several amino acids are deleted, substituted or added in the polypeptide of SEQ ID NO: 2, and has VEGF binding ability; and
(iii) a polypeptide that has 75% or more, 80% or more, 85% or more, 90% or more, 95% or more, 98% or more, or 99% or more homology to the polypeptide of SEQ ID NO: 2, and has VEGF binding ability; and
a gene encoding a polypeptide of any one of following (iv) to (vi):
(iv) a polypeptide of SEQ ID NO: 4;
(v) a polypeptide that consists of an amino acid sequence in which one or several amino acids are deleted, substituted or added in the polypeptide of SEQ ID NO: 4, and has VEGF binding ability; and
(vi) a polypeptide that has 75% or more, 80% or more, 85% or more, 90% or more, 95% or more, 98% or more, or 99% or more homology to the polypeptide of SEQ ID NO: 4, and has VEGF binding ability.

5. The oncolytic virus according to any one of claims 2 to 4, wherein the anti-VEGF antibody is a human monoclonal antibody or a humanized monoclonal antibody.

6. The oncolytic virus according to claim 1, wherein the VEGF antagonist is a soluble VEGF receptor.

7. The oncolytic virus according to claim 6, wherein the oncolytic virus comprises a gene encoding a polypeptide of any one of following (xiii) to (xv):
(xiii) a polypeptide encoded by a nucleotide sequence of SEQ ID NO: 26;
(xiv) a polypeptide that consists of an amino acid sequence in which one or several amino acids are deleted, substituted or added in the polypeptide encoded by the nucleotide sequence of SEQ ID NO: 26, and has VEGF binding ability; and
(xv) a polypeptide that has 75% or more, 80% or more, 85% or more, 90% or more, 95% or more, 98% or more, or 99% or more homology to the polypeptide encoded by the nucleotide sequence of SEQ ID NO: 26, and has VEGF binding ability.

8. The oncolytic virus according to any one of claims 1 to 7, wherein the oncolytic virus is a variant of a virus selected from the group consisting of herpes simplex virus type I and type II (HSV-1 and HSV-2), adenovirus, poliovirus, measles virus, reovirus, vaccinia virus, seneca virus, vesicular stomatitis virus (VSV), Newcastle disease virus and coxsackievirus.

9. The oncolytic virus according to any one of claims 1 to 7, wherein the oncolytic virus is a herpes simplex virus type I variant having one or more characteristics of (a) to (c):
(a) ICP6 gene is deleted or inactivated, or expressed under control of a tumor-specific promoter or a tissue-specific promoter;
(b) γ34.5 gene is deleted or inactivated; and
(c) ICP47 gene is deleted or inactivated.

10. A pharmaceutical composition for treating a tumor, comprising a therapeutically effective amount of the oncolytic virus according to any one of claims 1 to 9.

11. The pharmaceutical composition for treating a tumor according to claim 10, wherein the tumor is a human tumor selected from the group consisting of a nervous system tumor, a pituitary tumor, medulloblastoma, melanoma, a brain tumor, a prostate cancer, a head and neck cancer, an esophageal cancer, a kidney cancer, a renal cell carcinoma, a pancreatic cancer, a breast cancer, a lung cancer, a colorectal cancer, a colon cancer, a gastric cancer, a skin cancer, an ovarian cancer, a bladder cancer, sarcoma, a squamous cell cancer, neuroectodermal tumor, a thyroid tumor, lymphoma, a hepatocellular carcinoma, mesothelioma, an epidermoid cancer, and a benign tumor.

12. The pharmaceutical composition for treating a tumor according to claim 10, wherein the tumor is a brain tumor and the pharmaceutical composition is administered topically and swelling-suppressive.

13. The pharmaceutical composition for treating a tumor according to any one of claims 10 to 12, wherein the pharmaceutical composition is used in combination with another tumor therapy selected from a chemotherapy and a radiation therapy.


SEQUENCE LISTING

<110>  University of Tokyo

<120>  Oncolytic virus that prevents tumor swelling

<130>  T0529AJP0014

<150>  JP2018-068847
<151>  2018-03-30

<160>  31

<170>  PatentIn version 3.5

<210>  1
<211>  743
<212>  PRT
<213>  Artificial

<220>
<223>  Sequence for anti-human VEGF antibody expression

<400>  1

Glu Thr Asp Thr Leu Leu Leu Trp Val Leu Leu Leu Trp Val Pro Gly
1               5                   10                  15

Ser Thr Gly Asp Ala Ala Gln Pro Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Tyr Thr Phe Thr Asn Tyr Gly Met Asn Trp Val Arg Gln Ala
    50                  55                  60

Pro Gly Lys Gly Leu Glu Trp Val Gly Trp Ile Asn Thr Tyr Thr Gly
65                  70                  75                  80

Glu Pro Thr Tyr Ala Ala Asp Phe Lys Arg Arg Phe Thr Phe Ser Leu
                85                  90                  95

Asp Thr Ser Lys Ser Thr Ala Tyr Leu Gln Met Asn Ser Leu Arg Ala
            100                 105                 110

Glu Asp Thr Ala Val Tyr Tyr Cys Ala Lys Tyr Pro His Tyr Tyr Gly
        115                 120                 125

Ser Ser His Trp Tyr Phe Asp Val Trp Gly Gln Gly Thr Leu Val Thr
    130                 135                 140

Val Ser Ser Ala Ser Thr Lys Gly Pro Ser Val Phe Pro Leu Ala Pro

145                 150                 155                 160

Ser Ser Lys Ser Thr Ser Gly Gly Thr Ala Ala Leu Gly Cys Leu Val
                165                 170                 175

Lys Asp Tyr Phe Pro Glu Pro Val Thr Val Ser Trp Asn Ser Gly Ala
            180                 185                 190

Leu Thr Ser Gly Val His Thr Phe Pro Ala Val Leu Gln Ser Ser Gly
        195                 200                 205

Leu Tyr Ser Leu Ser Ser Val Val Thr Val Pro Ser Ser Ser Leu Gly
    210                 215                 220

Thr Gln Thr Tyr Ile Cys Asn Val Asn His Lys Pro Ser Asn Thr Lys
225                 230                 235                 240

Val Asp Lys Lys Val Glu Pro Lys Ser Cys Asp Lys Thr His Thr Cys
                245                 250                 255

Pro Pro Cys Pro Ala Pro Glu Leu Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser His Glu Asp Pro Glu Val Lys
    290                 295                 300

Phe Asn Trp Tyr Val Asp Gly Val Glu Val His Asn Ala Lys Thr Lys
305                 310                 315                 320

Pro Arg Glu Glu Gln Tyr Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Ala Leu Pro Ala Pro Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Arg Asp Glu Leu Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Lys Leu Thr Val Asp Lys Ser Arg Trp Gln
        435                 440                 445

Gln Gly Asn Val Phe Ser Cys Ser Val Met His Glu Gly Leu His Asn
    450                 455                 460

His Tyr Thr Gln Lys Ser Leu Ser Leu Ser Pro Gly Lys Arg Lys Arg
465                 470                 475                 480

Arg Ala Pro Val Lys Gln Thr Leu Asn Phe Asp Leu Leu Lys Leu Ala
                485                 490                 495

Gly Asp Val Glu Ser Asn Pro Gly Pro Glu Thr Asp Thr Leu Leu Leu
            500                 505                 510

Trp Val Leu Leu Leu Trp Val Pro Gly Ser Thr Gly Asp Ala Ala Gln
        515                 520                 525

Pro Asp Ile Gln Met Thr Gln Ser Pro Ser Ser Leu Ser Ala Ser Val
    530                 535                 540

Gly Asp Arg Val Thr Ile Thr Cys Ser Ala Ser Gln Asp Ile Ser Asn
545                 550                 555                 560

Tyr Leu Asn Trp Tyr Gln Gln Lys Pro Gly Lys Ala Pro Lys Val Leu
                565                 570                 575

Ile Tyr Phe Thr Ser Ser Leu His Ser Gly Val Pro Ser Arg Phe Ser
            580                 585                 590

Gly Ser Gly Ser Gly Thr Asp Phe Thr Leu Thr Ile Ser Ser Leu Gln
        595                 600                 605

Pro Glu Asp Phe Ala Thr Tyr Tyr Cys Gln Gln Tyr Ser Thr Val Pro
    610                 615                 620

Trp Thr Phe Gly Gln Gly Thr Lys Val Glu Ile Lys Arg Thr Val Ala
625                 630                 635                 640

Ala Pro Ser Val Phe Ile Phe Pro Pro Ser Asp Glu Gln Leu Lys Ser
                645                 650                 655

Gly Thr Ala Ser Val Val Cys Leu Leu Asn Asn Phe Tyr Pro Arg Glu
            660                 665                 670

Ala Lys Val Gln Trp Lys Val Asp Asn Ala Leu Gln Ser Gly Asn Ser
        675                 680                 685

Gln Glu Ser Val Thr Glu Gln Asp Ser Lys Asp Ser Thr Tyr Ser Leu
    690                 695                 700

Ser Ser Thr Leu Thr Leu Ser Lys Ala Asp Tyr Glu Lys His Lys Val
705                 710                 715                 720

Tyr Ala Cys Glu Val Thr His Gln Gly Leu Ser Ser Pro Val Thr Lys
                725                 730                 735

Ser Phe Asn Arg Gly Glu Cys
            740

<210> 2
<211> 123
<212> PRT
<213> Artificial

<220>
<223> VH chain

<400> 2

<210>  3
<211>  330
<212>  PRT
<213>  Artificial

<220>
<223>  CH Chain

<400>  3
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Gly Leu His Asn His Tyr Thr
   305                 310                 315                 320
   Gln Lys Ser Leu Ser Leu Ser Pro Gly Lys
                   325                 330

<210>  4
<211>  108
<212>  PRT
<213>  Artificial

<220>
<223>  VL Chain

<400>  4

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg
            100                 105

<210>  5
<211>  106
<212>  PRT
<213>  Artificial

<220>
<223>  CL Chain

<400>  5

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  6
<211>  20
<212>  PRT
<213>  Artificial

<220>
<223>  Ig kappa leader sequence

<400>  6

Glu Thr Asp Thr Leu Leu Leu Trp Val Leu Leu Leu Trp Val Pro Gly
1               5                   10                  15

Ser Thr Gly Asp

            20

<210>  7
<211>  4
<212>  PRT
<213>  Artificial

<220>
<223>  Signal peptidase recognition sequence

<400>  7

Ala Ala Gln Pro
1

<210>  8
<211>  4
<212>  PRT
<213>  Artificial

<220>
<223>  furin recognition sequence

<400>  8

Arg Lys Arg Arg
1

<210>  9
<211>  24
<212>  PRT
<213>  Artificial

<220>
<223>  FMDV-2A

<400>  9

Ala Pro Val Lys Gln Thr Leu Asn Phe Asp Leu Leu Lys Leu Ala Gly
1               5                   10                  15

Asp Val Glu Ser Asn Pro Gly Pro
            20

<210>  10
<211>  263
<212>  PRT
<213>  Artificial

<220>
<223>  sequence for anti-VEGFscFv expression

<400>  10

Glu Thr Asp Thr Leu Leu Leu Trp Val Leu Leu Leu Trp Val Pro Gly
1               5                   10                  15

Ser Thr Gly Asp Ala Ala Gln Pro Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Phe Thr Ile Ser Asp Tyr Trp Ile His Trp Val Arg Gln Ala
    50                  55                  60

Pro Gly Lys Gly Leu Glu Trp Val Ala Gly Ile Thr Pro Ala Gly Gly
65                  70                  75                  80

Tyr Thr Tyr Tyr Ala Asp Ser Val Lys Gly Arg Phe Thr Ile Ser Ala
                85                  90                  95

Asp Thr Ser Lys Asn Thr Ala Tyr Leu Gln Met Asn Ser Leu Arg Ala
            100                 105                 110

Glu Asp Thr Ala Val Tyr Tyr Cys Ala Arg Phe Val Phe Phe Leu Pro
        115                 120                 125

Tyr Ala Met Asp Tyr Trp Gly Gln Gly Thr Leu Val Gly Gly Gly Gly
    130                 135                 140

Ser Gly Gly Gly Gly Ser Gly Gly Gly Gly Ser Asp Ile Gln Met Thr
145                 150                 155                 160

Gln Ser Pro Ser Ser Leu Ser Ala Ser Val Gly Asp Arg Val Thr Ile
                165                 170                 175

Thr Cys Arg Ala Ser Gln Asp Val Ser Thr Ala Val Ala Trp Tyr Gln
            180                 185                 190

Gln Lys Pro Gly Lys Ala Pro Lys Leu Leu Ile Tyr Ser Ala Ser Phe
        195                 200                 205

Leu Tyr Ser Gly Val Pro Ser Arg Phe Ser Gly Ser Gly Ser Gly Thr
    210                 215                 220

Asp Phe Thr Leu Thr Ile Ser Ser Leu Gln Pro Glu Asp Phe Ala Thr
225                 230                 235                 240

Tyr Tyr Cys Gln Gln Gly Tyr Gly Asn Pro Phe Thr Phe Gly Gln Gly
                245                 250                 255

Thr Lys Val Glu Ile Lys Arg
            260

<210>  11
<211>  15
<212>  PRT
<213>  Artificial

<220>
<223>  GS linker

<400>  11

Gly Gly Gly Gly Ser Gly Gly Gly Gly Ser Gly Gly Gly Gly Ser
1               5                   10                  15


<210>  12
<211>  5
<212>  PRT
<213>  Artificial

<220>
<223>  GS linker (unit)

<400>  12

Gly Gly Gly Gly Ser
1               5


<210>  13
<211>  2261
<212>  DNA
<213>  Artificial

<220>
<223>  sequence for anti-human VEGF antibody expression

<400>  13
ggatccgcca ccatggaaac tgacacgttg ctgctctggg tgctgttgct ttgggttcca

ggcagtaccg gcgatgccgc ccaaccggag gttcagttgg tggaaagtgg aggtggcctc      1

gtccagcccg gaggctcact gagactgtcc tgtgccgcca gtggctatac cttcactaac      1

tatgggatga actgggtgag acaagctccc ggaaaaggtc tggagtgggt tgggtggatc      2

aatacctaca caggggaacc tacctacgca gccgacttca agcgacgctt taccttttcc      3

ctggatacct ctaagtctac tgcctatttg cagatgaaca gtctgagagc ggaagatact      3

gcggtctatt actgcgctaa atacccacat tattacggat catctcactg gtacttcgat      4

gtttggggcc aggggaccct tgtcactgtg agttcagcaa gcacaaaagg gccgtccgtg      4

tttccattgg caccatcttc caagtccaca tctggcggaa ctgcagctct ggggtgcctg      5

gtaaaggact atttccccga acctgttacc gtaagttgga acagcggggc attgacaagc      6

ggagtgcaca cttttcccgc ggtgctgcaa agctccggcc tctattccct ctcaagcgta      6

gtcacggtac ccagctccag cctcggcaca cagacgtaca tctgcaatgt gaatcataag      7

gggaggaaga ggaaggtgga gaagaaggtg gaagggaagt ggtgtgataa gagggagaga

tgtcctccat gtccggcacc cgagctgctt ggcggaccct ctgtgtttct gttccctccc 840

aagcccaaag acacccttat gatatctcgt acaccagagg tcacatgtgt ggtcgtcgac 900

gttagccacg aggacccaga ggtgaagttc aactggtacg tggacggtgt agaagtccac 960

aacgccaaaa caaagccacg cgaagaacag tacaatagca catatcgtgt cgtgtcagtg 1020

ctgactgtcc tgcatcagga ttggctgaat ggcaaagagt acaaatgcaa ggtttccaac 1080

aaggctcttc cagctcccat tgagaaaaca atcagcaagg ccaaaggaca acctagagag 1140

cctcaggtgt ataccctccc tcccagtcgg gatgagctca caaagaatca ggtgtcactt 1200

acttgtctcg tcaaaggttt ctatccctct gatattgccg tagagtggga gagcaatggg 1260

cagccagaga acaactacaa aaccactccg cccgtactgg actcagacgg ctcattcttc 1320

ctctactcca agctgacagt ggataaatcc cgatggcaac agggcaatgt tttcagctgc 1380

agcgtcatgc atgagggact gcacaaccac tatacccaga agagtctcag cctgagccct 1440

ggtaagagga agaggagggc acccgtgaaa cagaccttga actttgacct tctgaagctg 1500

gccggtgatg tggaaagcaa tccagggcca gaaaccgata ccctgcttct ctgggtcttg 1560

ctgctgtggg ttccagggtc tacaggtgac gctgcccaac ctgatatcca gatgacccag 1620

agccctagta gcctgtctgc ctcagtgggt gatcgggtga ccataacttg ctccgcttcc 1680

caagacattt ctaactacct gaactggtac cagcagaaac ccgggaaagc ccccaaggtt 1740

ctgatctact ttactagctc tcttcattcc ggagtccctt ctcggtttag cggctctgga 1800

agcggcacag actttaccct gacgattagc agtctgcagc cggaggattt tgcaacgtat 1860

tattgccagc agtacagcac ggtaccatgg acttttggcc agggcaccaa ggttgagatt 1920

aagcgcaccg tggcagctcc ttctgtgttc atctttcctc cttccgatga gcagctgaaa 1980

agtggaacag cgtcagtggt ctgcctgctg aataacttct atcctaggga agccaaggtt 2040

cagtggaaag tggacaatgc cctgcaatca ggcaattctc aggaatccgt gacagagcag 2100

gactcaaaag actccactta cagtctgagc agtaccctca cactcagcaa agctgactac 2160

gaaaagcaca aagtgtatgc ttgcgaggtg actcatcaag ggctgtcctc tcccgtgact 2220

aaatcattca atcggggaga gtgttagtga taagcggccg c 2261

<210> 14
<211> 369
<212> DNA
<213> Artificial

<220>
<223> VH chain

<400> 14
gaggttcagt tggtggaaag tggaggtggc ctcgtccagc ccggaggctc actgagactg 60

tcctgtgccg ccagtggcta taccttcact aactatggga tgaactgggt gagacaagct 120

cccggaaaag gtctggagtg ggttgggtgg atcaatacct acacagggga acctacctac 180

gcagccgact tcaagcgacg ctttaccttt tccctggata cctctaagtc tactgcctat 240

ttgcagatga acagtctgag agcggaagat actgcggtct attactgcgc taaataccca 300

cattattacg gatcatctca ctggtacttc gatgtttggg gccaggggac ccttgtcact 360

gtgagttca 369

<210> 15
<211> 990
<212> DNA
<213> Artificial

<220>
<223> CH chain

<400> 15
gcaagcacaa aagggccgtc cgtgtttcca ttggcaccat cttccaagtc cacatctggc 60

ggaactgcag ctctggggtg cctggtaaag gactatttcc ccgaacctgt taccgtaagt 120

tggaacagcg gggcattgac aagcggagtg cacacttttc ccgcggtgct gcaaagctcc 180

ggcctctatt ccctctcaag cgtagtcacg gtacccagct ccagcctcgg cacacagacg 240

tacatctgca atgtgaatca taagccgagc aacaccaagg tcgacaagaa ggtggaaccc 300

aagtcctgtg ataagacgca cacatgtcct ccatgtccgg cacccgagct gcttggcgga 360

ccctctgtgt ttctgttccc tcccaagccc aaagacaccc ttatgatatc tcgtacacca 420

gaggtcacat gtgtggtcgt cgacgttagc cacgaggacc cagaggtgaa gttcaactgg 480

tacgtggacg gtgtagaagt ccacaacgcc aaaacaaagc cacgcgaaga acagtacaat 540

agcacatatc gtgtcgtgtc agtgctgact gtcctgcatc aggattggct gaatggcaaa 600

gagtacaaat gcaaggtttc caacaaggct cttccagctc ccattgagaa aacaatcagc 660

aaggccaaag gacaacctag agagcctcag gtgtataccc tccctcccag tcgggatgag 720

ctcacaaaga atcaggtgtc acttacttgt ctcgtcaaag gtttctatcc ctctgatatt 780

gccgtagagt gggagagcaa tgggcagcca gagaacaact acaaaaccac tccgcccgta 840

ctggactcag acggctcatt cttcctctac tccaagctga cagtggataa atcccgatgg 900

caacagggca atgttttcag ctgcagcgtc atgcatgagg gactgcacaa ccactatacc 960

cagaagagtc tcagcctgag ccctggtaag 990

<210> 16
<211> 324
<212> DNA
<213> Artificial

<220>

<223>  VL chain

<400>  16
gatatccaga tgacccagag ccctagtagc ctgtctgcct cagtgggtga tcgggtgacc      60

ataacttgct ccgcttccca agacatttct aactacctga actggtacca gcagaaaccc     120

gggaaagccc caaaggttct gatctacttt actagctctc ttcattccgg agtcccttct     180

cggtttagcg gctctggaag cggcacagac tttaccctga cgattagcag tctgcagccg     240

gaggattttg caacgtatta ttgccagcag tacagcacgg taccatggac ttttgggcag     300

ggcaccaagg ttgagattaa gcgc                                            324


<210>  17
<211>  318
<212>  DNA
<213>  Artificial

<220>
<223>  CL chain

<400>  17
accgtggcag ctccttctgt gttcatcttt cctccttccg atgagcagct gaaaagtgga      60

acagcgtcag tggtctgcct gctgaataac ttctatccta gggaagccaa ggttcagtgg     120

aaagtggaca atgccctgca atcaggcaat tctcaggaat ccgtgacaga gcaggactca     180

aaagactcca cttacagtct gagcagtacc ctcacactca gcaaagctga ctacgaaaag     240

cacaaagtgt atgcttgcga ggtgactcat caagggctgt cctctcccgt gactaaatca     300

ttcaatcggg gagagtgt                                                   318


<210>  18
<211>  60
<212>  DNA
<213>  Artificial

<220>
<223>  Ig kappa leader sequence

<400>  18
gaaactgaca cgttgctgct ctgggtgctg ttgctttggg ttccaggcag taccggcgat      60


<210>  19
<211>  12
<212>  DNA
<213>  Artificial

<220>
<223>  signal peptidase recognition sequence

<400>  19
gccgcccaac cg                                                          12


<210>  20

<211>  12
<212>  DNA
<213>  Artificial

<220>
<223>  furin recognition sequence

<400>  20
aggaagagga gg                                                          12


<210>  21
<211>  72
<212>  DNA
<213>  Artificial

<220>
<223>  FMDV-2A

<400>  21
gcacccgtga aacagacctt gaactttgac cttctgaagc tggccggtga tgtggaaagc      60

aatccagggc ca                                                          72


<210>  22
<211>  815
<212>  DNA
<213>  Artificial

<220>
<223>  sequence for anti-VEGFscFv expression

<400>  22
ggattcgcca ccatggagac agacacactc ctgctatggg tactgctgct ctgggttcca      60

ggttccactg gtgacgcggc ccagccggag gtgcaattgg tggagtctgg gggaggcttg     120

gtacagccgg gggggtccct gagactctcc tgtgcagcct ctggattcac aatcagcgac     180

tattggattc actgggtccg ccaggctcca gggaaggggc tggagtgggt tgcaggtata     240

accccggctg gtggctacac atattacgca gactccgtta agggccgatt caccatctcc     300

gcagacactt ccaagaacac ggcttatctg cagatgaaca gcctgcgagc cgaggacacg     360

gccgtctatt actgtgctag gttcgtgttc tttctccctt atgctatgga ctactggggc     420

cagggaaccc tggtcggtgg cggaggttca ggcggtggag gttccggtgg aggtgggtct     480

gacatccaga tgacccagtc tccatcctcc ctgtctgcat ctgtaggaga cagagtcacc     540

atcacttgcc gggcaagtca ggacgttagc accgctgtag catggtatca gcagaaacca     600

gggaaagccc ctaagctcct gatctattct gcatccttct tgtacagtgg ggtcccatca     660

aggttcagtg gcagtggatc tgggacagat ttcactctca ccatcagcag tctgcaacct     720

gaagattttg caacttacta ctgtcaacag ggttacggta acccgttcac ttttggccag     780

gggaccaagg tggagatcaa acgataggcg gccgc                                815

<210>  23
<211>  45
<212>  DNA
<213>  Artificial

<220>
<223>  GS linker

<400>  23
ggtggcggag gttcaggcgg tggaggttcc ggtggaggtg ggtct                    45


<210>  24
<211>  1133
<212>  DNA
<213>  Artificial

<220>
<223>  T-VEGFscFv(VHVLCL)

<400>  24
ggatccgcca ccatggagac agacacactc ctgctatggg tactgctgct ctgggttcca      60

ggttccactg gtgacgcggc ccagccggag gtgcaattgg tggagtctgg gggaggcttg     120

gtacagccgg gggggtccct gagactctcc tgtgcagcct ctggattcac aatcagcgac     180

tattggattc actgggtccg ccaggctcca gggaaggggc tggagtgggt tgcaggtata     240

accccggctg gtggctacac atattacgca gactccgtta agggccgatt caccatctcc     300

gcagacactt ccaagaacac ggcttatctg cagatgaaca gcctgcgagc cgaggacacg     360

gccgtctatt actgtgctag gttcgtgttc tttctccctt atgctatgga ctactggggc     420

cagggaaccc tggtcggtgg cggaggttca ggcggtggag gttccggtgg aggtgggtct     480

gacatccaga tgacccagtc tccatcctcc ctgtctgcat ctgtaggaga cagagtcacc     540

atcacttgcc gggcaagtca ggacgttagc accgctgtag catggtatca gcagaaacca     600

gggaaagccc ctaagctcct gatctattct gcatccttct tgtacagtgg ggtcccatca     660

aggttcagtg gcagtggatc tgggacagat ttcactctca ccatcagcag tctgcaacct     720

gaagattttg caacttacta ctgtcaacag ggttacggta acccgttcac ttttggccag     780

gggaccaagg tggagatcaa acgaactgtg gctgcaccat ctgtcttcat cttcccgcca     840

tctgatgagc agttgaaatc tggaactgcc tctgttgtgt gcctgctgaa taacttctat     900

cccagagagg ccaaagtaca gtggaaggtg gataacgccc tccaatcggg taactcccag     960

gagagtgtca cagagcagga cagcaaggac agcacctaca gcctcagcag caccctgacg    1020

ctgagcaaag cagactacga gaaacacaaa gtctacgcct gcgaagtcac ccatcagggc    1080

ctgagctcgc ccgtcacaaa gagcttcaac aggggagagt gttaggcggc cgc           1133


<210>  25
<211>  318
<212>  DNA

<213>  Artificial

<220>
<223>  CL chain

<400>  25
actgtggctg caccatctgt cttcatcttc ccgccatctg atgagcagtt gaaatctgga      60

actgcctctg ttgtgtgcct gctgaataac ttctatccca gagaggccaa agtacagtgg     120

aaggtggata acgccctcca atcgggtaac tcccaggaga gtgtcacaga gcaggacagc     180

aaggacagca cctacagcct cagcagcacc ctgacgctga gcaaagcaga ctacgagaaa     240

cacaaagtct acgcctgcga agtcacccat cagggcctga gctcgcccgt cacaaagagc     300

ttcaacaggg gagagtgt                                                   318


<210>  26
<211>  2064
<212>  DNA
<213>  Artificial

<220>
<223>  sCEGFR1

<400>  26
atggtcagct actgggacac cggggtcctg ctgtgcgcgc tgctcagctg tctgcttctc      60

acaggatcta gttcaggttc aaaattaaaa gatcctgaac tgagtttaaa aggcacccag     120

cacatcatgc aagcaggcca gacactgcat ctccaatgca ggggggaagc agcccataaa     180

tggtctttgc ctgaaatggt gagtaaggaa agcgaaaggc tgagcataac taaatctgcc     240

tgtggaagaa atggcaaaca attctgcagt actttaacct tgaacacagc tcaagcaaac     300

cacactggct tctacagctg caaatatcta gctgtaccta cttcaaagaa gaaggaaaca     360

gaatctgcaa tctatatatt tattagtgat acaggtagac ctttcgtaga gatgtacagt     420

gaaatccccg aaattataca catgactgaa ggaagggagc tcgtcattcc ctgccgggtt     480

acgtcaccta acatcactgt tactttaaaa aagtttccac ttgacacttt gatccctgat     540

ggaaaacgca taatctggga cagtagaaag ggcttcatca tatcaaatgc aacgtacaaa     600

gaaatagggc ttctgacctg tgaagcaaca gtcaatgggc atttgtataa gacaaactat     660

ctcacacatc gacaaaccaa tacaatcata gatgtccaaa taagcacacc acgcccagtc     720

aaattactta gaggccatac tcttgtcctc aattgtactg ctaccactcc cttgaacacg     780

agagttcaaa tgacctggag ttaccctgat gaaaaaaata agagagcttc cgtaaggcga     840

cgaattgacc aaagcaattc ccatgccaac atattctaca gtgttcttac tattgacaaa     900

atgcagaaca aagacaaagg actttatact tgtcgtgtaa ggagtggacc atcattcaaa     960

tctgttaaca cctcagtgca tatatatgat aaagcattca tcactgtgaa acatcgaaaa    1020

cagcaggtgc ttgaaaccgt agctggcaag cggtcttacc ggctctctat gaaagtgaag    1080

gcatttccct cgccggaagt tgtatggtta aaagatgggt tacctgcgac tgagaaatct 1140

gctcgctatt tgactcgtgg ctactcgtta attatcaagg acgtaactga agaggatgca 1200

gggaattata caatcttgct gagcataaaa cagtcaaatg tgtttaaaaa cctcactgcc 1260

actctaattg tcaatgtgaa accccagatt tacgaaaagg ccgtgtcatc gtttccagac 1320

ccggctctct acccactggg cagcagacaa atcctgactt gtaccgcata tggtatccct 1380

caacctacaa tcaagtggtt ctggcacccc tgtaaccata atcattccga agcaaggtgt 1440

gacttttgtt ccaataatga agagtccttt atcctggatg ctgacagcaa catgggaaac 1500

agaattgaga gcatcactca gcgcatggca ataatagaag gaaagaataa gatggctagc 1560

accttggttg tggctgactc tagaatttct ggaatctaca tttgcatagc ttccaataaa 1620

gttgggactg tgggaagaaa cataagcttt tatatcacag atgtgccaaa tgggtttcat 1680

gttaacttgg aaaaaatgcc gacggaagga gaggacctga aactgtcttg cacagttaac 1740

aagttcttat acagagacgt tacttggatt ttactgcgga cagttaataa cagaacaatg 1800

cactacagta ttagcaagca aaaaatggcc atcactaagg agcactccat cactcttaat 1860

cttaccatca tgaatgtttc cctgcaagat tcaggcacct atgcctgcag agccaggaat 1920

gtatacacag gggaagaaat cctccagaag aaagaaatta caatcagagg tgagcactgc 1980

aacaaaaagg ctgttttctc tcggatctcc aaatttaaaa gcacaaggaa tgattgtacc 2040

acacaaagta atgtaaaaca ttaa 2064

<210> 27
<211> 18
<212> DNA
<213> Artificial

<220>
<223> HSV DNApoly-F

<400> 27
ggcacgcggc agtacttt 18

<210> 28
<211> 17
<212> DNA
<213> Artificial

<220>
<223> HSV DNApoly-R

<400> 28
ccatgcgctc gcagaga 17

<210> 29
<211> 25
<212> DNA

<213>  Artificial

<220>
<223>  HSV DNApoly-T

<400>  29
aggtcgacag gcacctacaa tgccg                                          25


<210>  30
<211>  453
<212>  PRT
<213>  Artificial

<220>
<223>  VH chain and CH chain

<400>  30

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Gly Leu His Asn His Tyr Thr Gln Lys Ser Leu Ser
        435                 440                 445

Leu Ser Pro Gly Lys
    450

<210>  31
<211>  214
<212>  PRT
<213>  Artificial

<220>
<223>  VL chain and CL chain

<400>  31

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210